# FASCICULE DE BREVET EUROPEEN

(11) **EP 4 149 957 B1**
(45) Date de publication et mention de la délivrance du brevet: **03.07.2024**
(21) Numéro de dépôt: 21731556.3
(22) Date de dépôt: 11.05.2021
(51) Int. Cl.: C07K 14/245, C12N 9/10, C12N 9/12, C12N 9/16, C12N 9/38, C12N 9/90, C12N 15/52, C12P 19/00

(54) **SOUCHES BACTÉRIENNES ET PROCÉDÉ DE PRODUCTION D'OLIGOSACCHARIDES**
BAKTERIENSTÄMME UND VERFAHREN ZUR HERSTELLUNG VON OLIGOSACCHARIDEN
BACTERIAL STRAINS AND PROCESS FOR THE PRODUCTION OF OLIGOSACCHARIDES

(30) Priorité: 12.05.2020 FR 2004669
(43) Date de publication de la demande: 22.03.2023
(73) Titulaire: Institut National des Sciences Appliquées de Toulouse, 31400 Toulouse (FR); Institut National de la Recherche pour l'Agriculture, l'Alimentation et l'Environnement, 75007 Paris (FR); Centre National de la Recherche Scientifique, 75016 Paris (FR); Université Paul Sabatier Toulouse III, 31400 Toulouse (FR)
(72) Inventeur: DURAND, Julien, 31077 TOULOUSE CEDEX 04 (FR); LETISSE, Fabien, 31077 TOULOUSE CEDEX 04 (FR); TEDESCO, Pietro, 31077 TOULOUSE CEDEX 04 (FR); VERONESE, Gabrielle, 31077 TOULOUSE CEDEX 04 (FR)
(74) Mandataire: Novagraaf Technologies
(86) Numéro de dépôt international: PCT/FR2021/050827
(87) Numéro de publication internationale: WO 2021/229185

(56) Documents cités:
- WO-A1-01/04341
- WO-A1-2019/123324
- FIERFORT N ET AL: "Genetic engineering of Escherichia coli for the economical production of sialylated oligosaccharides", JOURNAL OF BIOTECHNOLOGY, ELSEVIER, AMSTERDAM NL, vol. 134, no. 3-4, 30 avril 2008 (2008-04-30), pages 261-265, XP022588000, ISSN: 0168-1656, DOI: 10.1016/J.JBIOTEC.2008.02.010 [extrait le 2008-03-10] cité dans la demande

## Description

### Domaine technique

La présente invention se rapporte à des souches bactériennes et également à l'utilisation *in vitro* des souches bactériennes pour la production d'oligosaccharides et/ou dans un procédé de production d'oligosaccharides. La présente invention se rapporte également à un procédé *in vitro,* en particulier *in cellulo,* de production d'oligosaccharides.

La présente invention trouve une application, notamment dans le domaine de la bioproduction, par exemple la production de composés, par exemple de biocomposés.

Dans la description ci-dessous, les références entre crochets ([ ]) renvoient à la liste des références présentée à la fin du texte.

### Etat de la technique

Les oligosaccharides représentent une classe de biomolécules très diverses constituées de chaînes linéaires ou ramifiées d'unités glycosyles liées entre elles par différents types de liaisons α ou β-osidique. Leur diversité structurale résulte à la fois de la nature des monomères, de la position et de l'anomérie de leurs liaisons covalentes (α ou β). Ces molécules jouent des rôles divers et importants dans une multitude de processus cellulaires, y compris la signalisation et la différenciation cellulaire, la modulation de la réponse immunitaire et de l'inflammation, la médiation des interactions microbiennes et des interactions hôte-microbe. En raison de leurs structures et de leurs fonctions biologiques très diversifiées, les oligosaccharides sont utilisés dans un large éventail d'applications dans l'industrie alimentaire (aliments fonctionnels, prébiotiques ou additifs) et dans l'industrie de la santé (en tant qu'éléments de base pour la synthèse chimique de médicaments, kits de dépistage, supports pharmaceutiques ou intermédiaires de vaccins).

Par conséquent, l'intérêt pour la production d'oligosaccharides augmente très rapidement. Le défi est de développer des voies de synthèse conformes aux principes de la chimie verte (Anastas et Warner, 2000 Anastas, P.T., Warner, J.C., 2000. Green chemistry: theory and practice, 1. paperback. ed. Oxford Univ. Press, Oxford. [15]), basées sur l'utilisation de bioressources bon marché et versatiles, pour produire une large diversité d'oligosaccharides.

Il existe donc un réel besoin de trouver un moyen et/ou procédé permettant de maîtriser la production d'oligosaccharides tout en réduisant les coûts de production de différents oligosaccharides et/ou d'améliorer les rendements de production, tout en évitant et/ou réduisant et/ou supprimant l'utilisation de composés et/ou solutions néfastes pour l'environnement.

Il existe actuellement différents procédés de productions d'oligosaccharides. Certains oligosaccharides sont actuellement produits par synthèse chimique. Cette approche nécessite de multiples étapes réactionnelles et de purification pour protéger et déprotéger les groupes fonctionnels et réaliser des synthèses régio- et stéréo- spécifiques. Malgré des progrès significatifs dans ce domaine (Rudroff, F., Mihovilovic, M.D., Gröger, H., Snajdrova, R., Iding, H., Bornscheuer, U.T., 2018. Opportunities and challenges for combining chemo- and biocatalysis. Nature Catalysis 1, 12-22. https://doi.org/10.1038/s41929-017-0010-4 [18]), ces voies de production, dans la plupart des cas, conduisent à un faible niveau de production (Ager, 2012 Ager, D., 2012. Final Analysis: Biological or Chemical Catalysis Platinum Metals Review 56 [19]), et nécessitent l'utilisation de solvants polluants. De plus, la diversité des structures oligosaccharidiques qui peuvent être obtenues par synthèse chimique est limitée.

Aussi, il existe donc un réel besoin de trouver un moyen et/ou procédé permettant de maîtriser la production d'oligosaccharides tout en réduisant les coûts, notamment de production, d'améliorer les rendements de productions tout en évitant et/ou réduisant et/ou supprimant l'utilisation de composés et/ou solutions néfastes pour l'environnement.

Des voies de production d'oligosaccharides chimio-enzymatiques et enzymatiques ont été mises au point ces vingt dernières années afin de tirer parti de la diversité naturelle et de la sélectivité des enzymes, et ainsi élargir le panel des structures oligosaccharidiques pouvant être produits par biocatalyse (Combes, Monsan, 2009. Biocatalyse ou catalyse enzymatique. Procédés chimie - bio- agro I Chimie verte, Techniques de l'ingénieur 1-18. [13]; Reetz, M.T., 2013. Biocatalysis in Organic Chemistry and Biotechnology: Past, Présent, and Future. Journal of the American Chemical Society 135, 12480-12496. https://doi.org/10.1021/ja405051f [20]; Rudroff, F., Mihovilovic, M.D., Gröger, H., Snajdrova, R., Iding, H., Bornscheuer, U.T., 2018. Opportunities and challenges for combining chemo- and biocatalysis. Nature Catalysis 1, 12-22. [14]). Cependant, l'utilisation des enzymes dans les procédés *in vitro* est limitée en raison du coût de production et de purification des enzymes, de la perte d'activité des enzymes pendant leur stockage, et leur mise en oeuvre, et en outre du coût des substrats/matières premières nécessaires.

Il existe donc un réel besoin de trouver un moyen et/ou procédé et/ou nouveaux procédés permettant de maîtriser la production d'oligosaccharides à grande échelle, possédant des coûts de production économiquement viables et avec un faible impact environnemental. Cinq principaux types d'enzymes ont été décrits comme pouvant être utilisés pour catalyser efficacement la production d'oligosaccharides : les glycosides-hydrolases (GH), les glycosynthases (GS), les transglycosylases (TG), les glycoside-transférases (GT) et les glycoside-phosphorylases (GP). Toutefois chacune présente des inconvénients et limites.
- Les GH permettent d'hydrolyser plus ou moins spécifiquement les glycanes pour obtenir des mélanges d'oligosaccharides. Leur utilisation est néanmoins limitée à la conversion de polymères très abondants, comme ceux issus de la biomasse végétale, et, dans la majorité des cas, leur spécificité de produit n'est pas suffisante pour donner des fractions d'oligosaccharides monodisperses.
- Les GS sont des mutants de GH produites par ingénierie enzymatique. Elles nécessitent des substrats synthétiques onéreux.
- Les TG peuvent opérer la synthèse en utilisant des substrats bon marché (comme le saccharose ou l'amidon) mais leur diversité structurale et fonctionnelle naturelle est limitée au transfert d'un type très limité d'unités glycosyles.
- Les GT permettent la synthèse d'oligosaccharides à partir de substrats activés très coûteux (glycosyl-mono- ou diphosphonucleotides, et glycosyl-polyprenol mono- ou diphosphates), ce qui rend leur utilisation *in vitro* peu rentable.
- Les GP peuvent catalyser des réactions de synthèse d'oligosaccharides lors de la réaction de "phosphorolyse inverse"

La réaction dite de "phosphorolyse inverse" (Puchart, V., 2015. Glycoside phosphorylases: Structure, catalytic properties and biotechnological potential. Biotechnology Advances 33, 261-276. https://doi.org/10.1016/j.biotechadv.2015.02.002 [21]), formant une liaison osidique entre l'unité glycosidique issue d'un glycosyl-phosphate, qui agit comme "donneur" de glycosyl, et un « accepteur » hydroxylé, est représentée sur le schéma ci-dessous. Cependant, sans élimination continue du produit de phosphorolyse inverse ou du phosphate inorganique libéré pour modifier l'équilibre réactionnel, les rendements de synthèse des oligosaccharides sont faibles et le coût élevé des glycosyl-phosphates reste un frein important à l'utilisation de ces enzymes dans des procédés industriels *in vitro.*

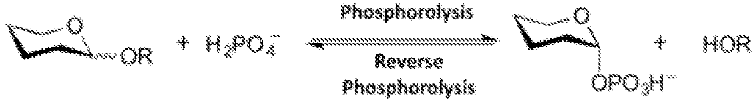

Il existe donc un réel besoin de trouver un moyen et/ou procédé permettant de maîtriser la production d'oligosaccharides, en particulier d'oligosaccharides à grande échelle, possédant des coûts de production économiquement viables et avec un faible impact environnemental.

Des procédés de synthèse *in vitro* basés sur l'utilisation de GS ont été décrits (Cobucci-Ponzano, B., Moracci, M., 2012. Glycosynthases as tools for the production of glycan analogs of natural products. Natural Product Reports 29, 697. [22]). Ils nécessitent des donneurs de glycosyl modifiés chimiquement qui sont difficiles à produire à haut rendement à un coût raisonnable. Les GT sont utilisées pour la production d'oligosaccharides, en particulier en utilisant des souches recombinantes *d'Escherichia coli* (Samain & Priem (2001) Procédé de production d'oligosaccharides WO0104341 [23] ; WO2008040717 Samain (2008) High yield production of sialic acid (Neu5ac) by fermentation [24]; WO2007101862 Samain (2007) Method of producing sialylated oligosaccharides [54]; WO2007023348, SAMAIN et al. (2007) Production of globoside oligosaccharides using metabolically engineered microorganisms [55] ; Fort, S., Birikaki, L., Dubois, M. P., Antoine, T., Samain, E., & Driguez, H. (2005). Biosynthesis of conjugatable saccharidic moieties of GM 2 and GM 3 gangliosides by engineered E. coli. Chemical communications, (20), 2558-2560 [56]; Fierfort, N., & Samain, E. (2008). Genetic engineering of Escherichia coli for the economical production of sialylated oligosaccharides. Journal of biotechnology, 134(3-4), 261-265) [57].

Certains de ces procédés connus utilisent des GP dans des souches recombinantes dans le sens de la phosphorolyse, pour augmenter la concentration intracellulaire de monosaccharides phosphorylés.

Il existe donc un réel besoin de trouver un moyen et/ou produit et/ou procédé simple et/ou ne comprenant pas d'étapes complexes et/ou susceptibles d'être mises en oeuvre uniquement par un expert, de production d'oligosaccharides notamment *in cellulo via* un micro-organisme adapté.

Il existe également un réel besoin de trouver un moyen et/ou produit et/ou procédé permettant de maîtriser la production d'oligosaccharides en particulier le type d'oligosaccharides produits, et également de réduire les coûts, notamment de production, tout en améliorant les rendements et la quantité d'oligosaccharides produits.

### Description de l'invention

La présente invention permet de résoudre et de surmonter les obstacles et inconvénients de l'art antérieur précités en fournissant une souche déposée le 26 février 2020 auprès de la CNCM (Collection Nationale de Culture de Microorganismes, Institut Pasteur, 25 rue du Docteur Roux, 75724 Paris Cedex 15, France), sous le numéro CNCM I-5499.

La présente invention permet de résoudre et de surmonter les obstacles et inconvénients de l'art antérieur précités en fournissant les souches déposées auprès de la CNCM (Collection Nationale de Culture de Microorganismes, Institut Pasteur, 25 rue du Docteur Roux, 75724 Paris Cedex 15, France), sous le numéros CNCM I-5499, CNCM I-5681 et CNCM I-5682.

En particulier, les inventeurs ont élaboré avantageusement un nouveau moyen de production d'oligosaccharides. En particulier, la souche selon l'invention permet la production d'oligosaccharides *via* des glycoside-phosphorylases et également avantageusement à la fois une internalisation de précurseurs pour la synthèse, en particulier d'hydrates de carbone non phosphorylés, et une accumulation intracellulaire d'hydrates de carbone phosphorylés.

En particulier, les inventeurs ont démontré de manière surprenante et inattendue que la souche selon l'invention permet avantageusement notamment d'accumuler des hydrates de carbone non phosphorylés requis pour la synthèse d'oligosaccharides par les glycoside-phosphorylases. Les inventeurs ont également démontré de manière surprenante que la souche selon l'invention permet avantageusement, lors de la production d'oligosaccharides, l'excrétion desdits oligosaccharides dans le milieu de culture. Avantageusement, les inventeurs ont démontré que la souche selon l'invention permet une récupération et une purification facilitée des oligosaccharides produits de par leur excrétion dans le milieu de culture.

En outre, avantageusement la diffusion ou excrétion des oligosaccharides dans le milieu extracellulaire pourrait permettre l'obtention d'une réaction catalysée par les glycoside-phosphorylases favorisée dans le sens de la phosphorolyse inverse.

La présente invention permet avantageusement, notamment par rapport aux synthèses chimiques d'oligosaccharides connues, une production d'oligosaccharides qui réduit énormément l'impact environnemental.

Les inventeurs ont ainsi démontré que l'invention permet avantageusement de fournir un nouveau procédé et/ou une nouvelle voie de synthèse d'oligosaccharides qui surmonte les inconvénients rencontrés dans les procédés connus de synthèse d'oligosaccharides, notamment dans les synthèses enzymatiques *in vitro* sans utilisation de support biologique vivant.

La présente invention permet comme évoqué ci-dessus et de manière synergique de réduire considérablement les coûts de production, le nombre d'étapes de synthèse et de purification par rapport aux procédés de synthèse, notamment chimique et enzymatique *in vitro* sans utilisation de support biologique vivant.

La présente invention se rapporte à une souche *Escherichia coli* dont les gènes recA1, gyrA96, thi-1, glnV44 relA1 hsdR17, endA1, lacZ, nanKETA, lacA, melA, wcaJ, mdoH, ptsG, manX, manY, et pfkA sont inactivés. Selon l'invention, il peut s'agir par exemple de la souche déposée auprès de la CNCM (Collection Nationale de Culture de Microorganismes, Institut Pasteur, 25 rue du Docteur Roux, 75724 Paris Cedex 15, France), sous le numéro CNCM I-5499 qui est une souche dérivée de *Escherichia coli* en ce que les gènes recA1, gyrA96, thi-1, glnV44 relA1 hsdR17, endA1, lacZ, nanKETA, lacA, melA, wcaJ, mdoH, ptsG, manX, manY, et pfkA sont inactivés

Selon l'invention, la souche déposée auprès de la CNCM (Collection Nationale de Culture de Microorganismes, Institut Pasteur, 25 rue du Docteur Roux, 75724 Paris Cedex 15, France), sous le numéro CNCM I-5499 est une souche dérivée de Escherichia coli dont les gènes recA1, gyrA96, thi-1, glnV44 relA1 hsdR17, endA1, lacZ, nanKETA, lacA, melA, wcaJ, mdoH, ptsG, manX, manY, et pfkA sont inactivés

Les inventeurs ont également démontré de manière surprenante que la mutation à savoir l'inactivation additionnelle d'au moins un des gènes maa et manA, de préférence l'inactivation des gènes maa et manA permet avantageusement une augmentation du rendement de production d'oligosaccharides et d'éviter la production d'éventuel produit parasite et/ou considéré comme contaminant, par exemple comme des sucres acétylés.

Aussi la souche selon l'invention peut comprendre en outre les mutations Δmaa et ΔmanA.

Dans la présente par Δmaa on entend l'inactivation du gène maa.

Dans la présente par ΔmanA on entend l'inactivation du gène manA.

Dans la présente, l'inactivation d'un gène peut être effectué par tout procédé adapté connu de l'homme du métier. Il peut s'agir par exemple du procédé décrit dans Kirill A. Datsenko, Barry L. Wanner. One-step inactivation of chromosomal genes in Escherichia coli K-12 using PCR products. Proceedings of the National Academy of Sciences Jun 2000, 97 (12) 6640-6645; DOI: 10.1073/pnas.120163297 [62].

La présente invention a également pour objet, la souche déposée le 7 mai 2021 auprès de la CNCM (Collection Nationale de Culture de Microorganismes, Institut Pasteur, 25 rue du Docteur Roux, 75724 Paris Cedex 15, France), sous le numéro CNCM I-5681 qui est une souche *Escherichia coli* dont les gènes recA1, gyrA96, thi-1, glnV44 relA1 hsdR17, endA1, lacZ, *nanKETA,* lacA, *melA, wcaJ, mdoH, ptsG, manX, manY, pfkA, maa et manA* sont inactivés.
Dans la présente invention, par recA1 on entend le gène codant pour la protéine RecA 1 (NCBI Gene ID: 947170 UniProtKB - P0A7G6)
Dans la présente par gyrA96 on entend le gène codant pour la sous-unité A de la DNA gyrase (NCBI GeneID:946614 UniProtKB - P0AES4)
Dans la présente invention, par thi-1 on entend le gène codant pour la Thiamine thiazole synthase, chloroplastic (NCBI GeneID:948491 UniProtKB P30137)
Dans la présente invention, par glnV44 on entend le gène codant pour un glutamine tRNA (NCBI GeneID:945255).

Dans la présente invention, par relA1 on entend le gène codant pour la GTP pyrophosphokinase (NCBI GeneID:947244 UniProtKB - P0AG20).
Dans la présente invention, par hsdR17 on entend un mutant du gène hsdR codant pour un composant endonucléase de l'enzyme de restriction de type I EcoR124II R protéine (NCBI GeneID: 948878 UniProtKB - P10486)
Dans la présente invention, par endA1 on entend le gène codant pour l'endonucléase EndA1 (NCBI GeneID:949092 UniProtKB - P25736)
Dans la présente invention, par lacZ on entend le gène codant pour la β-galactosidase (NCBI GeneID: 945006 UniProtKB - P00722)
Dans la présente invention, par nanKETA on entend les gènes codant pour l'opéron permettant une internalisation et la métabolisation de l'acide sialique (Neu5Ac), en particulier les gènes : nanA codant pour une N-acétylneuraminate lyase (NCBI GeneID:947742 UniProtKB - P0A6L4); *nanT* codant pour la *N*-acétylneuraminate:H⁺ symporter ( NCBI GeneID:947740; UniProtKB - P41036); *nanE* codant pour la *N*-acétylmannosamine-6-phosphate épimérase (NCBI GeneID:947745 UniProtKB - P0A761) ; nanK codant pour la *N*-acétylmannosamine kinase (NCBI GeneID:947757 UniProtKB - P45425).

Dans la présente invention, par lacA on entend le gène codant pour la galactoside O-acétyltransférase (NCBI GeneID: 945674 UniProtKB - P07464).

Dans la présente invention, par melA on entend le gène codant pour l'α-galactosidase (NCBI eneID:948636 UniProtKB - P06720).

Dans la présente invention, par wcaJ on entend le gène codant pour l'UDP-glucose:undecaprényl-phosphate glucose-1-phosphate transférase (NCBI GeneID:946583 UniProtKB - P71241).

Dans la présente invention, par mdoH on entend le gène codant pour l'osmorégulé périplasmique glucanes (OPGs) biosynthèses protéine H (« osmoregulated periplasmic glucans OPGs biosynthesis protein H ») (NCBI GeneID:945624 UniProtKB - P62517).

Dans la présente invention, par ptsG on entend le gène codant pour la protéine glucose-spécifique PTS enzyme IIBC component (« glucose-specific PTS enzyme IIBC component ») (NCBI GeneID: 945651 UniProtKB - P69786).

Dans la présente invention, par manX on entend le gène codant pour la protéine mannose-spécifique PTS enzyme IIAB component (NCBI GeneID: 946334 UniProtKB - P69797).

Dans la présente invention, par manY on entend le gène codant pour la protéine mannose-spécifique PTS enzyme IIC component (NCBI GeneID: 946332 UniProtKB - P69801)
Dans la présente invention, par pfkA on entend le gène codant pour l'ATP-dépendent 6-phosphofructokinase isozyme 1 (NCBI GeneID: 948412 UniProtKB - P0A796).

Dans la présente invention, par maa on entend le gène codant pour le Maltose O-acetyltrasferase (NCBI GeneID : 414992 UniProtKB-P77791) ; Dans la présente invention, par manA on entend le gène codant pour le Mannose-6-phosphate isomerase (NCBI GeneID : 416130 UniProtKB-P00946);
Selon l'invention, la souche peut être en outre modifiée génétiquement, par tout procédé adapté connu de l'homme du métier. Il peut s'agir par exemple d'au moins une mutation, délétion et/ou substitution.

Selon l'invention, la souche peut par exemple être modifiée génétiquement. Il peut s'agir par exemple d'une modification génétique de l'expression d'un gène. Par exemple la souche peut comprendre la substitution d'un promoteur. Par exemple, la souche peut comprendre la substitution du promoteur du gène *galP* par un promoteur constitutif. Il peut s'agir par exemple de tout promoteur constitutif adapté connu de l'homme du métier. Il peut s'agir par exemple du promoteur du gène codant pour IHF également désigné promoteur HIF, par exemple tel que décrit dans Zhou, K., Zhou, L., Lim, Q. 'En, Zou, R., Stephanopoulos, G., and Too, H.-P. (2011) Novel reference genes for quantifying transcriptional responses of Escherichia coli to protein overexpression by quantitative PCR. BMC Mol Biol 12: 18 [65]. Il peut s'agir par exemple du promoteur du gène codant pour IHF (« Intégration host factor ») NCBI GeneID 415432, UniProtKB-P0A6Y1). Il peut s'agir par exemple du promoteur IHF de séquence 
TCTGAAACAGTGCAAAGCTAACCCGTGGCAGCAGTTCGCGGAAACCC
ACAACAAGGGCGACCGTGTTGAAGGTAAAATCAAGTCTATCACTGACT
TCGGTATCTTCATCGGCTTGGACGGCGGCATCGACGGCCTGGTTCAC
CTGTCTGACATCTCCTGGAACGTTGCAGGCGAAGAAGCAGTTCGTGA
ATACAAAAAAGGCGACGAAATCGCTGCAGTTGTTCTGCAGGTTGACG
CAGAACGTGAACGTATCTCCCTGGGCGTTAAACAGCTCGCAGAAGAT
CCGTTCAACAACTGGGTTGCTCTGAACAAGAAAGGCGCTATCGTAAC
CGGTAAAGTAACTGCAGTTGACGCTAAAGGCGCAACCGTAGAACTGG
CTGACGGCGTTGAAGGTTACCTGCGTGCTTCTGAAGCATCCCGTGAC
CGCGTTGAAGACGCTACCCTGGTTCTGAGCGTTGGCGACGAAGTTGA
AGCTAAATTCACCGGCGTTGATCGTAAAAACCGCGCAATCAGCCTGT
CTGTTCGTGCGAAAGACGAAGCTGACGAGAAAGATGCAATCGCAACT
GTTAACAAACAGGAAGATGCAAACTTCTCCAACAACGCAATGGCTGAA
GCTTTCAAAGCAGCTAAAGGCGAGTAATTCTCTGACTCTTCGGGATTT
TTATTCCGAAGTTTGTTGAGTTTACTTGACAGATTGCAGGTTTCGTCCT
GTAATCAAGCACTAAGGGCGGCTACGGCCGCCCTTAATCAATGCAGC
 
AACAGCAGCCGCTTAATTTGCCTTTAAGGAACCGGAGGAATC (SEQ ID
NO : 66).
 Avantageusement lorsque la souche comprend un promoteur constitutif pour le gène *galP,* les inventeurs ont démontré une éventuelle augmentation du rendement de production d'oligosaccharides.

Dans la présente, la substitution d'une séquence d'ADN peut être effectuée par tout procédé adapté connu de l'homme du métier. Il peut s'agir par exemple du protocole Datsenko et Wanner (Kirill A. Datsenko, Barry L. Wanner. One-step inactivation of chromosomal genes in *Escherichia coli* K-12 using PCR products. Proceedings of the National Academy of Sciences Jun 2000, 97 (12) 6640-6645; DOI: 10.1073/pnas.120163297 [62]).

La présente invention a également pour objet, la souche déposée le 7 mai 2021 auprès de la CNCM (Collection Nationale de Culture de Microorganismes, Institut Pasteur, 25 rue du Docteur Roux, 75724 Paris Cedex 15, France), sous le numéro CNCM I-5682 qui est une souche *Escherichia coli* dont les gènes recA1, gyrA96, thi-1, glnV44 relA1 hsdR17, endA1, lacZ, *nanKETA,* lacA, *melA, wcaJ, mdoH, ptsG, manX, manY, pfkA, maa et manA* sont inactivés, et comprenant un promoteur constitutif, de préférence le promoteur IHF de séquence SEQ ID NO : 66, pour le gène GalP.

Selon l'invention, la souche peut comprendre au moins un vecteur d'expression.

Dans la présente invention, par vecteur d'expression on entend tout vecteur d'expression connu de l'homme du métier adapté, notamment, pour l'expression de gènes codant pour des protéines dans des microorganismes. Il peut s'agir de tout vecteur d'expression connu de l'homme du métier adapté pour l'expression de gènes codant pour des protéines dans une bactérie. Il peut s'agir par exemple de n'importe quel vecteur sélectionné parmi les vecteurs listés dans le catalogue https://france.promega.com/products/vectors/protein-expression-vectors [35] ou dans le catalogue https://www.thermofisher.com/search/browse/category/fr/fr/85801/Bacterial %20Expression%20Vectors [36]. Il peut s'agir, par exemple, du vecteur d'expression décrit dans le document WO 83/004261 [37]. Le vecteur peut être, par exemple un plasmide, un chromosome artificiel de levure (YAC) ou un chromosome artificiel bactérien (BAC). Il peut s'agir par exemple de tout plasmide connu de l'homme du métier adapté pour l'expression de gènes codant pour des protéines dans des bactéries et/ou disponible dans le commerce. Il peut s'agir par exemple du plasmide pWKS130 décrit dans le document Rong Fu Wang, Kushner, S.R., 1991. Construction of versatile low-copy-number vectors for cloning, sequencing and gene expression in Escherichia coli. Gene 100, 195-199. [10], du plasmide commercialisé par la société Thermofisher sous la référence commerciale pBAD-HisA, du plasmide commercialisé par la société Thermofisher sous la référence commerciale pTRC. Il peut s'agir par exemple d'un plasmide choisi dans le groupe comprenant pBAD-HisA, pTRC, pMX, pUC19, pHP45-CmR, pcDNA3.1(+), pcDNA3.3-TOPO, pcDNA3.4-TOPO, pFastBac1, pET100/D-TOPO, pET151/D-TOPO, pRSET A, pYes2.1V5-His TOPO, pDONR221, pGEX-3X ; pBluescript, pET, pGEX, pLys, pRARE, pDEST, pETG, de préférence le plasmide pBAD-HisA. Il peut s'agir par exemple de tout vecteur d'expression mentionné, par exemple dans les pages internet https://www.embl.de/pepcore/pepcore_services/strains_vectors/vectors/ba cterial expression vectors/popup_bacterial expression vectors/index.html [38],
http://babel.ucmp.umu.se/cpep/web_content/Pages/CPEP_09_vectors.htm I [39] et/ ou https://www.embl.de/pepcore/pepcore_services/strains_vectors/vectors/gat eway_vectors/index.htm [40]

Le vecteur peut être n'importe quel chromosome artificiel de levure (YAC) adapté pour l'expression dans des bactéries, connu de l'homme du métier et/ou disponible dans le commerce. Il peut s'agir par exemple d'un chromosome artificiel de levure choisi dans le groupe comprenant pYAC-RC, pYAC3.

Le vecteur peut être n'importe quel chromosome artificiel bactérien (BAC) adapté pour l'expression dans des bactéries, connu de l'homme du métier et/ou disponible dans le commerce. Il peut s'agir par exemple d'un chromosome artificiel bactérien choisi dans le groupe comprenant pUvBBAC, pCC1BAC, pBAC 108L.

Selon l'invention, au moins un vecteur d'expression peut permettre l'expression d'au moins une glycoside-phosphorylase. Par exemple, le vecteur d'expression peut permettre l'expression d'une glycoside-phosphorylase choisie parmi les β-glycoside- ou α-glycoside-phosphorylases, par exemple les α-1,3-glucopyranosyl-L-rhamnose-phosphorylases, les α-1,2-glucosyl-glycérol-phosphorylases, les tréhalose-phosphorylases, les laminaribiose-phosphorylases, les D-galactosyl-β-1,4-L-rhamnose phosphorylases, les β-1,4-mannosyl-glucose-phosphorylases, les β-1,2-oligomannan-phosphorylases, les β-1,2-mannobiose-phosphorylases, les β-1,4-mannopyranosyl-[N-glycane]-phosphorylases / les β-1,4-mannopyranosyl-chitobiose-phosphorylases, les β-1,4-mannooligosaccharide-phosphorylases, les β-1,3-mannooligosaccharide-phosphorylases, les β-1,3-mannosyl-glucose phosphorylases, les β-1,4-mannosyl-glucuronate phosphorylases.

Par exemple, le vecteur d'expression peut permettre l'expression d'une glycoside-phosphorylase telle que mentionnée dans le tableau 1 ci-dessous. Le tableau 1 décrit des exemples de glycoside-phosphorylases et mentionne leur identifiant GenBank ou Gigabase.

**Tableau 1: Glycoside-phosphorylases et numéros d'accession GenBank ou Gigabase**

| **Glycoside-phosphorylase** | **GenBank** |
|---|---|
| 3-O-α-glucopyranosyl-L-rhamnose phosphorylase (Cphy_1019) | ABX41399.1 |
| 1,2-alpha-glucosylglycérol phosphorylase (Bsel_2816) | ADI00307.1 |
| tréhalose phosphorylase (TreP) | BAB97299.1 |
| tréhalose phosphorylase (TPase) | BAC20640.1 |
| D-galactosyl-1,4-L-rhamnose phosphorylase (Cphy_1920) | ABX42289.1 |
| D-galactosyl-3-1,4-L-rhamnose phosphorylase (GalRhaP;Oter_1377) | ACB74662.1 |
| β-1,4-mannosylglucose phosphorylase (Unk1) | AAS19693.1 |
| β-1,2-oligomannane phosphorylase (Teth514_1788) | ABY93073.1 |
| β-1,2-mannobiose phosphorylase (Teth514_1789) | ABY93074.1 |
| β-1,4-mannopyranosyl-[N-glycane] phosphorylase / β-1,4-mannopyranosyl-chitobiose phosphorylase (Uhgb_MP) | ADD61463.1 |
| β-1,4-mannooligosaccharide phosphorylase (MOP; RaM P2; Rumal_0099) | ADU20661.1 |
| β-1,4-mannosylglucose phosphorylase (RaMP1;RaMGP;Rumal_0852) | ADU21379.1 |
| β-1,2-mannobiose phosphorylase (Lin0857) | CAC96089.1 |
| β-1,4-mannosylglucose phosphorylase (MGP;BF0772) | CAH06518.1 |
| β-1,3-mannooligosaccharide phosphorylase (zobellia_231) | CAZ94304.1 |
| CalpoDRAFT_0075 | WP_026485574.1 |
| CalpoDRAFT_1209 | WP_026486530.1 |
| Tréhalose phosphorylase | AAF22230.1 |
| Tréhalose phosphorylase | ABC84380.1 |
| Tréhalose phosphorylase | BAA31350.1 |
| β-1,4-mannooligosaccharide phosphorylase (RIL182 01100;ROSINTL182_05474) | VCV21229.1 |
| mannosylglucose phosphorylase (RIL182 01099;ROSINTL182_07685) | VCV21228.1 |
| β-1,2-oligomannane phosphorylase MTP3 (LMXM_10_1250) | XP_003872966.1 |
| | CBZ24448.1 |
| β-1,2-oligomannane phosphorylase MTP4 (LMXM_10_1260) | CBZ24449.1 |
| | XP_003872967.1 |
| β-1,2-oligomannane phosphorylase MTP6 (LMXM_10_1280) | CBZ24451.1 |
| | XP_003872969.1 |
| β-1,2-oligomannane phosphorylase MTP7 (LMXM_10_1290) | CBZ24452.1 XP_003872970.1 |
| laminaribiose phosphorylase (ACL_0729) | ABX81345.1 |
| laminaribiose phosphorylase (LbpA) | BAJ10826.1 |

| **Glycoside-phosphorylase** | **GigaDB, DOI: 10.5524/100064** |
|---|---|
| β-1,4-mannosyl-glucuronate phosphorylase (« β-1,4-mannosyl-glucuronic acid phosphorylase ») | MH0431_GL0150624 |
| β-1,4-mannooligosaccharide phosphorylase | MH0373_GL0093988 |
| β-1,3-mannosyl-glucose phosphorylase β-1,3-mannooligosaccharide phosphorylase | 340101.Vvad_PD3074 |

Un vecteur d'expression peut comprendre une séquence polynucléotidique, par exemple une cassette génétique (« expression cassette »), comprenant les éléments suivants dans l'ordre 5' à 3' : une séquence promotrice; une séquence d'acide nucléique codant une enzyme, de préférence une glycoside-phosphorylase.

Dans le présent document, "polynucléotide(s)", "oligonucléotide(s)", "acide(s) nucléique(s)", "nucléotide(s)", "acide(s) polynucléique(s)" ou tout équivalent grammatical utilisé fait référence à une forme polymère de nucléotides ou d'acides nucléiques de toute longueur, soit ribonucléotides ou désoxyribonucléotides. Ce terme fait référence à la structure primaire de la molécule. Ainsi, ce terme comprend l'ADN double et simple brin, l'ADN triplex, ainsi que l'ARN double et simple brin. Elle comprend également les formes modifiées, par exemple par méthylation et/ou par coiffage, et les formes non modifiées du polynucléotide. Le terme est également destiné à comprendre les molécules qui comprennent les nucléotides non naturels ou synthétiques ainsi que les analogues nucléotidiques. Les séquences d'acides nucléiques et les vecteurs divulgués ou envisagés ici peuvent être introduits dans une cellule, par exemple par transfection, transformation ou transduction.

La glycoside-phosphorylase peut être une glycoside-phosphorylase telle que définie ci-dessus.

Le promoteur peut être tout promoteur connu de l'homme du métier adapté pour une expression dans des microorganismes, de préférence dans des bactéries. Il peut s'agir par exemple d'un promoteur constitutif ou d'un promoteur inductible. Il peut s'agir par exemple d'un promoteur constitutif choisi dans le groupe comprenant le promoteur CMV, EF1A, ou SV40. Il peut s'agir par exemple d'un promoteur constitutif mentionné sur la page internet http://parts.igem.org/Promoters/Catalog/Constitutive [41], d'un quelconque promoteur de la souche *E. coli* K-12 connu de l'homme du métier, par exemple un promoteur choisi parmi les 3908 promoteurs de la souche *E. coli* K-12 répertoriés sur le site biocyc.org: https://biocyc.org/group?id=:ALL-PROMOTERS&orgid=ECOLI [42]. Il peut s'agir par exemple d'un promoteur inductible, par exemple pBAD ou pLac. Il peut s'agir par exemple d'un promoteur inductible décrit et/ou mentionné sur la page : http://parts.igem.org/Promoters/Catalog [43].

L'homme du métier, de part ces connaissances générales saura choisir le promoteur en fonction de la séquence nucléique à exprimer et/ou de la cellule hôte, de préférence la souche selon l'invention, par exemple la déposée à la CNCM (Collection Nationale de Culture de Microorganismes, Institut Pasteur, 25 rue du Docteur Roux, 75724 Paris Cedex 15, France), sous le numéro CNCM I-5499, ou sous le numéro CNCM I-5681, ou sous le numéro CNCM I-5682.

Dans la présente invention, l'insertion de la séquence nucléique codante peut être réalisée par tout procédé adapté connu de l'homme du métier. Il peut s'agir par exemple de toute méthode de clonage de gènes connue de l'homme du métier. Il peut s'agir par exemple d'un procédé décrit et/ou disponible sur la page internet https://www.addgene.org/mol-bio-reference/cloning [31]. L'homme du métier, de part ces connaissances générales, saura adapter les procédés et/ou méthodes d'insertion et/ou de clonage en fonction du vecteur et/ou du gène.

Dans la présente invention, la souche peut comprendre en outre au moins un vecteur d'expression permettant l'expression d'au moins un système de transport, de préférence de monosaccharides ou d'oligosaccharides. Selon l'invention le vecteur d'expression peut être tel que défini ci-dessus. Dans la présente invention, par système de transport on entend toute protéine et/ou ensemble de protéines de transport et/ou transporteur protéique adapté pour le transport de monosaccharides connu de l'homme du métier. Il peut s'agir par exemple d'une protéine de transport ou perméase d'hydrates de carbone non phosphorylés, de préférence de monosaccharides, choisie parmi les familles de transporteurs comprenant les transporteurs « phosphotransferase system » (PTS)). Il peut s'agir par exemple d'un transporteur non-PTS choisi parmi les transporteurs de la famille des « Major Facilitor Superfamily » (MFS), de la famille des sodium-glucose transporteur (SGLT), de la famille Ton-B dépendant et/ou de la super famille des transporteurs « ATP-Binding Cassette » (ABC).

Il peut s'agir par exemple d'un transporteur de la famille des « Major Facilitor Superfamily » (MFS) choisi dans le groupe comprenant la Galactose perméase (GaIP), par exemple la Galactose perméase (GaIP) d'E. *coli* ou *Salmonella sp,* la Glucose perméase (GIcP), par exemple la Glucose perméase (GIcP) de *Streptomyces coelicolor* ou la glucose perméase (Glf) de *Zymomonas mobilis.*

Il peut s'agir par exemple d'un transporteur de la famille des sodium-glucose transporteur (SGLT) choisi dans le groupe comprenant le transporteur SgIS, par exemple le transporteur SglS de la souche *Vibrio parahemoliticus.*

Il peut s'agir par exemple d'un transporteur de la super famille des transporteurs « ATP-Binding Cassette » (ABC) choisi dans le groupe comprenant le transporteur MglABC, par exemple le transporteur MglABC d'*E*. *coli,* le transporteur malEFG, par exemple le transporteur malEFG de *Thermos termophilus,* le transporteur Teth514_1792, par exemple le transporteur Teth514_1792 de *Thermoanaerobacter sp,* ou le transporteur Teth514_1796, par exemple le transporteur Teth514_1796 de *Thermoanaerobacter sp* X-514.

Dans la présente invention, un vecteur d'expression peut permettre la production d'au moins un système de transport tel que mentionné ci-dessus. Par exemple, le vecteur d'expression peut permettre l'expression d'une perméase ou protéine de transport telle que mentionnée dans le tableau 2 ci-dessous.

Le tableau 2 décrit des exemples de perméase ou protéine de transport et mentionne leur famille.

**Tableau 2 : perméase ou protéine de transport et famille de transporteurs correspondant**

| **Perméase ou protéine de transport** | **Famille** |
|---|---|
| Galactose perméase (GaIP) E. coli K12 | MFS |
| Galactose perméase (GaIP) Salmonella sp | MFS |
| Glucose perméase (GlcP) Streptomyces coelicolor | MFS |
| Glucose perméase facteur facilité ("Glucose facilitated factor permease") (glf) Z. mobilis | MFS |
| Sodium-glucose transporteur SglS de Vibrio parahemoliticus | SGLT |
| MglABC de E. coli | ABC |
| malEFG of Thermos termophilus | ABC |
| Teth514_1792 | ABC |
| Teth514_1796 | ABC |

Dans la présente invention, un vecteur d'expression permettant l'expression d'au moins un gène codant pour une protéine de transport ou perméase peut comprendre une séquence polynucléotidique, par exemple une cassette génétique (« expression cassette »), comprenant les éléments suivants dans l'ordre 5' à 3' : une séquence promotrice; une séquence d'acide nucléique codant une protéine, de préférence une protéine de transport ou perméase ;
La protéine de transport ou perméase peut être une protéine de transport ou perméase telle que définie ci-dessus.

Selon l'invention, le promoteur peut être tel que défini ci-dessus.

L'homme du métier, de part ces connaissances générales saura choisir le promoteur en fonction de la séquence nucléique à exprimer et/ou de la cellule hôte, de préférence la souche selon l'invention, par exemple la déposée à la CNCM (Collection Nationale de Culture de Microorganismes, Institut Pasteur, 25 rue du Docteur Roux, 75724 Paris Cedex 15, France), sous le numéro CNCM I-5499, ou sous le numéro CNCM I-5681, ou sous le numéro CNCM I-5682.

Dans la présente invention, l'insertion de la séquence nucléique codant la protéine de transport ou perméase peut être réalisée par tout procédé tel que décrit ci-dessus.

Avantageusement, lorsque la souche comprend en outre un vecteur d'expression permettant l'expression d'au moins un gène codant pour une protéine de transport ou perméase, la souche selon l'invention peut avantageusement internaliser des sucres non phosphorylés qui peuvent être présents dans le milieu de culture.

Avantageusement, tel que mentionné ci-dessus, les inventeurs ont démontré de manière surprenante que la souche selon l'invention permet avantageusement d'accumuler des précurseurs de synthèse d'oligosaccharides, et peut avantageusement être utilisée comme un support et/ou moyen pour la production d'oligosaccharides.

Tel que mentionné ci-dessus, les inventeurs ont démontré de manière surprenante que la souche selon l'invention peut permettre avantageusement après production d'oligosaccharides une diffusion ou excrétion desdits oligosaccharides produits dans le milieu de culture.

En outre, avantageusement la diffusion ou excrétion des oligosaccharides dans le milieu extracellulaire pourrait permettre l'obtention d'une réaction catalysée par les glycoside-phosphorylases dans le sens de la phosphorolyse inverse.

La présente invention a donc également pour objet l'utilisation *in vitro* d'une souche selon l'invention pour la production d'oligosaccharides et/ou dans un procédé de production d'oligosaccharides.

Dans la présente invention, la souche peut être utilisée dans tout procédé *in vitro,* en particulier *in cellulo,* de production d'oligosaccharides, connu de l'homme du métier. Il peut s'agir de tout procédé de production d'oligosaccharides comprenant une synthèse enzymatique et/ou utilisant au moins un microorganisme. Il peut s'agir de tout procédé de production d'oligosaccharides connu comprenant une synthèse enzymatique et/ou utilisant au moins un microorganisme dans lequel l'enzyme ou le microorganisme peut être remplacé par la souche selon l'invention. Il peut s'agir par exemple d'un procédé de production d'oligosaccharides comprenant une synthèse enzymatique et/ou utilisant au moins un microorganisme, par exemple tel que décrit dans le document : Tathiana Souza Martins Meyer, Ângelo Samir Melim Miguel, Daniel Ernesto Rodriguez Fernández and Gisela Maria Dellamora Ortiz « Biotechnological Production of Oligosaccharides - Applications in the Food Industry » October 22nd 2015 [17], Erju Tang, Xialin Shen, Jia Wang, Xinxiao Sun, Qipeng Yuan, "Synergetic utilization of glucose and glycerol for efficient myo-inositol biosynthesis" Biotechnology and Bioengineering 2020 [32], Constantin Ruprecht, Friedericke Bönisch, Nele Ilmbergera, Tanja V. Heyera, Erhard T.K. Haupt, Wolfgang R. Streit, Ulrich Rabausch "High level production of flavonoid rhamnosides by metagenome-derived Glycosyltransferase C in Escherichia coli utilizing dextrins of starch as a single carbon source", Metabolic Engineering Volume 55, September 2019, Pages 212-219 [33], Tatiana Antoine, Alain Heyraud Dr., Claude Bosso Dr., Eric Samain Dr "Highly Efficient Biosynthesis of the Oligosaccharide Moiety of the GD3 Ganglioside by Using Metabolically Engineered Escherichia coli", Angewandte Chemie, Volume117, Issue9, February 18, 2005 Pages 1374-1376 [34].

Les inventeurs ont également démontré de manière surprenante que la souche selon l'invention permet avantageusement de produire des oligosaccharides et de les excréter dans le milieu de culture. En outre, les inventeurs ont démontré que la souche selon l'invention peut permettre une production/bioproduction d'oligosaccharides en continu. Avantageusement, la présente invention peut permettre la production d'oligosaccharides, il peut s'agir par exemple des oligosaccharides mentionnés dans le tableau 3 ci-dessous. Par exemple la présente invention peut permettre la production d'oligosaccharides en fonction de la glycoside-phosphorylase utilisée tel que mentionnés dans le tableau 3 ci-dessous.

**Tableau 3 : oligosaccharides produit en fonction de la glycoside-phosphorylase**

| **GenBank** | **Glycoside-phosphorylase** | **Produit** |
|---|---|---|
| ABX41399.1 | 3-O-α-glucopyranosyl-L-rhamnose phosphorylase (Cphy_1019) | 3-O-α-glucopyranosyl-L-rhamnose |
| ABX42289.1 | D-galactosyl-1,4-L-rhamnose phosphorylase (Cphy_1920) | D-galactosyl-1,4-L-rhamnose |
| ACB74662.1 | D-galactosyl-β-1,4-L-rhamnose phosphorylase (GalRhaP;Oter_1377) | D-galactosyl-β-1,4-L-rhamnose |
| ADI00307.1 | 1,2-alpha-glucosylglycérol phosphorylase(Bsel_2816) | 1,2-alpha-glucosylglycérol |
| BAB97299.1 | tréhalose phosphorylase (TreP) | tréhalose |
| BAC20640.1 | tréhalose phosphorylase (TPase) | tréhalose |
| AAF22230.1 | tréhalose phosphorylase | tréhalose |
| ABC84380.1 | tréhalose phosphorylase | tréhalose |
| BAA31350.1 | tréhalose phosphorylase | tréhalose |
| AAS19693.1 | β-1,4-mannosyl-glucose phosphorylase (Unk1) | β-1,4-mannosyl-glucose |
| ABY93073.1 | β-1,2-oligomannane phosphorylase (Teth514_1788) | β-1,2-oligomannane |
| ABY93074.1 | β-1,2-mannobiose phosphorylase (Teth514_1789) | β-1,2-mannobiose |
| ADD61463.1 | β-1,4-mannopyranosyl-[N-glycane] phosphorylase (Uhgb_MP) | β-1,4-mannooligosaccha ride |
| ADU20661.1 | β-1,4-mannooligosaccharide phosphorylase (MOP;RaMP2;Rumal_0099) | β-1,4-mannooligosaccha ride |
| ADU21379.1 | β-1,4-mannosyl-glucose phosphorylase (RaMP1;RaMGP;Rumal_0852) | β-1,4-mannosyl-glucose |
| CAC96089.1 | β-1,2-mannobiose phosphorylase (Lin0857) | β-1,2-mannobiose |
| CAH06518.1 | β-1,4-mannosyl-glucose phosphorylase (MGP;BF0772) | β-1,4-mannosyl-glucose |
| CAZ94304.1 | β-1,3-mannooligosaccharide phosphorylase (zobellia_231) | β-1,3-mannooligosaccha ride |
| WP_026485574.1 | β-1,4-mannooligosaccharide phosphorylase (CalpoDRAFT_0075 ) | β-1,4-mannooligosaccha ride |
| WP_026486530.1 | β-1,4-mannooligosaccharide phosphorylase (CalpoDRAFT_1209) | β-1,4-mannooligosaccha ride |
| VCV21229.1 | β-1,4-mannooligosaccharide phosphorylase (RIL182_01100;ROSINTL182_0547 4) | β-1,4-mannooligosaccha ride |
| VCV21228.1 | Mannosyl-glucose phosphorylase (RIL182_01099;ROSINTL182_0768 5) | β-1,4-mannosyl-glucose |
| XP_003872966.1 CBZ24448.1 | β-1,2-oligomannane phosphorylase MTP3 (LMXM_10_1250) | β-1,2-oligomannane |
| CBZ24449.1 XP_003872967.1 | β-1,2-oligomannane phosphorylase MTP4 (LMXM 10_1260) | β-1,2-oligomannane |
| CBZ24451.1 XP_003872969.1 | β-1,2-oligomannane phosphorylase MTP6 (LMXM 10_1280) | β-1,2-oligomannane |
| CBZ24452.1 XP_003872970.1 | β-1,2-oligomannane phosphorylase MTP7 (LMXM 10_1290) | β-1,2-oligomannane |
| ABX81345.1 | laminaribiose phosphorylase (ACL_0729) | laminaribiose |
| BAJ10826.1 | laminaribiose phosphorylase (LbpA) | laminaribiose |

| **GigaDB accession number** (GigaDB, DOI: 10.5524/100064) | **Glycoside-phosphorylase** | **Produit** |
|---|---|---|
| MH0373_GL0093988 | β-1,4-mannooligosaccharide phosphorylase | β-1,4-mannooligosaccha ride |
| 340101.Vvad_PD3074 | β-1,3-mannosyl-glucose phosphorylase/ β-1,3-mannooligosaccharide phosphorylase | β-1,3-mannosyl-glucose |
| 340101.Vvad_PD3074 | β-1,3-mannosyl-glucose phosphorylase/ β-1,3-mannooligosaccharide phosphorylase | β-1,3-mannooligosaccha ride |
| MH0431_GL0150624 | β-1,4-mannosyl-glucuronate phosphorylase | β-1,4-mannosyl-glucuronate |

La présente invention a donc également pour objet un procédé *in vitro,* en particulier *in cellulo,* de production d'oligosaccharides comprenant les étapes de
a) transformation d'une souche selon l'invention avec un vecteur d'expression d'une enzyme, de préférence une glycoside-phosphorylase,
b) culture de la souche transformée dans un milieu de culture,
c) récupération des oligosaccharides produits.

La présente invention a également pour objet un procédé *in vitro,* en particulier *in cellulo,* de production d'oligosaccharides comprenant les étapes de
a) transformation de la souche déposée à la CNCM (Collection Nationale de Culture de Microorganismes, Institut Pasteur, 25 rue du Docteur Roux, 75724 Paris Cedex 15, France), sous le numéro CNCM I-5499 avec un vecteur d'expression d'une enzyme, de préférence une glycoside-phosphorylase,
b) culture de la souche transformée dans un milieu de culture,
c) récupération des oligosaccharides produits.

La présente invention a en outre pour objet un procédé *in vitro,* en particulier *in cellulo,* de production d'oligosaccharides comprenant les étapes de
a) transformation de la souche déposée auprès de la CNCM (Collection Nationale de Culture de Microorganismes, Institut Pasteur, 25 rue du Docteur Roux, 75724 Paris Cedex 15, France), sous le numéro CNCM I-5499 comprenant en outre les mutations Δmaa et ΔmanA. avec un vecteur d'expression d'une enzyme, de préférence une glycoside-phosphorylase,
b) culture de la souche transformée dans un milieu de culture,
c) récupération des oligosaccharides produits.

La présente invention a en outre également pour objet un procédé *in vitro,* en particulier *in cellulo,* de production d'oligosaccharides comprenant les étapes de
a) transformation la souche déposée auprès de la CNCM (Collection Nationale de Culture de Microorganismes, Institut Pasteur, 25 rue du Docteur Roux, 75724 Paris Cedex 15, France), sous le numéro CNCM I-5681 avec un vecteur d'expression d'une enzyme, de préférence une glycoside-phosphorylase,
b) culture de la souche transformée dans un milieu de culture,
c) récupération des oligosaccharides produits.

La présente invention a en outre également pour objet un procédé *in vitro,* en particulier *in cellulo,* de production d'oligosaccharides comprenant les étapes de
a) transformation la souche déposée auprès de la CNCM (Collection Nationale de Culture de Microorganismes, Institut Pasteur, 25 rue du Docteur Roux, 75724 Paris Cedex 15, France), sous le numéro CNCM I-5682 avec un vecteur d'expression d'une enzyme, de préférence une glycoside-phosphorylase,
b) culture de la souche transformée dans un milieu de culture,
c) récupération des oligosaccharides produits.

Dans la présente invention, par "transfection", "transformation" ou "transduction" désigne l'introduction d'un ou plusieurs polynucléotides exogènes dans une cellule hôte par des méthodes physiques ou chimiques. Il peut s'agir de n'importe quelle méthode de transfection et/ou de transformation et/ou de transduction adaptée connue de l'homme du métier. Il peut s'agir par exemple d'un procédé décrit dans Murray E. J. (éd.), Methods in Molecular Biology, Vol. 7, Gene Transfer and Expression Protocols, Humana Press (1991) [25]) à savoir une coprécipitation d'ADN calcium phosphate, DEAE- dextran ; électroporation ; transfection par liposomes cationiques ; transfection par liposomes ; bombardement par microparticules facilitées de particules de tungstène (Johnston, Nature, 346 : 776-777 (1990) [26]) ; et la co-précipitation de l'ADN du phosphate de strontium (Brash et al., Mol. Cell Biol. 7 : 2031-2034 (1987) [28]). Dans certains cas, la lipofection, la nucléofection ou la rupture temporaire de la membrane (par exemple électroporation ou déformation) peuvent être utilisées pour introduire un ou plusieurs polynucléotides exogènes dans la cellule hôte.

Dans la présente invention, le vecteur d'expression peut être tel que décrit ci-dessus.

Dans la présente invention, l'enzyme peut être telle que décrite ci-dessus. Par exemple il peut s'agit d'une glycoside-phosphorylase telle que décrit ci-dessus.

Selon l'invention invention, le procédé peut comprendre en outre une étape a') de transformation d'une souche selon l'invention avec un vecteur d'expression d'au moins une protéine de transport ou perméase.

Selon l'invention invention, le procédé peut comprendre également en outre une étape a') de transformation de la souche déposée à la CNCM (Collection Nationale de Culture de Microorganismes, Institut Pasteur, 25 rue du Docteur Roux, 75724 Paris Cedex 15, France), sous le numéro CNCM I-5499 avec un vecteur d'expression d'au moins une protéine de transport ou perméase.

Selon l'invention invention, lorsque la souche utilisée dans le procédé est la souche déposée à la CNCM sous le numéro CNCM I-5499 comprenant en outre les mutations Δmaa et ΔmanA, le procédé peut comprendre en outre une étape a') de transformation de la souche déposée à la CNCM (Collection Nationale de Culture de Microorganismes, Institut Pasteur, 25 rue du Docteur Roux, 75724 Paris Cedex 15, France), sous le numéro CNCM I-5499 comprenant en outre les mutations Δmaa et ΔmanA avec un vecteur d'expression d'au moins une protéine de transport ou perméase.

Selon l'invention invention, lorsque la souche utilisée dans le procédé est la souche déposée à la CNCM sous le numéro CNCM I-5681, le procédé peut comprendre en outre une étape a') de transformation de la souche déposée à la CNCM (Collection Nationale de Culture de Microorganismes, Institut Pasteur, 25 rue du Docteur Roux, 75724 Paris Cedex 15, France), sous le numéro CNCM I-5681 avec un vecteur d'expression d'au moins une protéine de transport ou perméase.

Selon l'invention invention, lorsque la souche utilisée dans le procédé est la souche déposée à la CNCM sous le numéro CNCM I-5682, le procédé peut comprendre en outre une étape a') de transformation de la souche déposée à la CNCM (Collection Nationale de Culture de Microorganismes, Institut Pasteur, 25 rue du Docteur Roux, 75724 Paris Cedex 15, France), sous le numéro CNCM I-5682 avec un vecteur d'expression d'au moins une protéine de transport ou perméase.

Dans la présente invention, le vecteur d'expression peut être tel que décrit ci-dessus.

Dans la présente invention, une protéine de transport ou perméase peut être telle que décrite ci-dessus. Par exemple il peut s'agir de la galactose perméase telle que décrite ci-dessus.

Selon l'invention les étapes a) et a') peuvent être successives ou concomitantes.

Dans la présente invention, l'étape de culture de la souche peut être réalisée par tout procédé et/ou méthode adapté(e)(s) connu(e)(s) de l'homme du métier.

Il peut s'agir par exemple d'un procédé de culture continue (« continuous fermentation »), d'une culture discontinue alimentée (« fed-batch fermentation ») ou discontinue (« batch fermentation »).

Dans la présente invention, l'étape de culture peut être une culture continue ou une culture pendant une durée. Par exemple la culture peut être réalisée sur une durée de 1 à 12 heures, de 1 à 24 heures de 1 à 36 heures, de 1 à 48 heures de 1 à 240 heures. Par exemple, l'étape de culture peut être réalisée jusqu'à confluence de la souche dans le milieu.

Dans la présente invention, l'étape de culture de la souche peut être réalisée dans tout contenant et/ou dispositif adapté pour la culture de microorganismes, de préférence de bactéries, connu de l'homme du métier. Il peut s'agir par exemple d'un réacteur de culture disponible dans le commerce, par exemple un bioréacteur Multifors (Infors, Suisse).

Dans la présente invention, le milieu de culture peut être ensemencé à partir d'une préculture de la souche, ladite préculture ayant une densité optique (DO) à 600 nm comprises entre 0,01 et 1, de préférence égale à 0,1. Dans la présente invention, le milieu de culture peut être ensemencé avec une concentration de la souche de 2.10⁷ à 2.10¹⁰ bactéries par mL, de préférence de 2.10⁸ bactéries par mL de milieu de culture.

Dans la présente invention, le milieu de culture peut être tout milieu de culture connu de l'homme du métier adapté. Il peut s'agir par exemple d'un milieu liquide ou solide, de préférence un milieu liquide. Il peut s'agir de tous milieux riches connus de l'homme du métier adapté. Il peut s'agir par exemple du milieu LB (Lysogeny Broth), Superbroth, TB (Terrific Broth), YPD (Yeast Extract-Peptone Dextrose). Il peut s'agir par exemple de tout milieu minimum adapté connu de l'homme du métier, par exemple un milieu A, le milieu M9 ou M63 complété d'une source de carbone adéquate, par exemple de glucose, galactose et/ou mannose. Il peut s'agir par exemple de milieux sélectifs adaptés connus de l'homme du métier, par exemple le milieu YNB (Yeast Nitrogen Base).

Il peut s'agir de préférence d'un milieu liquide de culture de bactéries. Il peut s'agir par exemple d'un milieu de culture liquide disponible dans le commerce, par exemple le milieu M9 commercialisé par la société Thermofischer, le milieu Terrific Broth commercialisé par la société Thermofischer, le milieu « MagicMedia (marque de commerce) *E. coli* Expression Medium » commercialisé par la société Thermofischer, le milieu S.O.C. Medium commercialisé par la société Thermofischer.

Il peut s'agir d'un milieu décrit dans le document Karen L. Elbing Roger Brent, Recipes and Tools for Culture of Escherichia coli, Current Tools, 09 November 2018 [52]
Il peut s'agir de préférence d'un milieu liquide comprenant les composants suivants : Na₂HPO₄ 12H₂O 17.4 g/L, KH₂PO₄ 3.02 g/L, NaCl 0,51 g/L, NH₄Cl 2,04 g/L, Na₂EDTA 2 H₂O 15 mg/L, ZnSO₄ 7 H₂O 4,5 mg/L, CoCl₂ 6H₂O 0,3 mg/L, MnCl₂ 4H₂O 1 mg/L, H₃BO₃1 mg/L, Na₂MoO₄ 2 H₂O 0,4 mg/L, FeSO₄ 7 H₂O 3 mg/L, CuSO₄ 5 H₂O 0,3 mg/L. MgSO₄ 0,5 g/L CaCl₂ 4,38 mg/L, Thiamine hypochloride 0,1 g/L, ou d'un milieu liquide comprenant les composants suivants : KH₂PO₄ 3.02 g/L, NaCl 0,51 g/L, NH₄Cl 2,04 g/L, (NH₄)₂SO₄ 5 g/L, Na₂EDTA 2 H₂O 15 mg/L, ZnSO₄ 7 H₂O 4,5 mg/L, CoCl₂ 6H₂O 0,3 mg/L, MnCl₂ 4H₂O 1 mg/L, H₃BO₃ 1 mg/L, Na₂MoO₄ 2 H₂O 0,4 mg/L, FeSO₄ 7 H₂O 3 mg/L, CuSO₄ 5 H₂O 0,3 mg/L. MgSO₄ 0,5 g/L CaCl₂ 4,38 mg/L, Thiamine hypochloride 0,1 g/L.

Dans la présente invention, le milieu de culture peut comprendre en outre au moins un monosaccharide phosphorylé. Par exemple le milieu de culture peut comprendre au moins un monosaccharide phosphorylé choisi dans le groupe comprenant le glucose 1-Phosphate, la N-acétyl-α-D-glucosamine 1-Phosphate, le Galactose 1-Phosphate, le mannose 1-Phosphate; ou un quelconque mélange de ceux-ci.

Dans la présente invention, le monosaccharide phosphorylé peut être présent à une concentration comprise de 1 à 200 g.L⁻¹, de préférence de 5 à 200 g.L⁻¹ de milieu de culture.

Dans la présente invention, le milieu de culture peut comprendre en outre au moins un monosaccharide phosphorylé en fonction de la glycoside-phosphorylase exprimée par le vecteur d'expression tel que présenté dans le tableau 4 ci-dessous :

**Tableau 4 : monosaccharide phosphorylé en fonction de la glycoside-phosphorylase utilisée**

| **GenBank** | **Glycoside-phosphorylase** | **Monosaccharid e-phosphorylé** |
|---|---|---|
| ABX41399.1 | 3-O-α-g1ucopyranosyl-L-rhamnose phosphorylase (Cphy_1019) | Glucose 1-P |
| ABX42289.1 | D-galactosyl-1,4-L-rhamnose phosphorylase (Cphy_1920) | Galactose 1-P |
| ACB74662.1 | D-galactosyl-β-1,4-L-rhamnose phosphorylase (GalRhaP;Oter_1377) | Galactose 1-P |
| ADI00307.1 | 1,2-alpha-glucosylglycérol phosphorylase(Bsel_2816) | Glucose 1-P |
| BAB97299.1 | tréhalose phosphorylase (TreP) | Glucose 1-P |
| BAC20640.1 | tréhalose phosphorylase (TPase) | Glucose 1-P |
| AAF22230.1 | tréhalose phosphorylase | Glucose 1-P |
| ABC84380.1 | tréhalose phosphorylase | Glucose 1-P |
| BAA31350.1 | tréhalose phosphorylase | Glucose 1-P |
| ABX81345.1 | laminaribiose phosphorylase (ACL_0729) | Glucose 1-P |
| BAJ10826.1 | laminaribiose phosphorylase (LbpA) | Glucose 1-P |
| AAS19693.1 | P-1,4-mannosyl-glucose phosphorylase (Unk1) | Mannose 1-P |
| ABY93073.1 | β-1,2-oligomannane phosphorylase (Teth514_1788) | Mannose 1-P |
| ABY93074.1 | β-1,2-mannobiose phosphorylase (Teth514_1789) | Mannose 1-P |
| ADD61463.1 | β-1,4-mannopyranosyl-[N-glycan] phosphorylase (Uhgb_MP) | Mannose 1-P |
| ADU20661.1 | β-1,4-mannooligosaccharide phosphorylase (MOP;RaMP2;Rumal_0099) | Mannose 1-P |
| ADU21379.1 | P-1,4-mannosyl-glucose phosphorylase (RaMP1;RaMGP;Rumal_0852) | Mannose 1-P |
| CAC96089.1 | β-1,2-mannobiose phosphorylase (Lin0857) | Mannose 1-P |
| CAH06518.1 | P-1,4-mannosyl-glucose phosphorylase (MGP;BF0772) | Mannose 1-P |
| CAZ94304.1 | β-1,3-mannooligosaccharide phosphorylase (zobellia_231) | Mannose 1-P |
| WP_026485574.1 | β-1,4-mannooligosaccharide phosphorylase (CalpoDRAFT_0075) | Mannose 1-P |
| WP_026486530.1 | β-1,4-mannooligosaccharide phosphorylase (CalpoDRAFT_1209) | Mannose 1-P |
| VCV21229.1 | β-1,4-mannooligosaccharide phosphorylase (RIL182_01100;ROSINTL182_05474) | Mannose 1-P |
| VCV21228.1 | Mannosyl-glucose phosphorylase (RIL182_01099;ROSINTL182_07685) | Mannose 1-P |
| XP_003872966.1 | β-1,2-oligomannane phosphorylase MTP3 (LMXM_10_1250) | Mannose 1-P |
| CBZ24448.1 | | |
| CBZ24449.1 | β-1,2-oligomannane phosphorylase MTP4 (LMXM_10_1260) | Mannose 1-P |
| XP_003872967.1 | | |
| CBZ24451.1 | β-1,2-oligomannane phosphorylase MTP6 (LMXM_10_1280) | Mannose 1-P |
| XP 003872969.1 | | |
| CBZ24452.1 | β-1,2-oligomannane phosphorylase MTP7 (LMXM_10_1290) | Mannose 1-P |
| XP 003872970.1 | | |

| **GigaBase accession number** (GigaDB, DOI: 10.5524/100064) | **Glycoside-phosphorylase** | **Monosaccharid e-phosphorylé** |
|---|---|---|
| MH0373_GL0093988 | β-1,4-mannooligosaccharide phosphorylase | Mannose 1-P |
| 340101.Vvad_PD307 4 | β-1,3-mannosyl-glucose phosphorylase/ β-1,3-mannooligosaccharide phosphorylase | Mannose 1-P |
| 340101.Vvad_PD307 4 | β-1,3-mannosyl-glucose phosphorylase/ β-1,3-mannooligosaccharide phosphorylase | Mannose 1-P |
| MH0431_GL0150624 | β-1,4-mannosyl-glucuronate phosphorylase ("β-1,4-mannosyl-glucuronic acid phosphorylase") | Mannose 1-P |

Avantageusement, les inventeurs ont démontré que lorsque la souche comprend en outre un vecteur d'expression permettant l'expression d'au moins un gène codant pour une protéine de transport ou perméase non PTS, la souche selon l'invention peut avantageusement internaliser des hydrates de carbone non phosphorylés qui peuvent être présents dans le milieu de culture.

Aussi, le procédé selon l'invention peut permettre avantageusement d'utiliser des hydrates de carbone non phosphorylés et de s'affranchir de l'utilisation couteuse de monosaccharides phosphorylés usuels et/ou de monosaccharides phosphorylés tels que mentionnés dans le tableau 4 ci-dessus.

Selon l'invention, le milieu de culture peut comprendre en outre au moins un hydrate de carbone non phosphorylé, de préférence un monosaccharide. Par exemple le milieu de culture peut comprendre au moins un hydrate de carbone non phosphorylé choisi dans le groupe comprenant le D- glucose, le L-rhamnose, le glycérol, le D-mannose ou un quelconque mélange de ceux-ci.

Dans la présente invention, la concentration dans le milieu de culture dudit au moins un hydrate de carbone non phosphorylé peut être comprise de 1 à 100 g.L⁻¹, de préférence de 2 à 200 g.L⁻¹, de préférence de 1 à 5 g.L⁻¹ de milieu de culture, en un ou plusieurs ajouts successifs.

Dans la présente invention, le milieu de culture peut comprendre en outre au moins un hydrate de carbone non phosphorylé en fonction de la glycoside-phosphorylase exprimée par le vecteur d'expression par exemple tel que présenté dans le tableau 5 ci-dessous :

**Tableau 5 : hydrates de carbone non phosphorylés en fonction de la glycoside-phosphorylase**

| **GenBank** | **Glycoside-phosphorylase** | **Hydrates de carbone non phosphorylés** |
|---|---|---|
| ABX41399.1 | 3-O-α-glucopyranosyl-L-rhamnose phosphorylase(Cphy_1019) | L-rhamnose |
| ABX42289.1 | D-galactosyl-1,4-L-rhamnose phosphorylase (Cphy_1920) | L-rhamnose |
| ACB74662.1 | D-galactosyl-β-1,4-L-rhamnose phosphorylase (GalRhaP;Oter_1377) | L-rhamnose |
| ADI00307.1 | 1,2-alpha-glucosylglycérol phosphorylase(Bsel_2816) | glycérol |
| BAB97299.1 | tréhalose phosphorylase (TreP) | D-glucose |
| BAC20640.1 | tréhalose phosphorylase (TPase) | D-glucose |
| AAF22230.1 | tréhalose phosphorylase | D-glucose |
| ABC84380.1 | tréhalose phosphorylase | D-glucose |
| BAA31350.1 | tréhalose phosphorylase | D-glucose |
| AAS19693.1 | β-1,4-mannosyl-glucose phosphorylase (Unk1) | D-glucose |
| ABX81345.1 | laminaribiose phosphorylase (ACL_0729) | D-glucose |
| BAJ10826.1 | laminaribiose phosphorylase (LbpA) | D-glucose |
| ABY93073.1 | β-1,2-oligomannane phosphorylase (Teth514_1788) | D-mannose |
| ABY93074.1 | β-1,2-mannobiose phosphorylase (Teth514_1789) | D-mannose |
| ADD61463.1 | β-1,4-mannopyranosyl-[N-glycan] phosphorylase (Uhgb_MP) | D-mannose |
| ADU20661.1 | β-1,4-mannooligosaccharide phosphorylase (MOP;RaMP2;Rumal_0099) | D-mannose |
| ADU21379.1 | β-1,4-mannosyl-glucose phosphorylase (RaMP1;RaMGP;Rumal_0852) | D-glucose |
| CAC96089.1 | β-1,2-mannobiose phosphorylase (Lin0857) | D-mannose |
| CAH06518.1 | β-1,4-mannosyl-glucose phosphorylase (MGP;BF0772) | D-glucose |
| CAZ94304.1 | β-1,3-mannooligosaccharide phosphorylase (zobellia_231) | D-mannose |
| WP_026485574.1 | β-1,4-mannooligosaccharide phosphorylase (CalpoDRAFT_0075) | D-mannose |
| WP_026486530.1 | β-1,4-mannooligosaccharide phosphorylase (CalpoDRAFT_1209) | D-mannose |
| VCV21229.1 | β-1,4-mannooligosaccharide phosphorylase (RIL182_01100;ROSINTL182_05474 ) | D-mannose |
| VCV21228.1 | Mannosyl-glucose phosphorylase (RIL182_01099;ROSINTL182_07685 ) | D-glucose |
| XP_003872966.1 CBZ24448.1 | β-1,2-oligomannane phosphorylase MTP3 (LMXM_10_1250) | D-mannose |
| CBZ24449.1 XP 003872967.1 | β-1,2-oligomannane phosphorylase MTP4 (LMXM_10_1260) | D-mannose |
| CBZ24451.1 XP_003872969.1 | β-1,2-oligomannane phosphorylase MTP6 (LMXM_10_1280) | D-mannose |
| CBZ24452.1 XP_003872970.1 | β-1,2-oligomannane phosphorylase MTP7 (LMXM_10_1290) | D-mannose |

| **GigaDB accession number (GigaDB, DOI: 10.5524/100064)** | **Glycoside-phosphorylase** | **Hydrates de carbone non phosphorylés** |
|---|---|---|
| MH0373_GL0093988 | β-1,4-mannooligosaccharide phosphorylase | D-mannose |
| 340101.Vvad_PD3074 | β-1,3-mannosyl-glucose phosphorylase/ β-1,3-mannooligosaccharide phosphorylase | D-glucose |
| 340101.Vvad_PD3074 | β-1,3-mannosyl-glucose phosphorylase/ β-1,3-mannooligosaccharide phosphorylase | D-mannose |
| MH0431_GL0150624 | β-1,4-mannosyl-glucuronate phosphorylase ("β-1,4-mannosyl-glucuronic acid phosphorylase") | Acide D-glucuronique |

Dans la présente invention, lors de l'étape de culture, le procédé peut comprendre en outre au moins une étape d'ajout d'au moins un hydrate de carbone non phosphorylé dans le milieu de culture. Il peut s'agir d'au moins un hydrate de carbone non phosphorylé tel que mentionné ci-dessus. Dans la présente invention, l'étape de culture peut être réalisée à une température de 25 à 45°C, par exemple de 35 à 38°C, par exemple égale à 37°C.

Dans la présente invention la récupération d'oligosaccharides produit peut être réalisée dans le milieu de culture ou dans la souche, de préférence dans le milieu de culture.

Dans la présente invention, l'étape de récupération d'oligosaccharides peut être réalisée par tout procédé adapté connu de l'homme du métier. Il peut s'agir, par exemple d'une étape comprenant ou non une ou des étapes de purification. Il peut s'agir par exemple d'un procédé décrit dans le document Israel Pedruzzi, Eduardo Borges da Silva, Alirio E Rodrigues "Sélection of resins, equilibrium and sorption kinetics of lactobionic acid, fructose, lactose and sorbitol" Separation and Purification Technology 2008 Volume 63, Issue 3, 3 November 2008, Pages 600-611 [44], Clarisse Nobre, José A. Teixeira & Lígia R. Rodrigues (2015) "New Trends and Technological Challenges in the Industrial Production and Purification of Fructo-oligosaccharides", Critical Reviews in Food Science and Nutrition, 55:10, 1444-1455, 11 octobre 2013 [45] et/ou sur la page internet https://www.novasep.com/technologies/industrial-technologies-for-evaporation-concentration-and-crystallization.html [46].

Il peut s'agir par exemple d'une étape comprenant une étape de microfiltration, une étape d'ultrafiltration, une étape de nanofiltration, une étape d'osmose inverse, une étape de lyophilisation, une étape d'évaporation, par exemple sur évaporateur rotatif, une étape d'atomisation, une étape d'extraction par phase liquide, une étape de cristallisation du produit, une étape de chromatographie, une étape de dialyse et/ou d'électrodialyse.

Il peut s'agir par exemple d'une étape comprenant une filtration du milieu à travers un filtre, par exemple avec des pores de 0,3 nm à 10 µm de diamètre par exemple avec des pores de 0,1 à 10 µm de diamètre pour une microfiltration, de 1 à 100 nm de diamètre pour une ultrafiltration, ou 0,3 à 1 nm de diamètre pour une nanofiltration.

Il peut s'agir par exemple d'un procédé comprenant une chromatographie, par exemple en phase gazeuse, en phase liquide ou ionique, d'une électrophorèse, par exemple d'une électrophorèse capillaire, d'une filtration, par exemple d'une nanofiltration, d'une extraction en phase solide, d'une micro-extraction en phase solide, d'une extraction par solvant. Il peut s'agir par exemple d'une chromatographie en phase liquide, une chromatographie liquide à haute performance, une chromatographie liquide-solide, une chromatographie liquide-liquide, une chromatographie de partage à polarité de phases inversée, une chromatographie d'échange d'ions, une chromatographie ionique, une chromatographie d'interactions ioniques, une chromatographie d'interactions hydrophobes, une chromatographie d'exclusion stérique, par exemple avec une résine de type *Sephadex G15* ou *Biogel P2,* une chromatographie de perméation sur gel, une chromatographie de filtration sur gel, une chromatographie d'échange de ligands, un fractionnement flux forcé, une chromatographie planaire, une chromatographie de partage centrifuge, une chromatographie à contre-courant, une chromatographie liquide-liquide centrifuge, une chromatographie phase stationnaire chirale.

L'homme du métier, de part ces connaissances générales saura adapter et/ou choisir le procédé de récupération en fonction du milieu et/ou de l'oligosaccharide produit.

Avantageusement, le procédé selon l'invention peut permettre la production d'oligosaccharides, par exemple des oligosaccharides mentionnés dans le tableau 6 ci-dessous. Par exemple la présente invention peut permettre la production d'oligosaccharides en fonction de la glycoside-phosphorylase utilisée tel que mentionnés dans le tableau 6 ci-dessous.

**Tableau 6 : oligosaccharides produit en fonction de la glycoside-phosphorylase**

| **GenBank** | **Glycoside-phosphorylase** | **Produit** |
|---|---|---|
| ABX41399.1 | 3-O-α-glucopyranosyl-L-rhamnose phosphorylase (Cphy_1019) | 3-O-α-glucopyranosyl-L-rhamnose |
| ABX42289.1 | D-galactosyl-1,4-L-rhamnose phosphorylase (Cphy_1920) | D-galactosyl-1,4-L-rhamnose |
| ACB74662.1 | D-galactosyl-β-1,4-L-rhamnose phosphorylase (GalRhaP;Oter_1377) | D-galactosyl-β-1,4-L-rhamnose |
| ADI00307.1 | 1,2-alpha-glucosylglycérol phosphorylase(Bsel_2816) | 1,2-alpha-glucosylglycérol |
| BAB97299.1 | tréhalose phosphorylase (TreP) | tréhalose |
| BAC20640.1 | tréhalose phosphorylase (TPase) | tréhalose |
| AAF22230.1 | tréhalose phosphorylase | tréhalose |
| ABC84380.1 | tréhalose phosphorylase | tréhalose |
| BAA31350.1 | tréhalose phosphorylase | tréhalose |
| AAS19693.1 | β-1,4-mannosyl-glucose phosphorylase (Unk1) | β-1,4-mannosyl-glucose |
| ABY93073.1 | β-1,2-oligomannane phosphorylase (Teth514_1788) | β-1,2-oligomannane |
| ABY93074.1 | β-1,2-mannobiose phosphorylase (Teth514_1789) | β-1,2-mannobiose |
| ADD61463.1 | β-1,4-mannopyranosyl-[N-glycan] phosphorylase (Uhgb_MP) | β-1,4-mannooligosaccha ride |
| ADU20661.1 | β-1,4-mannooligosaccharide phosphorylase (MOP;RaMP2;Rumal_0099) | β-1,4-mannooligosaccha ride |
| ADU21379.1 | β-1,4-mannosyl-glucose phosphorylase (RaMP1;RaMGP;Rumal_0852) | β-1,4-mannosyl-glucose |
| CAC96089.1 | β-1,2-mannobiose phosphorylase (Lin0857) | β-1,2-mannobiose |
| CAH06518.1 | β-1,4-mannosyl-glucose phosphorylase (MGP;BF0772) | β-1,4-mannosyl-glucose |
| CAZ94304.1 | β-1,3-mannooligosaccharide phosphorylase (zobellia_231) | β-1,3-mannooligosaccha ride |
| WP_026485574.1 | β-1,4-mannooligosaccharide phosphorylase (CalpoDRAFT_0075 ) | β-1,4-mannooligosaccha ride |
| WP_026486530.1 | β-1,4-mannooligosaccharide phosphorylase (CalpoDRAFT_1209) | β-1,4-mannooligosaccha ride |
| VCV21229.1 | β-1,4-mannooligosaccharide phosphorylase (RIL182_01100;RQSINTL182_0547 4) | β-1,4-mannooligosaccha ride |
| VCV21228.1 | Mannosyl-glucose phosphorylase (RIL182_01099;ROSINTL182_0768 5) | β-1,4-mannosyl-glucose |
| XP_003872966.1 CBZ24448.1 | β-1,2-oligomannane phosphorylase MTP3 (LMXM_10_1250) | β-1,2-oligomannane |
| CBZ24449.1 XP_003872967.1 | β-1,2-oligomannane phosphorylase MTP4 (LMXM_10_1260) | β-1,2-oligomannane |
| CBZ24451.1 XP_003872969.1 | β-1,2-oligomannane phosphorylase MTP6 (LMXM_10_1280) | β-1,2-oligomannane |
| CBZ24452.1 XP_003872970.1 | β-1,2-oligomannane phosphorylase MTP7 (LMXM_10_1290) | β-1,2-oligomannane |
| ABX81345.1 | laminaribiose phosphorylase (ACL_0729) | laminaribiose |
| BAJ 10826.1 | laminaribiose phosphorylase (LbpA) | laminaribiose |

| **GigaB** (GigaDB, DOI: 10.5524/100064) | **Glycoside-phosphorylase** | **Produit** |
|---|---|---|
| MH0373_GL0093988 | β-1,4-mannooligosaccharide phosphorylase | β-1,4-mannooligosaccha ride |
| 340101.Vvad_PD3074 | β-1,3-mannosyl-glucose phosphorylase/ β-1,3-mannooligosaccharide phosphorylase | β-1,3-mannosyl-glucose |
| 340101.Vvad_PD3074 | β-1,3-mannosyl-glucose phosphorylase/ β-1,3-mannooligosaccharide phosphorylase | β-1,3-mannooligosaccha ride |
| MH0431_GL0150624 | β-1,4-mannosyl-glucuronate phosphorylase ("β-1,4-mannosyl-glucuronic acid phosphorylase") | β-1,4-mannosyl-glucuronate |

D'autres avantages pourront encore apparaître à l'homme du métier à la lecture des exemples ci-dessous, illustrés par les figures annexées, donnés à titre illustratif.

### Brève description des figures

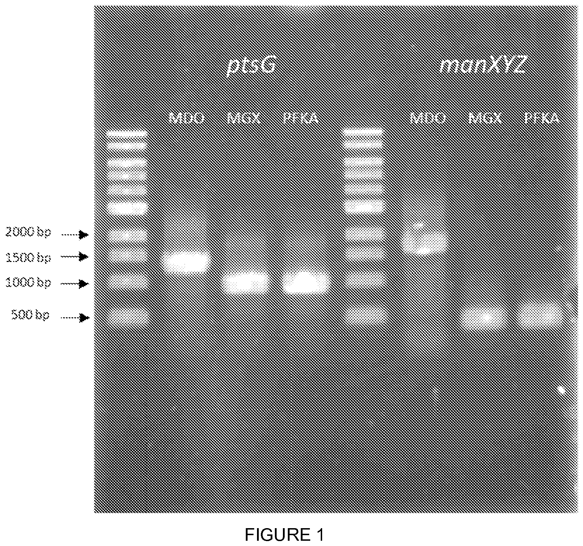

- La figure 1 est une photographie qui présente les produits PCR des fragments d'ADN amplifiés depuis le génome des souches MDO, MGX et PFKA, et ayant migré sur un gel d'agarose. Gel d'agarose des produits PCR des souches MDO, MGX et PFKA. Pistes 1 et 5, marqueur de taille ; pistes 2, 3 et 4, produits PCR du fragment du gène ptsG ; pistes 6, 7 et 8, produits PCR du fragment de l'opéron manXYG.

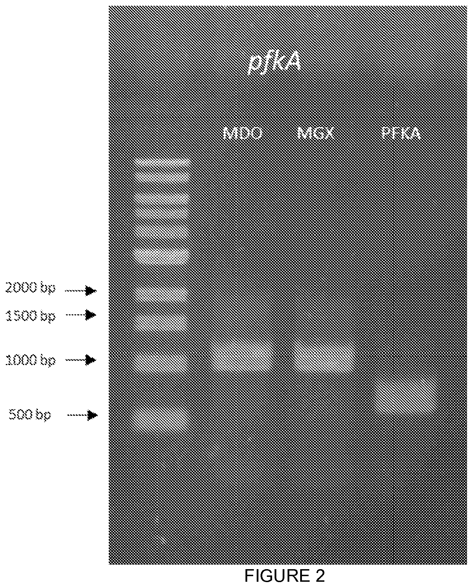

- La figure 2 est une photographie qui présente les produits PCR des fragments d'ADN amplifiés depuis le génome des souches MDO, MGX et PFKA, et ayant migré sur un gel d'agarose. Gel d'agarose des produits PCR des souches MDO, MGX et PFKA. Piste 1, marqueur de taille ; pistes 2, 3 et 4, produits PCR du fragment du gène pfkA.

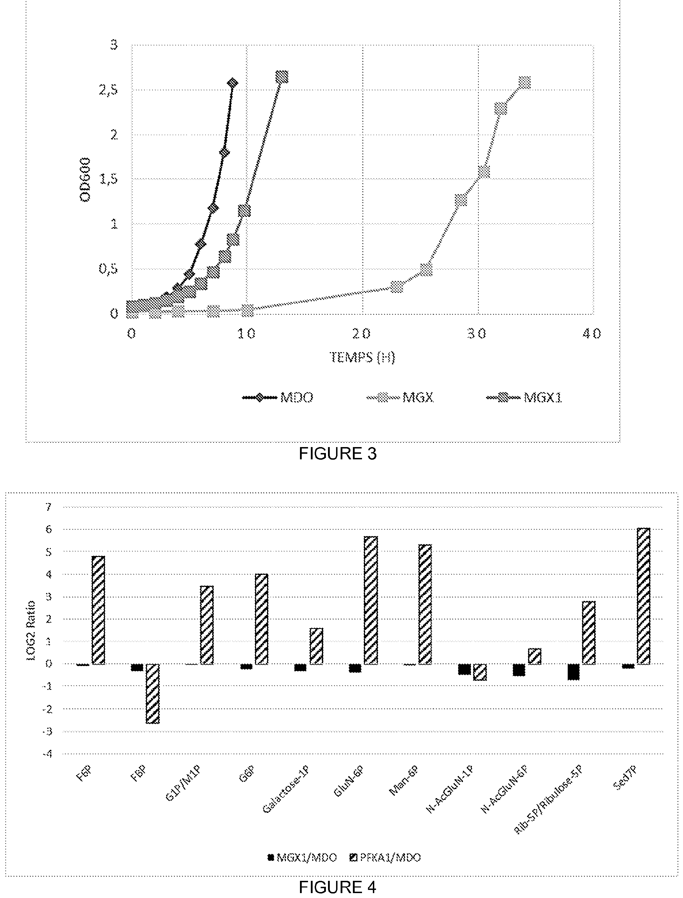

- La figure 3 est un diagramme représentant les courbes de croissance des souches *E. coli* MDO (losanges), MGX (carrés gris claires) et MGX1 (carrés gris foncés) dans un milieu minimum M9 supplémenté avec 3 g/L de glucose, l'ordonné correspond à la Densité Optique mesuré à 600 nm (OD600) et l'abscisse au temps en heures (H).
- La figure 4 est un histogramme représentant une comparaison de concentrations en métabolites entre la souche *E. coli* MDO et la souche MGX1 (bâtons noirs) et entre la souche *E. coli* MDO et la souche PFKA1 (bâtons hachurés). Sur la figure F6P signifie Fructose-6-phosphate, FBP signifie Fructose-1,6-Bisphosphate, G1P/M1P: Glucose-1-Phosphate/Mannose-1-Phosphate, G6P : Glucose-6-Phosphate, Galactose-1P : Galactose-1-Phosphate, GluN-6P :Glucosamine-6-Phosphate , Man-6P : Mannose-6-Phosphate, N-AcGlucoseN-1P : N-acétyl glucose-1-Phosphate, N-AcGlucoseN-6P : N-acétyl glucose-6-Phosphate, Rib-5P/Ribulose-5P : Ribose-5-Phosphate/ Ribulose-5-Phosphate, Sed7P sedoheptulose-7-phosphate. L'ordonné correspond à la valeur des rapports des concentrations de métabolites exprimés en log₂.

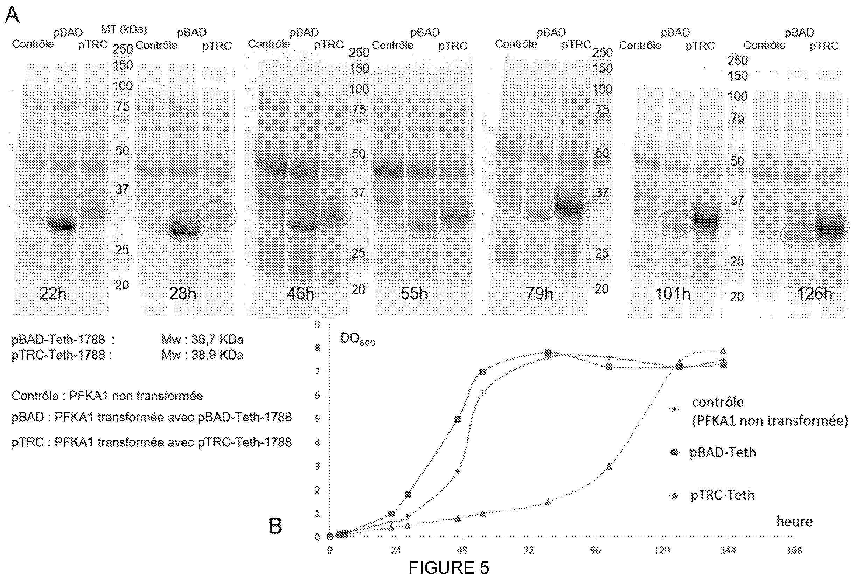

- La figure 5 représente des photographies gel d'électrophorèse SDS-PAGE (figure 5 A) et un diagramme correspondant à l'évolution de la Densité Optique dans le milieu de culture en fonction du temps (figure 5 B). La figure 5 A correspond à la photographie de 7 gels d'électrophorèse SDS-PAGE effectués respectivement à 22h, 28h, 46h, 55h, 79h, 101h et 126h de culture de la souche PFKA1 non transformée (contrôle), avec le vecteur pBAD-Teth1788 ou pTRC-Teth-1788. La figure 5 B est un diagramme de l'évolution de la Densité Optique à 600nm du milieu de culture (ordonnée) de la souche PFKA1 non transformée (contrôle - croix), avec le vecteur pBAD-Teth1788 (carrés) ou pTRC-Teth1788 (triangle) en fonction du temps (abscisse).

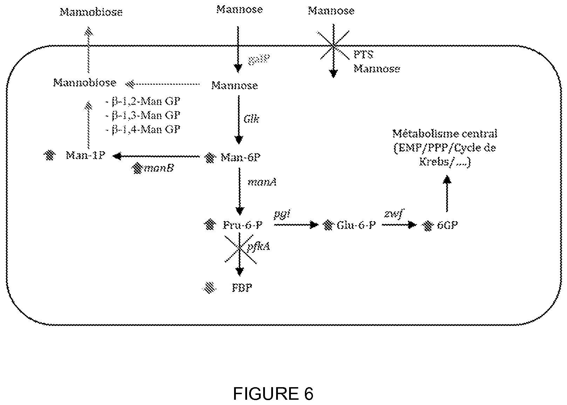

- La figure 6 correspond à un schéma du métabolisme de la souche PFKA1 transformée avec le vecteur pBAD-Teth1788 ou avec le vecteur pBAD-Uhgb_MP, ou avec le vecteur pBAD-β-1,3-mannoside-phosphorylase. Sur cette figure les abréviations signifient : galP : galactose perméase, PTS : Phosphotransférases, Glk : Glucokinase, manA : mannose-6-phosphate isomérase, pgi : glucose-6-phosphate isomérase, zwf : Glucose-6-phosphate dehydrogenase, Man-1P : Mannose-1-Phosphate, Man-6P : Mannose-6-Phosphate, Glu-6-P : Glucose-6-Phosphate, Fru-6-P : Fructose-6-Phosphate, FBP : Fructose-1,6-Bisphosphate.

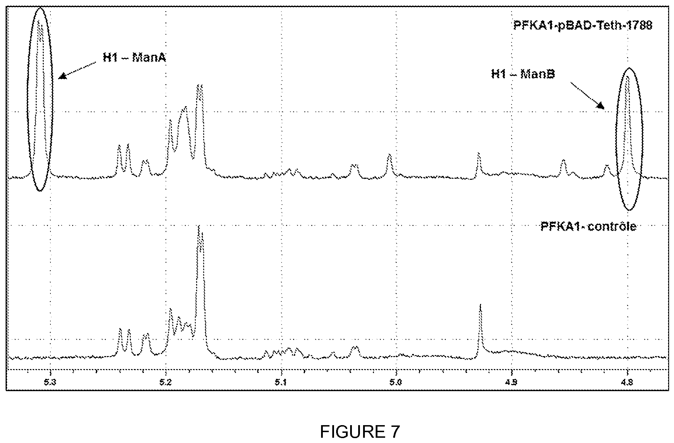

- La figure 7 correspond au spectre RMN d'échantillons de milieu de culture de souche PFKA1 transformée avec le vecteur pBAD -Teth1788 (courbe supérieure) ou d'échantillons de milieu de culture de souche PFKA1 non transformée (contrôle).

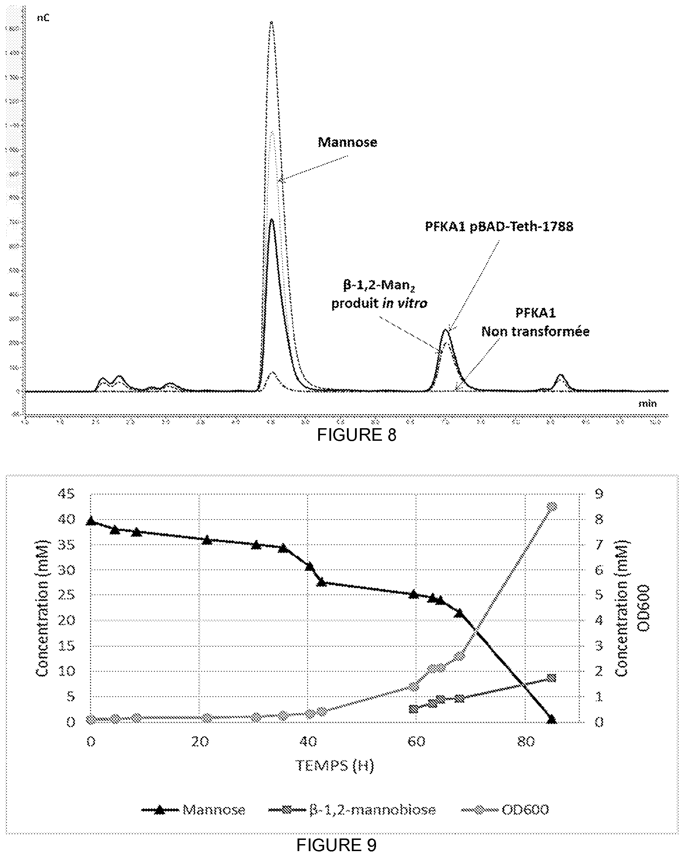

- La figure 8 correspond au chromatogramme d'une Chromatographie Haute-Performance Echangeuse d'Anion avec Détection par Ampérométrie Pulsée (HPAEC-PAD) d'échantillons de milieu de culture de la souche PFKA1 transformée avec le vecteur pBAD-HisA-Teth1788 (courbe en trait continu gras) ou d'échantillons de milieu de culture de souche PFKA1 non transformée (courbe en pointillés) ou de β-1,2-mannobiose (β-1,2-Man₂) purifié (courbe en pointillés discontinus).
- La figure 9 correspond à un diagramme de l'évolution de la concentration en milliMolaire (mM) en mannose (triangles), β1,2-mannobiose (ronds) et densité optique à 600nm (carrés) en fonction du temps en minutes dans le milieu de culture de la souche PFKA1 transformée avec pBAD-Teth-1788.

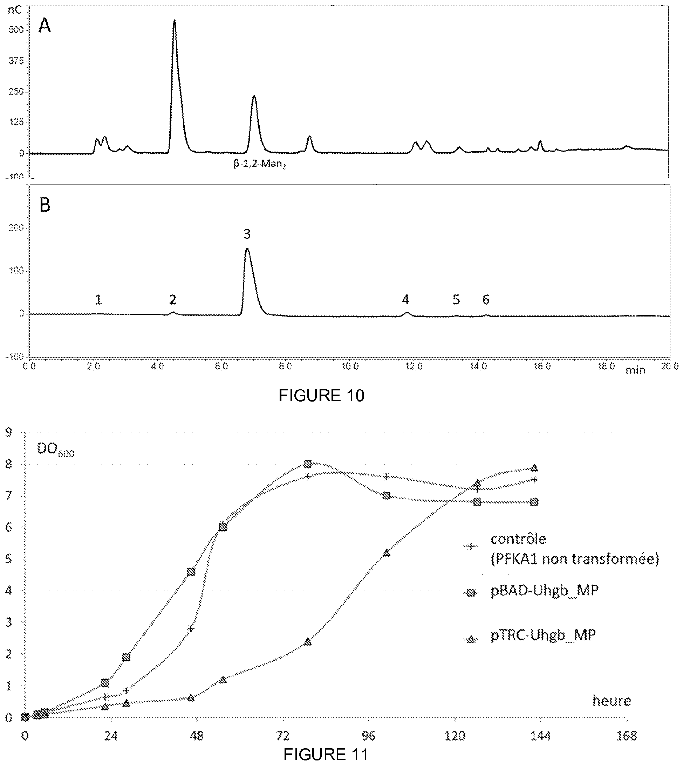

- La figure 10 correspond à des chromatogrammes d'analyses par HPAEC-PAD de la purification du β-1,2-mannobiose (β-1,2-man₂) à partir d'un surnageant de culture PFKA1-pBAD-Teth-1788. La figure 10 A correspond au chromatogramme obtenu par HPAEC-PAD avec le surnageant de culture PFKA1-pBAD-Teth-1788 avant purification. B : Chromatogramme du β-1,2-mannobiose après purification. Sur les chromatogrammes, l'ordonnée représente la conductivité en nanoCoulomb (nC) et l'abscisse le temps en minutes (min).
- La figure 11 est un diagramme de l'évolution de la densité optique à 600nm du milieu de culture (ordonnée) de la souche PKA1 non transformée (contrôle - croix), avec le vecteur pBAD-UhgbMP (carrés) ou pTRC-UhgbMP (triangle) en fonction du temps (abscisse).

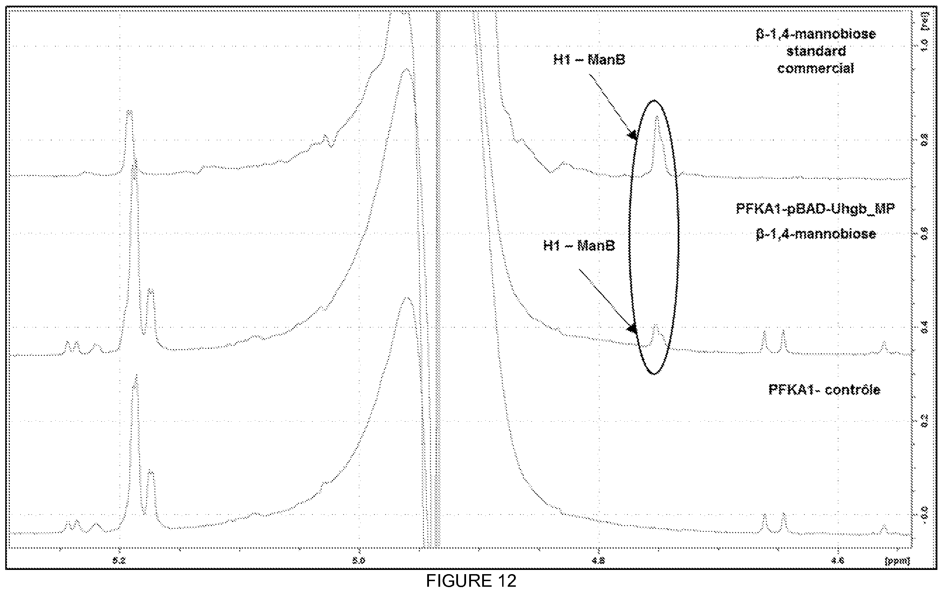

- La figure 12 est un spectre RMN d'échantillons de milieu de culture de souche PFKA1 transformée avec le vecteur pBAD-UhgbMP (courbe intermédiaire) ou d'échantillons de milieu de culture de souche PFKA1 non transformée (contrôle) (courbe inférieure) et d'une solution du standard commercial β-1,4-mannobiose (Carbosynth, Royaume-Uni) (courbe supérieure).

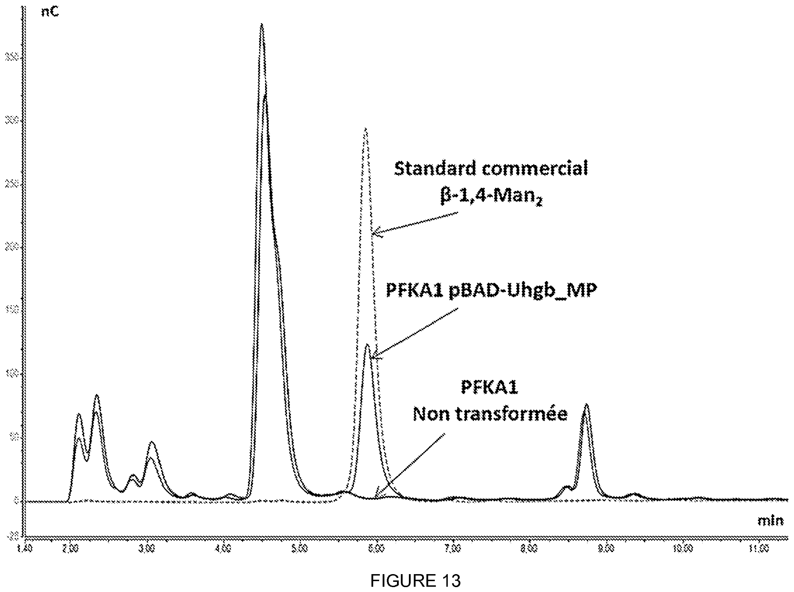

- La figure 13 correspond à l'analyse HPAEC-PAD d'échantillons, la courbe en trait continu fin correspond au chromatogramme du surnageant de culture souche PFKA1 transformée avec pBAD-UhgbMP, la courbe en trait continu gras correspond au chromatogramme du surnageant de culture de la souche PFKA1 non transformée, la courbe en trait pointillé correspond au standard commercial de β-1,4-mannobiose.

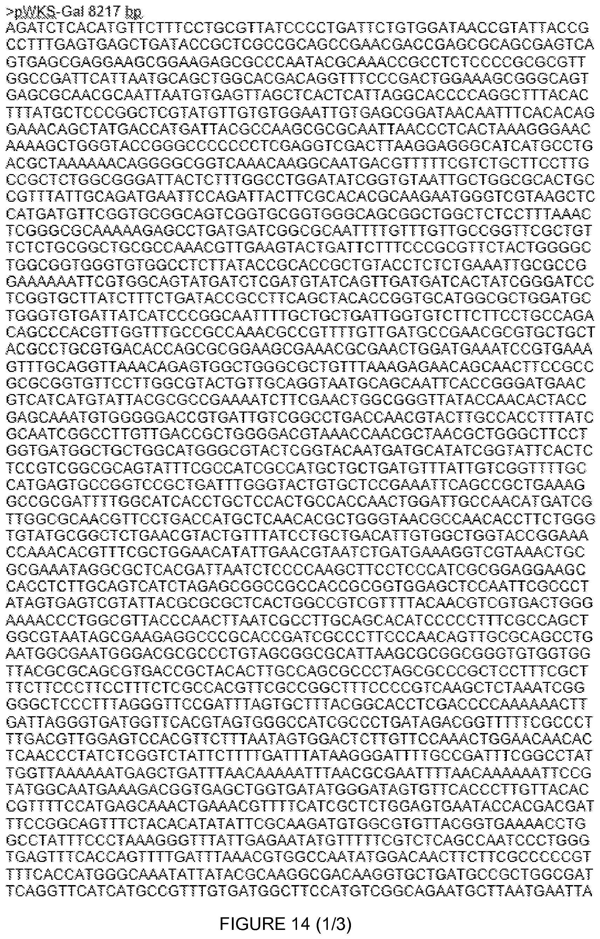

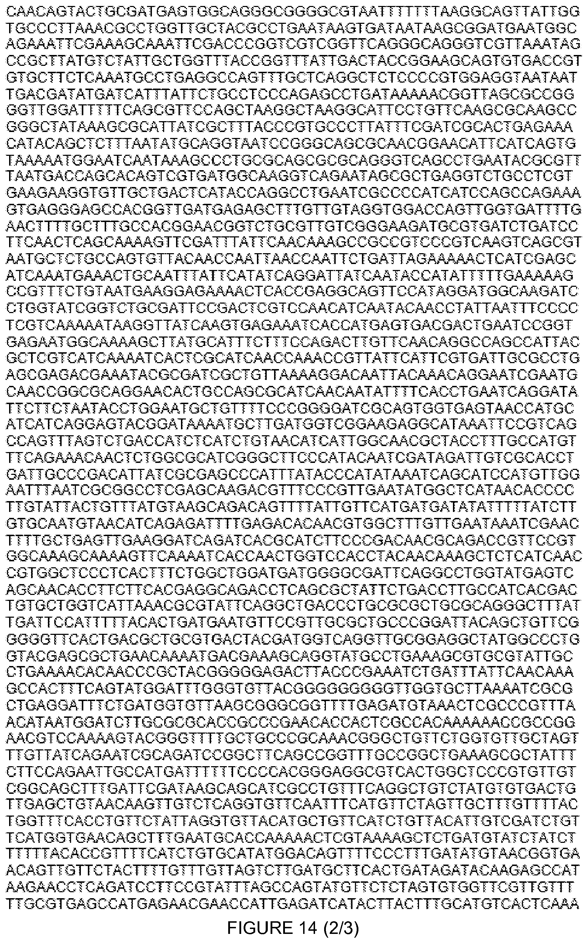

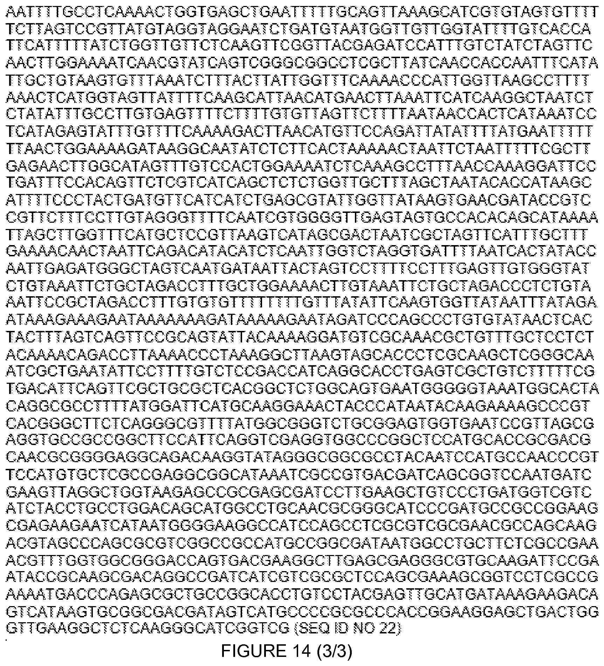

- La figure 14 représente la séquence plasmidique du vecteur pWKS-galP.

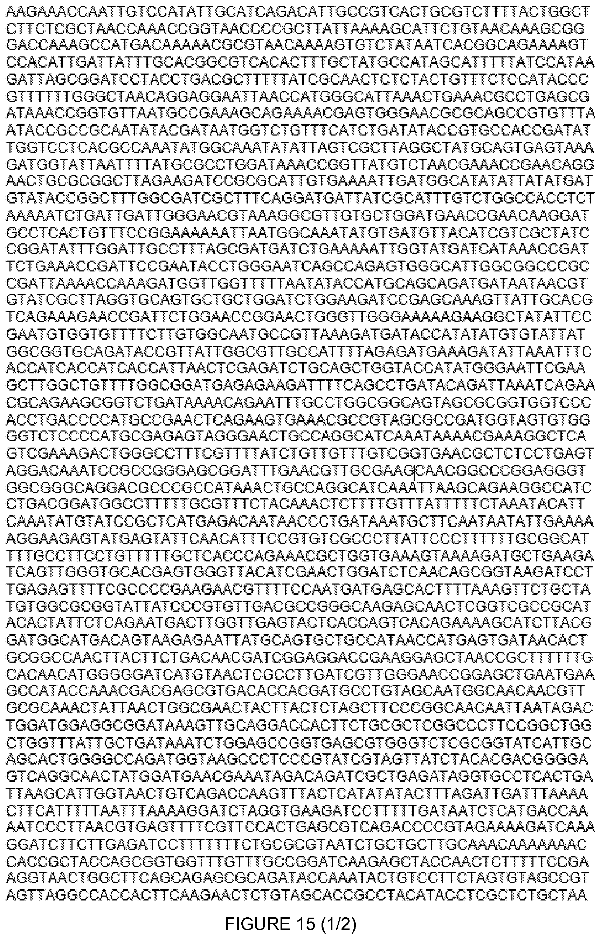

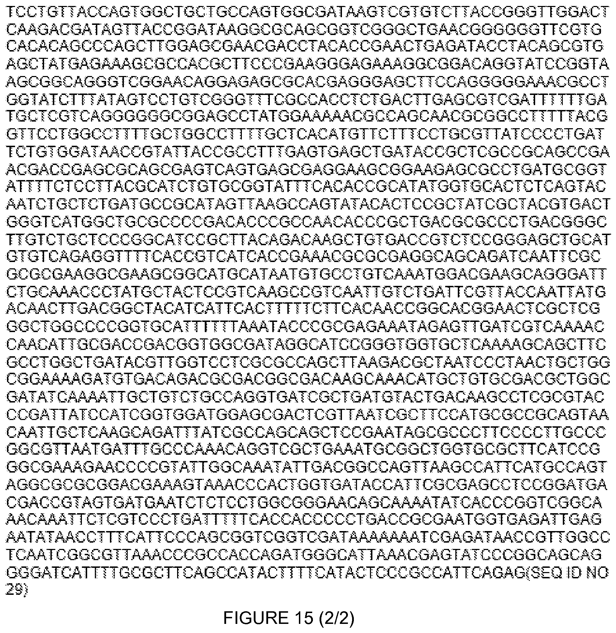

- La figure 15 représente la séquence plasmidique du vecteur pBAD-Teth 1788.

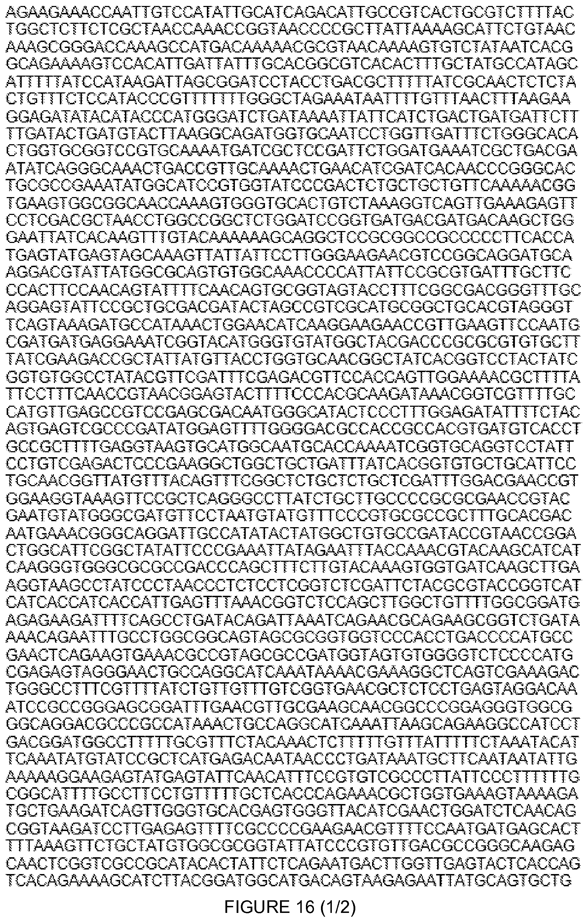

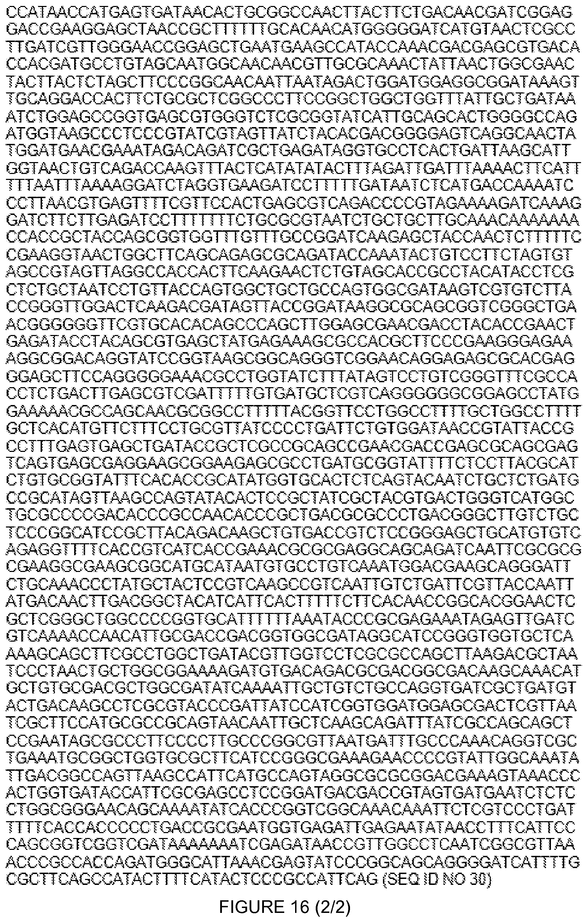

- La figure 16 représente la séquence plasmidique du vecteur pBAD-Uhgb_MP.

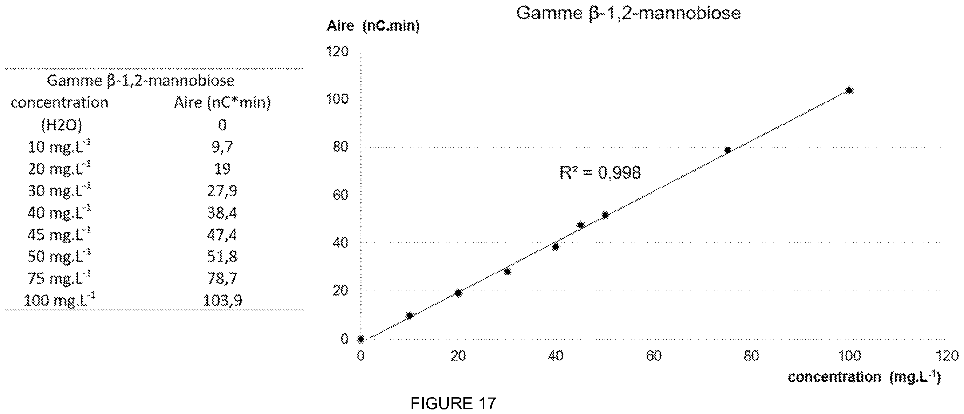

- La figure 17 représente une courbe étalon de la concentration de β-1,2-mannobiose en milligramme par litre (mg.L⁻¹) (abscisse) en fonction de l'aire sous la courbe en nanoCoulomb (nC) par minute (nC.min), quantifiée par HPAEC-PAD.

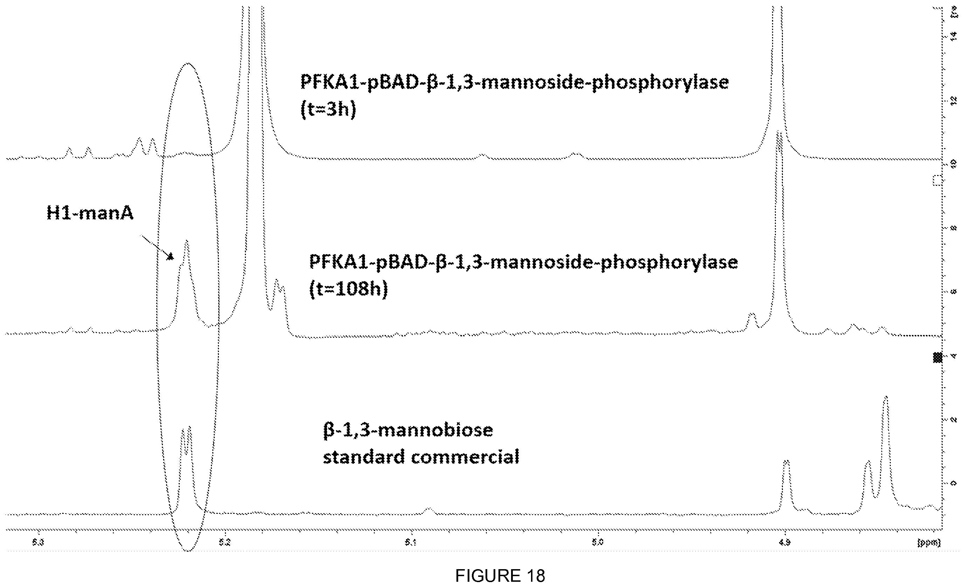

- La figure 18 représente correspond au spectre RMN d'échantillons de β-1,3-mannobiose commercial, du surnageant de culture souche PFKA1 transformée avec le plasmide pBAD-β-1,3-mannooligosaccharide phosphorylase après 3 (t=3h) ou 108 (t=108h) heures de culture.

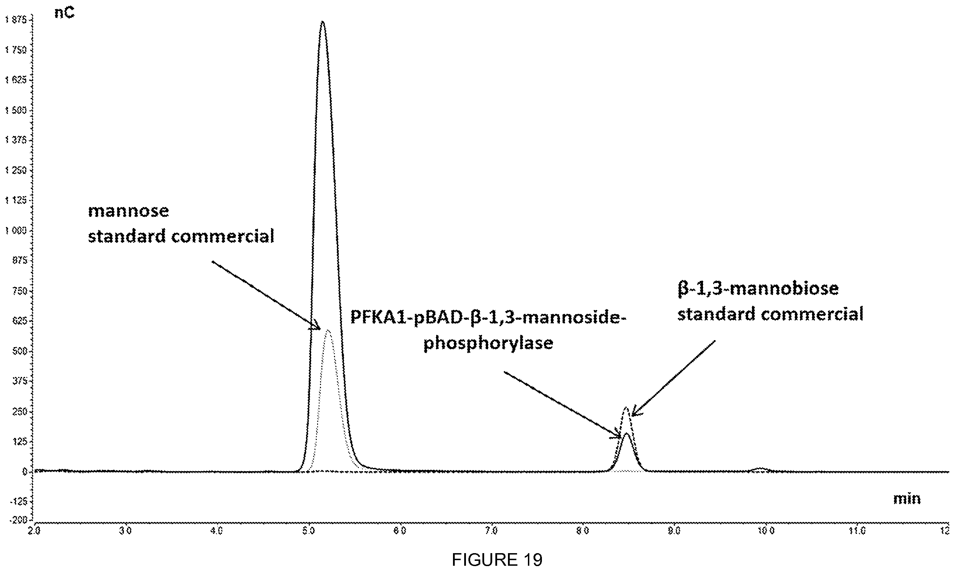

- La figure 19 représente les chromatogrammes de différents échantillons analysés par HPAEC-PAD, sur cette figure la courbe en trait continu gras correspond au chromatogramme du surnageant de culture de la souche PFKA1 transformée avec le plasmide pBAD-β-1,3-mannooligosaccharide phosphorylase après 108 heures de culture, la courbe en pointillé correspond au chromatogramme d'une solution commerciale de β-1,3-mannobiose.

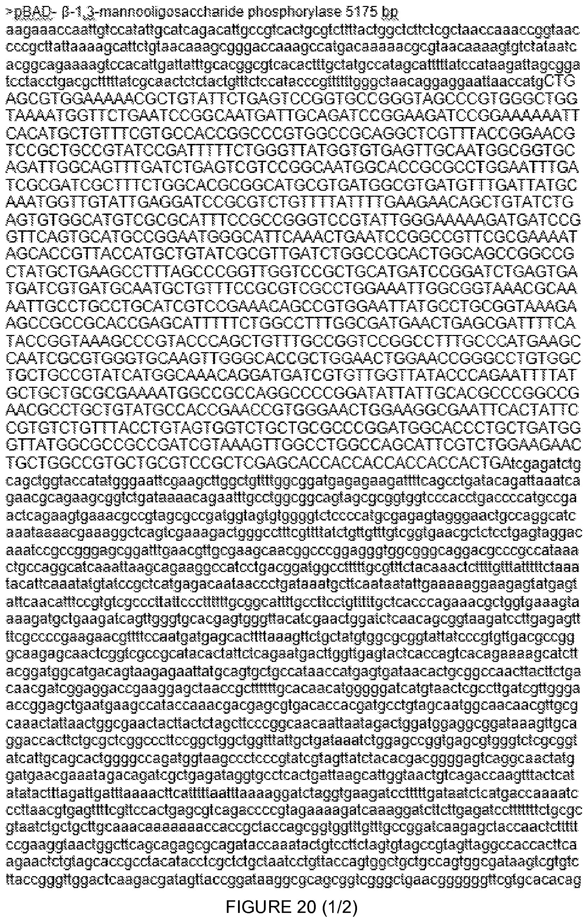

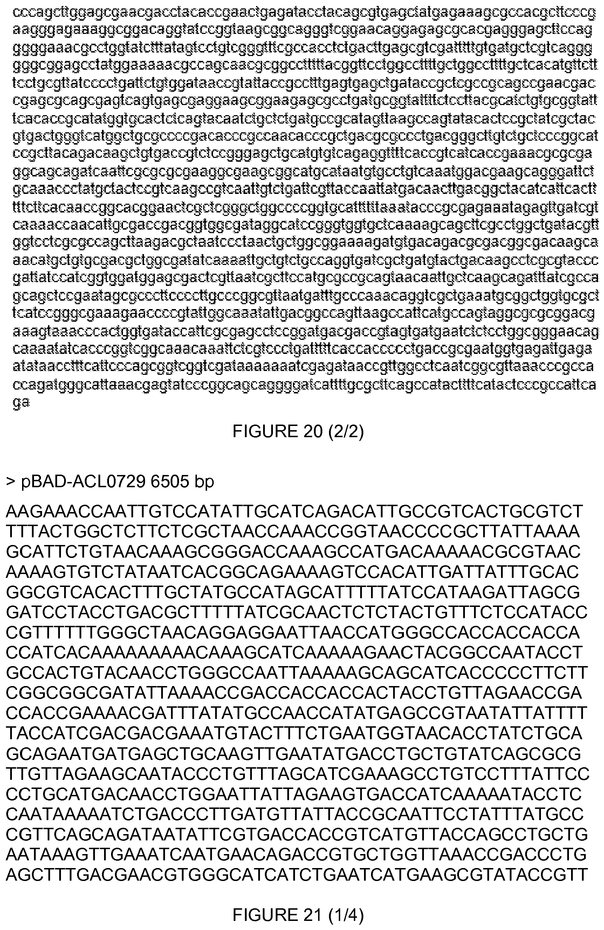

- La figure 20 représente la séquence plasmidique du vecteur pBAD-β-1,3-mannooligosaccharide phosphorylase.

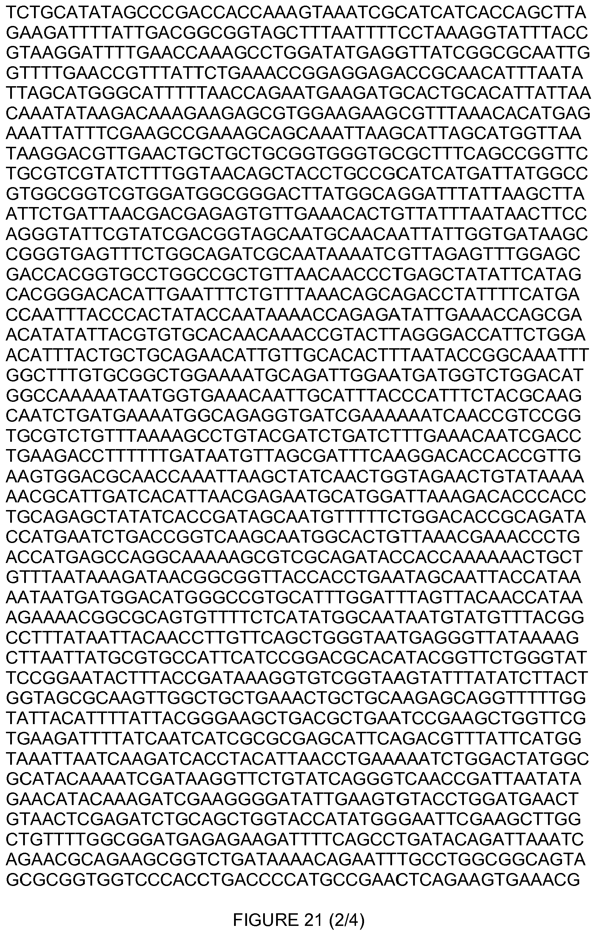

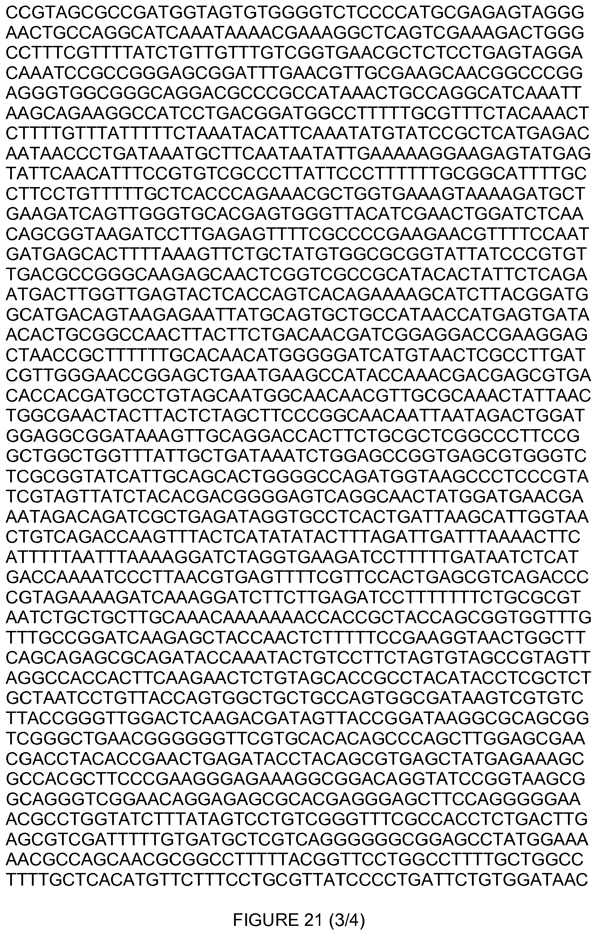

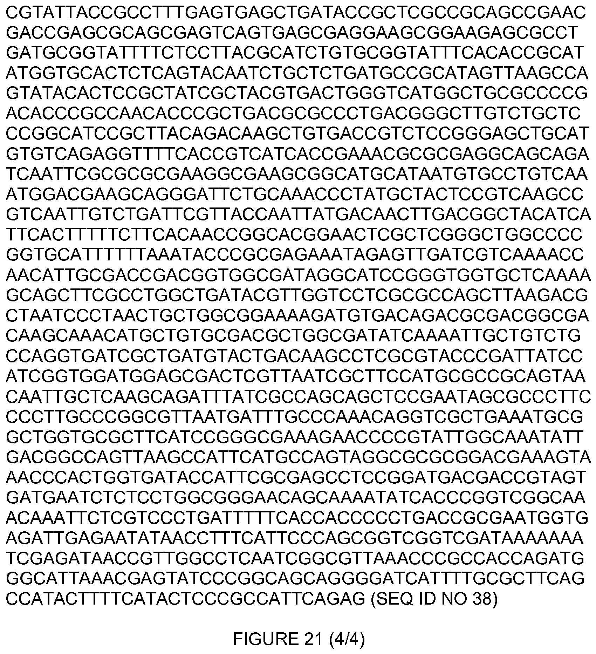

- La figure 21 représente la séquence plasmidique du vecteur pBAD-ACL_0729.

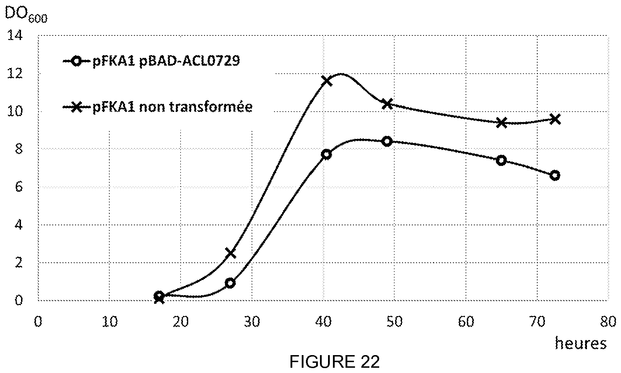

- La figure 22 est un diagramme de l'évolution de la densité optique à 600nm du milieu de culture (ordonnée) de la souche PKA1 non transformée (contrôle - croix) ou transformée avec le vecteur pBAD-ACL0729 (ronds) en fonction du temps en heure (abscisse).

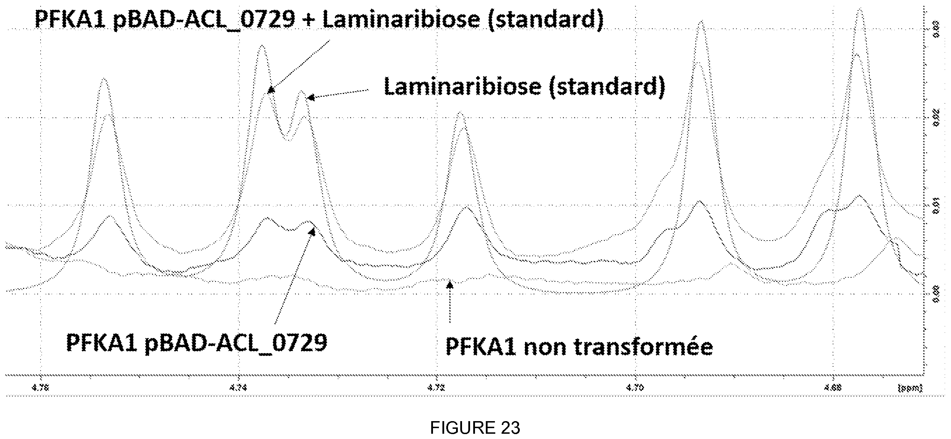

- La figure 23 correspond aux spectres RMN (normalisés grâce à l'ajout d'acide triméthylsilylpropanoïque) d'échantillons du laminaribiose commercial, du surnageant de culture avec la souche PFKA1 transformée avec le plasmide pBAD-ACL0729 (avec ou sans ajout de laminaribiose commercial) ou du surnageant de culture avec la souche PFKA1 non transformée après 49 heures de culture.

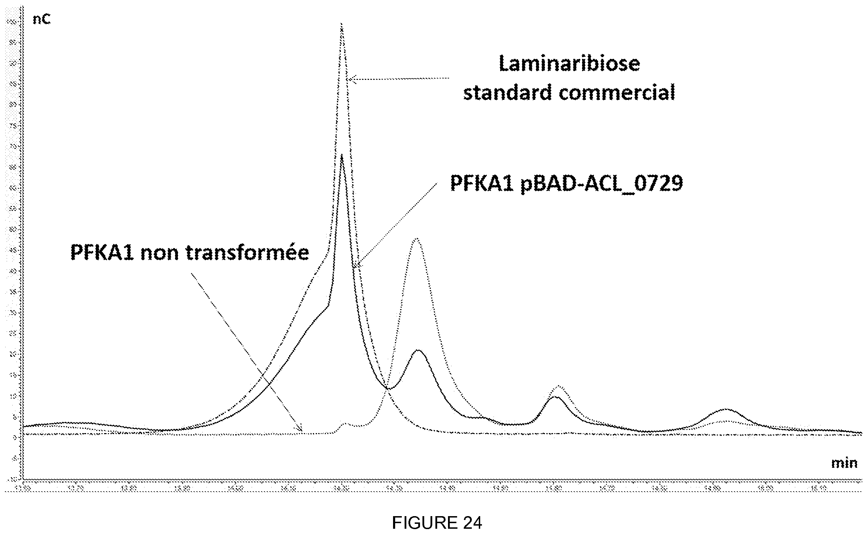

- La figure 24 représente les chromatogrammes de différents échantillons analysés par HPAEC-PAD, la courbe en trait continu correspond au chromatogramme du surnageant de culture de la souche PFKA1 transformée avec pBAD-ACL0729, la courbe en pointillés correspond au chromatogramme du surnageant de culture de la souche PFKA1 non transformée, la courbe en trait pointillé discontinus correspond au standard commercial du laminaribiose.

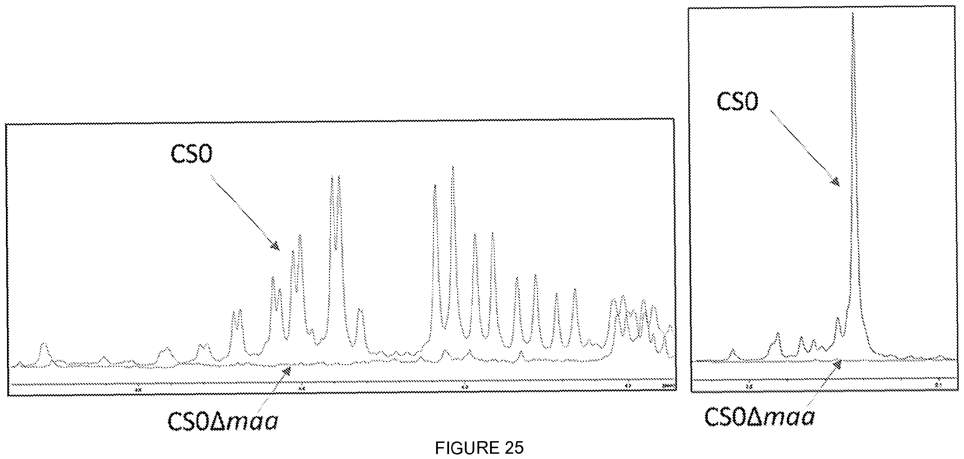

- La Figure 25 correspond aux spectres RMN d'échantillons de milieu de culture de la souche PFKA1 (CS0) ou d'échantillons de milieu de culture de la souche CS0Δmaa, transformées avec le vecteur pBAD -Teth1788.

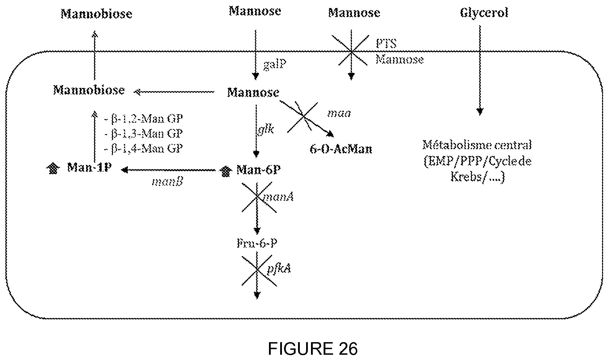

- La Figure 26 est un schéma métabolique de la souche CS1 pour la production de mannobiose à partir de glycérol et de mannose, en découplant la production du mannobiose de la croissance cellulaire. Sur cette figure les abréviations signifient : galP : galactose perméase, PTS : Phosphotransférases, glk : Glucokinase, maa : maltose acétyltransférase, manA : mannose-6-phosphate isomérase, manB : phosphomannomutase, Man-1P : Mannose-1-Phosphate, Man-6P : Mannose-6-Phosphate, Fru-6-P : Fructose-6-Phosphate, pfkA : ATP-dependent 6-phosphofructokinase isozyme 1, Teth514-1788 (β-1,2-Man_GP): β-1,2-oligomannane phosphorylase, Uhgb_MP (b-1,4-Man_GP) : β-1,4-mannobiose-phosphorylase/β-1,4-mannopyranosyl-[N-glycan] phosphorylase, β-1,3-Man_GP: b-1,3-mannobiose-phosphorylase.

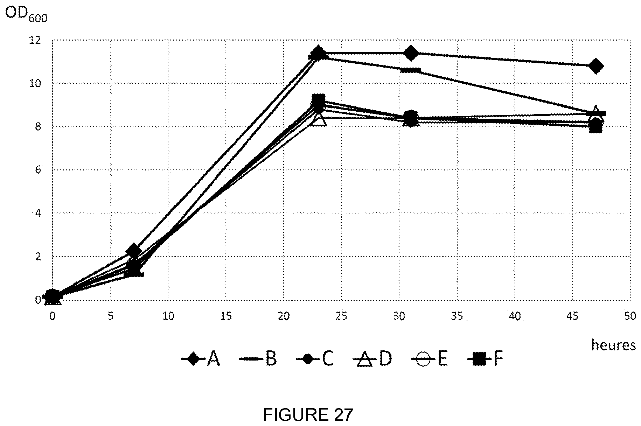

- La figure 27 représente les courbes de croissance des souches CS0 (losanges (A)), CS0-galP-Teth1788 (tirets (B)), CS1-galP-Teth1788 (ronds pleins (C)), CS1 IG-Teth1788 (triangles (D)), CS1-galP-Lin0857 (ronds vides (E)) et CS1 IG-gaP-Lin0857 (carrés (F)). L'ordonné correspond à la Densité Optique mesuré à 600 nm (OD₆₀₀) et l'abscisse au temps en heures (heures).

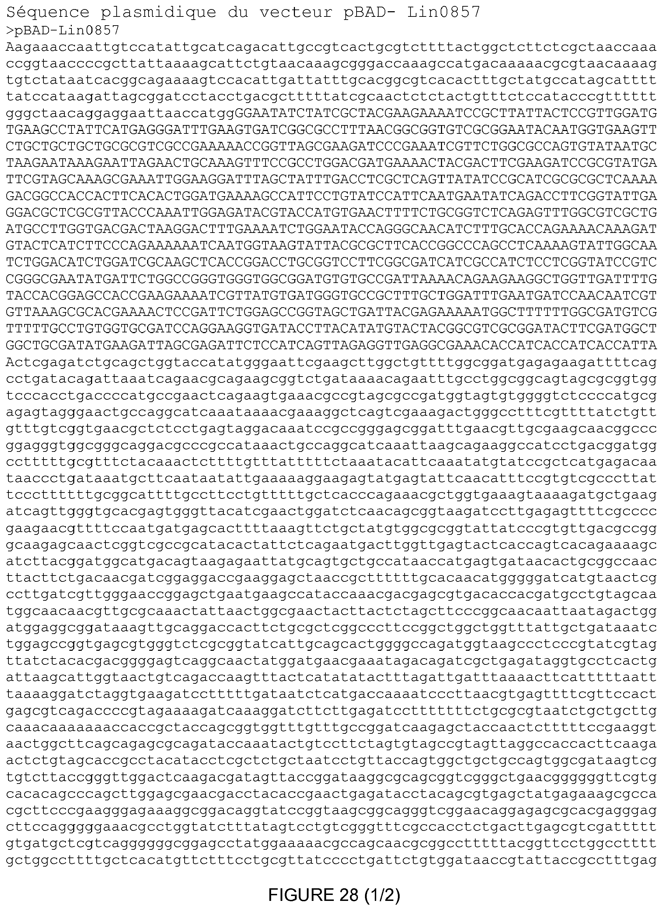

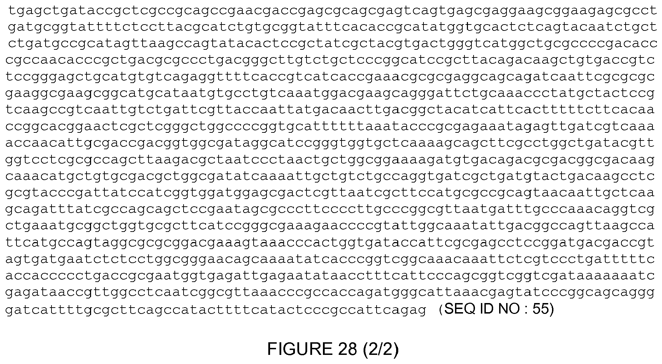

- La figure 28 représente la séquence plasmidique du vecteur pBAD-Lin0857.

### EXEMPLES

### Exemple 1 : fabrication et caractérisation de la souche déposée à la CNCM sous le numéro CNCM I-5499

Dans l'exemple ci-dessous, la souche déposée à la CNCM (Collection Nationale de Culture de Microorganismes, Institut Pasteur, 25 rue du Docteur Roux, 75724 Paris Cedex 15, France), sous le numéro CNCM I-5499 est également désignée souche PFKA.

### 1) Préparation et production de la souche PFKA (CNCM n° I-5499)/Validation phénotypique

La souche déposée à la CNCM (Collection Nationale de Culture de Microorganismes, Institut Pasteur, 25 rue du Docteur Roux, 75724 Paris Cedex 15, France), sous le n° CNCM I-5499 a été obtenue par modification génétique de la souche *Escherichia coli* MDO.

La souche *E. coli* MDO, a été obtenue et caractérisée dans le laboratoire Chimie et Biotechnologie des Oligosaccharides (CBO) (CERMAV-CNRS CS40700, 38041 Grenoble cedex 9, France) à partir de la souche *Escherichia coli* ZLKA tel que décrit dans Fierfort et Samain « Genetic engineering of Escherichia coli for the economical production of sialylated oligosaccharides » J Biotechnol. 2008 Apr 30;134(3-4):261-5. [3].

La souche *E. coli* ZLKA dérive de la souche *E. coli* K-12 DH1 (*endA1 recA1 gyrA96 thi-1 glnV44 relA1 hsdR17)* dont les gènes *lacZ, nanKETA* et *lacA* ont été inactivés tel que décrit dans Fierfort et Samain « Genetic engineering of Escherichia coli for the economical production of sialylated oligosaccharides » J Biotechnol. 2008 Apr 30;134(3-4):261-5 [3].

La souche *E. coli* MDO se caractérise par l'inactivation des gènes *melA, wcaJ* et *mdoH* ainsi que par l'insertion du promoteur Plac en amont du gène *gmd* codant pour une GDP-mannose 4,6-dehydratase. En conséquence, le gène *manB* de la phosphomannomutase, qui est situé en aval du gène *gmd,* est inductible par l'isopropyl β-D-1-thiogalactopyranoside (IPTG). Le génotype complet de la souche MDO est le suivant : *endA1 recA1 gyrA96 thi-1 glnV44 relA1 hsdR17 lacZ-wcaF, Plac nanKETA lacA melA wcaJ mdoH.*

Dans le présent exemple, la kanamycine et l'ampicilline ont été utilisées à une concentration finale de 50 µg/mL pour assurer le maintien des plasmides pWKS-GalP et pBAD-Teth-1788 ou pBAD-Uhgb_MP respectivement dans les cellules. La concentration finale d'IPTG utilisée pour l'induction de galP et manB était de 60 µM. La concentration finale de L-arabinose pour l'induction du gène codant pour la glycoside-phosphorylase (Teth-1788, UhgbMP ou β-1,3-mannooligosaccharide phosphorylase) était de 10 mM. L'IPTG a été ajouté dans le milieu de culture au moment de l'inoculation et le L-arabinose est ajouté à la culture en début de phase de croissance exponentielle (D0₆₀₀ ≈ 0,4).

### 1.1. Insertion du promoteur Plac en aval du gène gmd

Un fragment d'ADN de 302 paires de base (pb) incluant la séquence du promoteur d'expression Plac a été intégré dans le chromosome entre le codon *stop* du gène *wcaF* et le codon *start* du gène *gmd.* Deux séquences d'ADN, un segment de 0,88 kb se terminant 5 pb en aval du codon *stop* du gène *wcaF* et un segment de 0,96 kb démarrant 13 pb en amont du codon *start* du gène *gmd* ont été amplifiés par PCR à partir de la séquence d'ADN génomique d'E. *Coli* K-12, utilisée comme matrice. Le cycle de PCR était 1 cycle de 30 secondes (s) à 98°C, 35 cycles de 15 s à 98°C, 15 s à 69°C et 30 s à 72°C, 1 cycle de 5 minutes à 72°C. La Polymérase utilisée était la Phusion, (NEB). Les amorces PL1 (5' CTCGAGAGGCGATATTTTTCCCTGTATCC SEQ ID NO 1) et PL2 (5'GGTTTGCGTATTATTCAGTTTCAACGCGTTCG SEQ ID NO 2) ont été utilisées pour amplifier le fragment en amont et les amorces PL5 (5'CTGGAGCTCAGAGGAATAATACATGTCAAAAGTCG SEQ ID NO 3) et PL6 (5' CTCGAGACAACAGCGATAATCACATCACC SEQ ID NO 4) ont été utilisées pour amplifier le fragment aval. Un segment d'ADN de 0,3 kb incluant la séquence du promoteur Plac a été amplifié par PCR en utilisant le plasmide pBluescript II KS (Addegen) comme matrice et les amorces suivantes: PL3 (5' GTTGAAACTGAATAATACGCAAACCGCCTCTC SEQ ID NO 5) et PL4 (5' ATGTATTATTCCTCTGAGCTCCAGCTTTTGTTCC SEQ ID NO 6). Les amorces PL2 et PL3 ont des séquences adjacentes en 5' qui les rendent complémentaires l'une de l'autre. Les amorces PL4 et PL5 ont été conçues de manière similaire. Les trois fragments d'ADN amplifiés ont été épissés par chevauchement en utilisant les amorces PL1 et PL6. Le fragment d'ADN de 2,1 kb ainsi obtenu a été digéré avec l'enzyme de restriction *Xhol* puis cloné dans le site de restriction *Sali* du vecteur suicide pKO3. L'insertion chromosomique du promoteur Plac a ensuite été réalisée selon le protocole décrit dans Link, A.J., Phillips, D., Church, G.M., 1997. Methods for generating precise deletions and insertions in the genome of wild-type Escherichia coli: application to open reading frame characterization. J. Bacteriol. 179, 6228-6237. [7]. Le plasmide pKO3 a été fourni par le laboratoire Church (Harvard University, USA). Les clones positifs ont été criblés par PCR (Le cycle de PCR était 1 cycle de 3 minutes à 95°C, 35 cycles de 30 s à 95°C, 30 s à 53°C et 1 minute à 68°C, 1 cycle de 5 minutes à 68°C. La polymérase utilisée était la DNA Taq polymerase (NEB) pour la présence d'un fragment amplifié d'ADN de 1,396 kb en utilisant les amorces PL3 et PL7 (5'TCGAGGTCATTAGCCACCA SEQ ID NO 7).

### 1.2. Génération des mutants de délétion

Les gènes sélectionnés *ptsG, manXYZ* et *pfkA* ont été inactivés à l'aide du protocole pKO3 tel que décrit dans Link, A.J., Phillips, D., Church, G.M., 1997. Methods for generating precise deletions and insertions in the genome of wild-type Escherichia coli: application to open reading frame characterization. J. Bacteriol. 179, 6228-6237, 1997 [7]. Pour obtenir le mutant de délétion du gène *ptsG,* un segment d'ADN de 351 pb situé entre les nucléotides 568 et 919 de *ptsG* a été supprimé et remplacé par la séquence 5'AGC comme suit: deux segments d'ADN flanquant la séquence à supprimer ont été amplifiés par PCR. Le cycle de PCR était 1 cycle de 30 s à 98°C, 35 cycles de 15 s à 98°C, 15 s à 70°C et 30 s à 72°C, 1 cycle de 5 minutes à 72°C. La polymérase utilisée était la Phusion (NEB) utilisant l'ADN génomique de la souche *E. coli* K-12 comme matrice. Le fragment de 900 pb en amont a été amplifié avec les amorces (5'CTAGGATCCGCCGAAAATTGGGCGGTGAATAAC SEQ ID NO 8) et (5'GTAAAGCTTCTGGTAAGCAGCCCAGAGAAG SEQ ID NO 9) et le fragment en aval de 958 pb a été amplifié avec les amorces (5'CTCAAGCTTGCGCCGATCCTGTACATCATCC SEQ ID NO 10) et (5'CGTGTCGACCGTTAATCCTAATCTGCCACGCACC SEQ ID NO 11). Les deux fragments amplifiés ont été ligués au niveau de leur site de restriction *HindIII* terminal et clonés ensemble au niveau des sites de restrictions *BamHI* et *Sali* du vecteur suicide pKO3. La délétion a ensuite été réalisée selon le protocole décrit dans Link, A.J., Phillips, D., Church, G.M., 1997. Methods for generating precise deletions and insertions in the genome of wild-type Escherichia coli: application to open reading frame characterization. J. Bacteriol. 179, 6228-6237 [7].

Dans le fond génétique /souche décrite ci-dessus, un fragment de 1,219 kb situé entre les nucléotides 332 de *manX* et 522 de *manY* a été supprimé pour obtenir le mutant de délétion des gènes *manXYZ,* comme suit : deux fragments d'ADN flanquant la séquence à supprimer ont été amplifiés par PCR en utilisant l'ADN génomique de la souche *E. coli* K-12 comme matrice. Le segment en amont de 915 pb a été amplifié avec les amorces (5'ACTCGAGAATGGCGATGAAGAGAG SEQ ID NO 12) et (5'CCAGTGCCACCAGTTCATC SEQ ID NO 13), et le fragment en aval de 902 pb a été amplifié avec les amorces (5'CAAGCTTCGATTCCGGAAGTGGTGAC SEQ ID NO 14) et (5'AGGATCCATGCCCCCATGACAAACAG SEQ ID NO 15). Les deux fragments amplifiés ont été ligués au niveau de leur site de restriction *HindIII* terminal et clonés ensemble au niveau des sites de restrictions *BamHI* et *Sali* du vecteur suicide pKO3. La délétion a ensuite été réalisée selon le protocole décrit dans Link, A.J., Phillips, D., Church, G.M., 1997. Methods for generating precise deletions and insertions in the genome of wild-type Escherichia coli: application to open reading frame characterization. J. Bacteriol. 179, 6228-6237 [7]. La souche ainsi obtenue a été nommée « MGX ».

Pour obtenir le mutant de délétion du gène *pfkA* dans le fond génétique de la souche MGX, un fragment d'ADN de 396 pb situé entre les nucléotides 146 et 542 du gène *pfkA* a été supprimé et remplacé par la séquence (5'AAGCTT) comme suit : deux fragments d'ADN flanquant la séquence à supprimer ont été amplifiés par PCR en utilisant l'ADN génomique d'*E*. *coli* K-12 comme matrice. Le fragment en amont de 890 pb a été amplifié avec les amorces (5'GGATCCAGGCGTCGGGGATATCGTG SEQ ID NO 16) et (5'AAGCTTCGGTCTTCATACAGACCCAGATAGC SEQ ID NO 17) et le fragment en aval de 931 pb a été amplifié avec les amorces (5'AAGCTTGGCCATTGCCGGGGGCTGTG SEQ ID NO 18) et (5'CTCGAGCCACCGTGTGACTGACGAATC SEQ ID NO 19). Les deux fragments amplifiés ont été ligués au niveau de leur site de restriction *HindIII* terminal et clonés au niveau des sites de restrictions *BamHI* et *Sali* du vecteur suicide pKO3. La délétion a ensuite été réalisée selon le protocole décrit dans Link, A.J., Phillips, D., Church, G.M., 1997. Methods for generating precise deletions and insertions in the genome of wild-type Escherichia coli: application to open reading frame characterization. J. Bacteriol. 179, 6228-6237 [7]. La souche ainsi obtenue est nommée « PFKA ».

### 1.3. Clonage du gène galP sur un plasmide (amorces et séquence plasmidique)

Un fragment d'ADN de 1,471 kb contenant la séquence du gène *galP* a été amplifié par PCR. Le cycle de PCR était 1 cycle de 30 s à 98°C, 35 cycles de 15 s à 98°C, 15 s à 68°C et 30 s à 72°C, 1 cycle de 5 minutes à 72°C. La polymérase utilisée était la Phusion (NEB) en utilisant l'ADN génomique d'E. *coli* K-12 comme matrice avec les amorces suivantes: (5'TTGTCGACTTAAGGAGGGCATCATGCCTGAC SEQ ID NO 20) et (5'GTTCTAGATGACTGCAAGAGGTGGCTTCC SEQ ID NO 21). Le fragment amplifié a ensuite été cloné au niveau des sites de restriction *Sali* et *Xbal* du vecteur d'expression pWKS130 tel que décrit dans Rong Fu Wang, Kushner, S.R., 1991. Construction of versatile low-copy-number vectors for cloning, sequencing and gene expression in Escherichia coli. Gene 100, 195-199 [10] pour former le plasmide pWKS-GalP. La séquence plasmidique résultante est représentée sur la figure 14 et correspond à la séquence d'acides nucléique SEQ ID NO 22. Ce plasmide a été transformé dans les souches MGX et PFKA pour donner les souches « MGX1 » et « PFKA1 » respectivement.

Le tableau 7 ci-dessous regroupe les différentes souches et leur génotype.

**Tableau 7 : Génotype des mutants générés**

| **Souche châssis** | **Génotype** |
|---|---|
| MDO | *endA1 recA1 gyrA96 thi-1 glnV44 relA1 hsdR17 lacZ-wcaF::Plac nanKETA lacA melA wcaJ mdoH* |
| MGX1 | *MDO* + *ptsG manXYZ pKWS-galP* |
| PFKA1 | *MDO*+ *ptsG manXYZ pfkA pKWS-galP* |

### 1.4. Validation génétique du mutant de délétion des gènes codants pour les transporteurs PTS et le gène pfkA

Une vérification de l'effectivité des délétions des gènes ciblés a été effectuée via des contrôles par PCR sur les souches MDO, MGX et PFKA.

### Mutants de délétion des gènes des systèmes de transport PTS

Les mutants dans le système de transport PTS ont été construits par délétion d'un fragment de 350 pb du gène ptsG et d'un fragment de 1200 pb de l'opéron manXYZ comme décrit ci-dessus.

Les PCRs ont été réalisées en utilisant de l'ADN génomique de chaque souche comme matrice en utilisant les amorces suivantes pour la vérification de la délétion du fragment du gène ptsG,
ptsG_FW: (5'CTTCTCTCAGTGGGCTGCTTACCAG 3' SEQ ID NO 23),
ptsG_RV: (5'CCGTTAATCCTAATCTGCCACGCACC 3' SEQ ID NO 24).

Le cycle de PCR était 1 cycle de 3 minutes à 95°C, 35 cycles de 30 s à 95°C, 30 s à 55°C et 1 minute à 68°C, 1 cycle de 5 minutes à 68°C. la polymérase utilisée était la DNA Taq polymerase (NEB). La taille attendue des fragments amplifiés est de 1300 pb pour la souche sauvage (WT) et de 950 pb pour les souches MGX et PFKA si les constructions génétiques sont correctes et comprennent la délétion.

Les amorces suivantes ont été utilisées pour la vérification de la délétion du fragment de l'opéron manXYZ :
manXYZ_FW (5'TGTTAGGCGAGCAGGAAAACGTCG 3' SEQ ID NO 25) manXYZ_RV (5'ACCAATCACACCCAGAGCAACCAG 3' SEQ ID NO 26). Dans ce cas, la taille des fragments amplifiés attendus est de 1600 pb pour la souche sauvage (WT) et de 450 pb pour les souches MGX et PFKA si les constructions génétiques sont correctes et comprennent la délétion.

Le contrôle de la PCR est réalisé par migration du fragment amplifié dans un gel d'agarose 1% dans une cuve d'électrophorèse MUPID-ONE (Eurogentec, Liege, Belgium). 5 µL de produit PCR sont homogénéisés avec 1 µL de tampon de charge 6x" (NEB, Ipswich, MA) puis déposés sur gel pour une migration dans un tampon TAE 1X (Sigma, Seelze, Germany). 5 µL de marqueur de taille1 kB (NEB) sont également déposés dans un autre puit. La migration est faite à 100 V pendant 30 minutes. Le gel est ensuite incubé 10 minutes dans une solution contenant 0,01% d'éthidium bromide (Euromedex, Souffelweyersheim, France). La détection des fragments amplifiés se fait via Gel DOC EZ (Biorad, Berkeley, CA) suivant les instructions du constructeur.

La figure 1 est une photographie qui présente les produits PCR des fragments d'ADN amplifiés depuis le génome des souches MDO, MGX et PFKA, et ayant migré sur un gel d'agarose.

Tel que représenté et démontré sur la figure 1, la taille des fragments amplifiés par PCR correspond à celle attendue. En effet, les fragments amplifiés depuis la souche MDO (sans délétion) ont une taille de 1300 pb et de 1600 pb pour les fragments du gène ptsG et de l'opéron manXYZ respectivement, au contraire de celles des fragments amplifiés depuis les souches MGX et PFKA, qui correspondent à celles attendues, à savoir respectivement environ 950 pb et 450 pb avec les délétions réalisées.

### Mutants de délétion du gène pfkA

Le mutant de délétion du gène pfkA a été construit par délétion d'un fragment de 400 pb du gène pfkA comme décrit ci-dessus. Les PCRs ont été réalisées en utilisant de l'ADN génomique de chaque souche comme matrice en utilisant les amorces suivantes pour la vérification de la délétion du fragment du gène pfkA,
pfkA_FW: (5' CATTTTGCATTCCAAAGTTCAGAGG3' SEQ ID NO 27), pfkA_RV: (5' TCATCGGTTTCAGGGTAAAGGAATCT 3' SEQ ID NO 28). Le cycle de PCR était1 cycle de 3 minutes à 95°C, 35 cycles de 30 s à 95°C, 30 s à 55°C et 1 s à 68°C, 1 cycle de 5 minutes à 68°C. La polymérase utilisée était la DNA Taq polymérase (NEB). La taille attendue du fragment amplifié est de 1000 pb pour la souche MDO et MGX et de 600 pb pour la souche PFKA si les constructions génétiques sont correctes et comprennent la délétion.

Le contrôle de la PCR est réalisé par migration du fragment d'ADN amplifié dans un gel d'agarose 1% dans une cuve d'électrophorèse MUPID-ONE (Eurogentec, Liege, Belgium). 5 µL de produit PCR sont homogénéisés avec 1 µL de tampon de charge 6x" (NEB, Ipswich, MA) puis déposés sur gel pour une migration dans un tampon TAE 1X (Sigma, Seelze, Germany). 5 µL de marqueur de taille1 kB (NEB) sont également déposés dans un autre puit. La migration est faite à 100 V pendant 30 minutes. Le gel est ensuite incubé 10 minutes dans une solution contenant 0,01% d'éthidium bromide (Euromedex, Souffelweyersheim, France). La détection des fragments d'ADN amplifiés se fait via Gel DOC EZ (Biorad, Berkeley, CA) suivant les instructions du constructeur.

La figure 2 est une photographie qui présente les produits PCR des fragments d'ADN amplifiés depuis le génome des souches MDO, MGX et PFKA, et ayant migrés sur un gel d'agarose.

Tel que représenté et démontré sur la figure 2, la taille du fragment amplifié depuis le génome de la souche PFKA était effectivement inférieure à celle observée pour les souches MDO et MGX, et correspond à la taille attendue à savoir environ 600 pdb, démontrant que la délétion est présente et correcte.

### 1.5. Validation phénotypique du mutant de délétion des gènes codants pour les transporteurs PTS et sur-exprimant le gène galP

Il est connu que la délétion des gènes *ptsG* and *manXYZ* diminue la capacité de transport du glucose dans la cellule d'E. *Coli.* (Quanfeng Liang,1 Fengyu Zhang,1 Yikui Li,1 Xu Zhang,1 Jiaojiao Li,1 Peng Yang,1 and Qingsheng Qia,1 Comparison of individual component deletions in a glucose-specific phosphotransferase system revealed their différent applications Sci Rep. 2015; 5: 13200. Published online 2015 Aug 19. [28], Sonja Steinsiek and Katja Bettenbrock Glucose Transport in Escherichia coli Mutant Strains with Defects in Sugar Transport Systems, J Bacteriol. 2012 Nov; 194(21): 5897-5908. [29]). Pour vérifier que les mutants de délétion des gènes codants pour des protéines des systèmes de transport dit « PTS » (PhosphoTransférase System) - souche nommée MGX - a le phénotype attendu, les souches MDO et MGX ont été cultivées dans un milieu minimum M9 (M9 milieu minimum - M9 sels: Na₂HPO₄ 12H₂O 17.4 g/L, KH₂PO₄ 3.02 g/L, NaCl 0,51 g/L, NH₄Cl 2,04 g/L. Trace métaux et sels: Na₂EDTA 2 H₂O 15 mg/L, ZnSO₄ 7 H₂O 4,5 mg/L, CoCl₂ 6H₂O 0,3 mg/L, MnCl₂ 4H₂O 1 mg/L, H₃BO₃ 1 mg/L, Na₂MoO₄ 2 H₂O 0,4 mg/L, FeSO₄ 7 H₂O 3 mg/L, CuSO₄ 5 H₂O 0,3 mg/L. MgSO₄ 0,5 g/L CaCl₂ 4,38 mg/L, Thiamine hypochloride 0,1 g/L) en utilisant le glucose comme seule source de carbone. De plus, pour vérifier que la sur-expression du gène *galP* permet de restaurer tout ou partie de la capacité de transport du glucose et donc le taux de croissance, le plasmide pWKS-GalP a été transformé dans la souche MGX. La souche résultante sélectionnée sur milieu LB+Kanamycine a été nommée MGX1 (voir ci-dessus). Cette souche MGX1 a également été cultivée dans le milieu M9 avec du glucose.

Cent microlitres d'une culture sur la nuit (10 à 12 heures de culture en milieu LB (Tryptone 10g/L, Extrait de levure 5g/L, NaCl, 1 0g/L) des souches *E. coli* MDO, MGX et MGX1 ont été transférés dans des erlenmeyers bafflés de 250 mL contenant 50 mL de milieu M9 minimum supplémenté avec du D-glucose (3 g/L) comme seule source de carbone. Les erlenmeyers ainsi inoculés ont été incubés pendant 16 h à 37°C avec une agitation orbitale de 220 tours par minutes. Les cellules ont ensuite été collectées par centrifugation (Sigma, Seelze, Germany) pendant 10 min à 2 000g à température ambiante, à savoir 25 °C, lavées avec du milieu M9 stérile et utilisées pour inoculer des erlenmeyers bafflés de 250 ml contenant 50 mL de milieu M9 supplémenté avec du D-glucose (3 g/L) à DO₆₀₀ₙₘ ∼0,1. Pour déclencher l'expression du gène *galP* porté par le plasmide pWKS-GalP, de l'IPTG a été ajouté dans le milieu de culture de la souche MGX1 à une concentration finale de 60µM. Les cultures des souches ont été réalisées à 37°C avec une agitation orbitale de 220 tours par minutes (220 tours par minutes). La croissance a été suivie par mesure de la turbidimétrie à une densité optique de 600 nm en utilisant un spectrophotomètre Genesys 6 (Thermo, USA). Les résultats sont présentés Figure 3.

Tel que représenté sur la figure 3, au contraire de la croissance de la souche MDO, celle de la souche MGX se caractérise par une longue phase de latence (de 10h au minium) ce qui est une trait phénotypique reporté dans la littérature, de ce type de souche (Liang, Q., Zhang, F., Li, Y., Zhang, X., Li, J., Yang, P., Qi, Q., 2015. Comparison of individual component deletions in a glucose-specific phosphotransferase system revealed their différent applications. Sci. Rep. 5, 13200. [6]). Le taux de croissance (µ) maximal de cette souche est de 0,25 h⁻¹ (calculé entre t=23h et t=34h), soit approximativement la moitié du taux de croissance de la souche MDO. L'induction de l'expression du gène *galP,* restaure en partie, le taux de croissance. En effet, le profil de croissance de la souche MGX1 est très similaire à celui de la souche MDO, avec une absence de phase de latence et un taux de croissance (µ=0,32h⁻¹) atteignant 70% de celui de la souche MDO (µ=0,45 h⁻¹). Les résultats démontrent donc clairement que la souche MGX1 possède des capacités de transport supérieures par rapport à la souche MGX.

### 1.6. Validation phénotypique du mutant de délétion PTS-PFKA

### a. Capacité de croissance

La capacité de croissance des souches MDO, MGX, MGX1, PFKA1 a été déterminée par culture indépendante desdites souches, cultivées dans le milieu M9 supplémenté avec du glucose.

Cent microlitres d'une culture sur la nuit (10 à 16 heures de culture en milieu LB (Tryptone 10g/L, Extrait de levure 5g/L, NaCl, 10g/L) des souches E. coli MDO, MGX et MGX1, PFKA1 ont été transférés dans des erlenmeyers bafflés de 250 mL contenant 50 mL de milieu M9 supplémenté avec du D-glucose (3 g/L) comme seule source de carbone. Les erlenmeyers ainsi inoculés ont été incubés pendant 16 h à 37°C avec une agitation orbitale de 220 tours par minutes (220 rpm). Les cellules ont ensuite été collectées par centrifugation (Sigma, Seelze, Germany) pendant 10 min à 2 000g à température ambiante, à savoir 25 °C lavées avec du milieu M9 stérile et utilisées pour inoculer des erlenmeyers bafflés de 250 ml contenant 50 mL de milieu M9 supplémenté avec du D-glucose (3 g/L) à DO600nm ~0,1. Pour déclencher l'expression du gène galP porté par le plasmide pWKS-GalP, de l'IPTG a été ajouté dans le milieu de culture de la souche MGX1 et PFKA1 à une concentration finale de 60µM. Les cultures des souches ont été réalisées à 37°C avec une agitation orbitale de 220 tours par minutes (220 rpm). La croissance a été suivie par mesure de la turbidimétrie à une densité optique de 600 nm en utilisant un spectrophotomètre Genesys 6 (Thermo, USA).

La capacité de croissance des souches a été déterminée par mesure du taux de croissance tel que décrit dans Growth Rates Made Easy Barry G. Hall, Hande Acar, Anna Nandipati, Miriam Barlow Author Notes Molecular Biology and Evolution, Volume 31, Issue 1, January 2014, Pages 232-238, https://doi.org/10.1093/molbev/mst187 28 October 2013 des différentes souches.

Les résultats obtenus sont représentés dans le tableau 8 ci-dessous.

**Tableau 8 : Taux de croissance des différentes souches cultivées en milieu M9 glucose (3 g/L)**

| Souche châssis | Taux de croissance en milieu M9 glucose (h⁻¹) |
|---|---|
| MDO | 0,45 |
| MGX | 0,18 |
| MGX1 | 0,32 |
| PFKA1 | 0,15 |

Comme indiqué ci-dessus, le gène *pfka,* codant pour la phosphofructokinase, a été supprimé du génome de la souche *E. coli* MGX, générant la souche *E. coli* PFKA (voir ci-dessus). Les résultats obtenus montrent clairement que l'absence de *pfka,* a provoqué une diminution du taux de croissance en milieu M9 avec du glucose (3g/L), et cette souche n'est plus capable de croître en milieu M9 supplémenté en glucose (données non présentées). L'introduction du gène GalP dans ce fond génétique (souche *E. coli* PFKA) a permis l'obtention de la souche désignée *E. coli* PFKA1. Le taux de croissance de la souche *E. coli* PFKA1 mesuré était proche de celui de la souche MGX montrant une « restauration » de la capacité de croissance de la souche malgré l'absence du gène *pfka* (0,15 h⁻¹ soit un tiers du taux de croissance de la souche MDO).

### b. Analyse métabolomique

Un autre trait phénotypique caractéristique du mutant de délétion du gène *pfka* est l'accumulation intracellulaire de Glucose-6-Phosphate (G6P) et de Fructose-6-Phosphate (F6P) (Ishii, N., Nakahigashi, K., Baba, T., Robert, M., Soga, T., Kanai, A., Hirasawa, T., Naba, M., Hirai, K., Hoque, A., Ho, P.Y., Kakazu, Y., Sugawara, K., Igarashi, S., Harada, S., Masuda, T., Sugiyama, N., Togashi, T., Hasegawa, M., Takai, Y., Yugi, K., Arakawa, K., Iwata, N., Toya, Y., Nakayama, Y., Nishioka, T., Shimizu, K., Mori, H., Tomita, M., 2007. Multiple High-Throughput Analyses Monitor the Response of E. coli to Perturbations. Science 316, 593-597. [4]) La vérification de l'accumulation de Glucose-1-Phosphate (G1P) et de Mannose-1-Phosphate

(M1P), des monosaccharides phosphorylés nécessaires à la synthèse d'oligosaccharides par les glycoside-phosphorylases (GP), dans la souche PFKA1 a été réalisé par analyse quantitative du métabolome (métabolites intracellulaires). En particulier, la détermination de la concentration d'hexoses phosphates, en particulier du Glucose-1-Phosphate (G1P) et du Mannose-1-Phosphate (M1P) a été réalisé comme suit.

### b.1. Echantillonnage et extraction des métabolites intracellulaires.

Cent microlitres d'une culture en milieu LB (lysogeny broth) (10 g/l de NaCl), sur la nuit (soit 12 à 16 heures), des différentes souches ont été utilisés pour inoculer des erlenmeyers bafflés de 50 mL contenant 10 mL de milieu M9 supplémenté avec du glucose à une concentration finale de 3 g/L. Les erlenmeyers ont été incubés pendant 24h à 37°C avec une agitation orbitale de 220 tours par minutes (rpm). Les cellules ont été collectées par centrifugation (Sigma, Seelze, Germany) pendant 10 min à 2 000g et à température ambiante (25°C), puis lavées avec du milieu M9 dilué cinq fois, et utilisées pour inoculer, à une densité optique à 600nm de DO₆₀₀ₙₘ = 0,1, des erlenmeyers bafflés de 50 mL contenant 50 mL de milieu M9 dilué supplémenté avec 3 g/L de glucose et incubées à 37°C avec une agitation orbitale de 220 tours par minutes (rpm). La croissance cellulaire a été suivie par mesure de la turbidimétrie à une densité optique de 600 nm en utilisant un spectrophotomètre Genesys 6 (Thermo, USA). En phase exponentielle de croissance, 120 µL de la culture ont été échantillonnés et immédiatement mélangés avec 1,25 mL d'une solution à -20°C d'acétonitrile/méthanol/H₂O (4:4:2) pour simultanément bloquer l'activité métabolique et extraire les métabolites ; 60 µL d'un extrait cellulaire contenant des métabolites complétement marqués au ¹³C ont été ajoutés dans chaque échantillon pour servir d'étalons internes tel que décrit dans Mashego, M.R., Wu, L., Van Dam, J.C., Ras, C., Vinke, J.L., Van Winden, W.A., Van Gulik, W.M., Heijnen, J.J., 2004. MIRACLE: mass isotopomer ratio analysis of U-13C-labeled extracts. A new method for accurate quantification of changes in concentrations of intracellular metabolites. Biotechnol. Bioeng. 85, 620-628. [9] et Wu, L., Mashego, M.R., van Dam, J.C., Proell, A.M., Vinke, J.L., Ras, C., van Winden, W.A., van Gulik, W.M., Heijnen, J.J., 2005. Quantitative analysis of the microbial metabolome by isotope dilution mass spectrometry using uniformly 13C-labeled cell extracts as internal standards. Anal. Biochem. 336, 164-171. [12]

Les échantillons ont été placés à -20°C pendant 20 minutes, puis centrifugés 10 minutes à 10 000g à 4°C pour éliminer les débris cellulaires. Le surnageant a ensuite été séché sous vide en utilisant un concentrateur sous vide (SC110A SpeedVac Plus, ThermoSavant, Waltham, MA, USA) et conservé à -80°C jusqu'à analyse.

### b.2. Analyse par IC-ESI-HRMS des métabolites.

Pour l'analyse, les échantillons ont été repris dans 120 µL d'eau ultrapure, puis analysés par chromatographie ionique (Thermo Scientific Dionex ICS-5000+ system, Dionex, Sunnyvale, CA, USA) couplée à un spectromètre de masse (LTQ Orbitrap mass spectrometer, Thermo Fisher Scientific, Waltham, MA, USA) équipé d'une source d'ionisation de type électrospray. La méthode de chromatographie ionique est une variante de celle décrite par Kiefer et al., 2007 (Kiefer, P., Nicolas, C., Letisse, F., Portais, J.-C., 2007. Détermination of carbon labeling distribution of intracellular metabolites from single fragment ions by ion chromatography tandem mass spectrometry. Anal. Biochem. 360, 182-188. [5]). Le gradient de KOH a été modifié comme suit: 0 min, 0,5 mM; 1 min, 0,5 mM; 9,5 min, 4,1 mM; 12,5 min, 30 mM; 24 min, 50 mM; 36 min, 60 mM; 36,1 min, 90 mM; 43 min, 90 mM; 43,5 min, 0,5 mM; 48 min, 0,5 mM. Tous les gradients sont linéaires. La suppression du signal lié au gradient de KOH a été réalisée par un suppresseur anionique électrochimique (AERS 300 - 2 mm, Dionex, Sunnyvale, CA, USA). Le courant d'électrolyse appliqué était de 87 mA, en mode de régénération externe à l'aide d'eau ultrapure. Le volume d'injection des échantillons était de 35 µL. L'analyse en spectrométrie de masse a été réalisée en mode négatif, à une résolution of 30 000 en mode « fullscan », avec les paramètres de source suivants: température du capillaire de source, 350°C; température de la source, 300°C; débit du « sheath gas », 50 a.u. (unité arbitraire); gaz auxiliaire, 5 a.u; niveau de radiofréquence (RF) de la S-Lens, 60%; et voltage du spray d'ionisation, 3.5 kV. Les données ont été acquises avec le logiciel Xcalibure et traitées avec le logiciel TraceFinder 3.1 (Thermo Fisher Scientific, Waltham, MA, USA). A l'aide de courbes de calibrations, les concentrations intracellulaires de chaque métabolite pour les différentes souches ont été déterminées. La figure 4 présente les rapports de concentration de chaque métabolite entre les souches MGX1 et PFKA1 et la souche MDO, pour mieux visualiser les différences. Les rapports ont été calculés en Log₂. Tel que représenté sur la figure 4, les concentrations des métabolites dans la souche MGX1 et celles dans la souche MDO sont très similaires et aucune différence significative n'a été mise en évidence. La délétion du système de transport PTS n'a donc pas d'effet majeur sur cette partie du réseau métabolique.

Tel que démontré sur la figure 4, le profil d'accumulation des métabolites dans la souche PFKA1 est différent de celui de la souche MDO. Par exemple, la concentration intracellulaire du Fructose-1,6-Bisphosphate (FBP) est plus faible que celle mesurée dans la souche MDO. L'absence de la phosphofructokinase permet avantageusement une augmentation des concentrations intracellulaires en F6P et en G6P (Ishii, N., Nakahigashi, K., Baba, T., Robert, M., Soga, T., Kanai, A., Hirasawa, T., Naba, M., Hirai, K., Hoque, A., Ho, P.Y., Kakazu, Y., Sugawara, K., Igarashi, S., Harada, S., Masuda, T., Sugiyama, N., Togashi, T., Hasegawa, M., Takai, Y., Yugi, K., Arakawa, K., Iwata, N., Toya, Y., Nakayama, Y., Nishioka, T., Shimizu, K., Mori, H., Tomita, M., 2007. Multiple High-Throughput Analyses Monitor the Response of E. coli to Perturbations. Science 316, 593-597. [4]). Tel que représenté et démontré sur la figure 4 la concentration intracellulaire d'autres monosaccharides phosphorylés est avantageusement également plus élevée dans la souche PFKA1. Par exemple, les concentrations de Mannose-6-Phosphate (M6P), de Galactose-1-Phosphate (Gal1P) et la concentration totale en Glucose-1-Phosphate (G1P) et Mannose-1-Phosphate (M1P) - ces deux monosaccharides phosphorylés ne pouvant être séparés ni par chromatographie ionique ni par spectrométrie de masse, sont significativement augmentées dans la souche PFKA1. Ces résultats démontrent donc clairement que la souche selon l'invention permet avantageusement d'accumuler des monosaccharides phosphorylés nécessaires à la synthèse d'oligosaccharides. Ces résultats démontrent également clairement que la souche selon l'invention permet avantageusement, notamment de par l'accumulation des monosaccharides phosphorylés nécessaires à la synthèse d'oligosaccharides, d'être un support et/ou moyen avantageux pour la production d'oligosaccharides.

En outre, les résultats démontrent clairement que les souches selon l'invention permettent la synthèse d'oligosaccharides et avantageusement leur excrétion dans le milieu de culture. Ainsi la production, la récupération et l'isolement des oligosaccharides ne nécessitent aucune altération ou destruction des souches, permettant avantageusement une production en continu.

### Exemple 2 : production d'oligosaccharides avec la souche déposée à la CNCM (Collection Nationale de Culture de Microorganismes, Institut Pasteur, 25 rue du Docteur Roux, 75724 Paris Cedex 15, France), sous le N°CNCM I-5499

Dans cet exemple la souche déposée à la CNCM sous le N°CNCM I-5499 également désignée souche PFKA1 ou souche *E. Coli* PFKA1 dans l'exemple 1 ci-dessus a été utilisée pour la production d'oligosaccharides. Pour ce faire, la souche a été transformée avec des vecteurs d'expressions comme suit.

### 1. Transformation de la souche PFKA1 avec les plasmides portant les gènes codant pour une GP

### 1.1. Clonage du gène codant pour l'enzyme Teth514-1788 dans les plasmides pBAD-HisA et pTRC (amorces et séquence plasmidique)

Le gène codant pour la protéine Teth514-1788 de la souche *Thermoanaerobacter* sp. X-514 (Teth514-1788, numéro d'accès Genbank ABY93073.1) appartenant à la famille GH130 de la classification CAZy (http://www.cazy.org/) (Lombard, V., Golaconda Ramulu, H., Drula, E., Coutinho, P.M., Henrissat, B., 2014. The carbohydrate-active enzymes database (CAZy) in 2013. Nucleic Acids Res. 42, D490-D495 [30]) a été synthétisé et cloné dans le vecteur pBAD HisA par la société Biomatik (Biomatik, Ontario, Canada) entre les sites de restriction *NcoI* et *XhoI.*

La séquence plasmidique obtenue correspond à la séquence SED ID NO 29 représentée sur la figure 15. La souche transformée a été mise en culture en milieu M9 supplémenté en mannose (20 g.L⁻¹) à 37°C sous agitation 180 tours par minutes (180 rpm) durant 6 jours. L'ajout de L-arabinose à 0,1% dans le milieu de culture lorsque la DO₆₀₀ est ≈ 0,4 permet l'expression du gène présent dans le plasmide et la production d'une protéine de fusion Teth514-1788- (His)₆, de 36,7 kDa.

Le gène codant pour la protéine Teth514-1788 a également été cloné dans le vecteur pTRC-His-A entre les sites de restriction *BamH1* et *EcoR1.* Le vecteur pTRC-His-A et l'insert (séquence codante de l'enzyme Teth514-1788) ont été digérés par les enzymes de restrictions *BamH1* et *EcoR1* suivant le protocole du fournisseur (https://international.neb.com/protocols/2014/05/07/double-digest-protocol-with-standard-restriction-enzymes [47]). Préalablement, les sites de restrictions *BamH1* et *EcoR1* ont été ajoutés aux extrémités de l'insert par réaction de polymérisation en chaîne (PCR) en utilisant les amorces fw : 5'-GAGGAAGGATCCATGGGCATTAAACTG-3' (SEQ ID NO 31) et rev : 5'-GCTGCAGATGAATTCTTAATGGTG-3' (SEQ ID NO 32) suivant le protocole du fournisseur : https://international.neb.com/protocols/0001/01/01/pcr-protocol-m0530 [48]. La ligation de l'insert et du vecteur purifiés sur gel d'agarose est faite par la T4 DNA ligase suivant le protocole du fournisseur (https://international.neb.com/protocols/0001/01/01/dna-ligation-with-t4-dna-ligase-m0202 [49]).

La souche transformée a été mise en culture dans un milieu M9 supplémenté en mannose (20 g.L⁻¹) à 37°C sous agitation à 180 rpm durant 6 jours. L'ajout d'isopropyl-β-D-thiogalactopyranoside (IPTG) à 1 mM dans le milieu de culture lorsque la DO₆₀₀ est ≈ 0,4 permet l'expression du gène qui conduit à la synthèse d'une protéine recombinante (His)e-Teth514-1788, de 38,9 kDa.

La souche *E. coli* PFKA1 a été transformée soit avec le plasmide pBAD-His-Teth514-1788 soit avec le plasmide pTRC-His-Teth514-1788.

Le procédé de transformation était identique quel que soit le plasmide et correspondait au protocole classique de transformation de cellules chimio-compétentes. (https://www.addgene.org/protocols/bacterial-transformation/ [50])

La protéine Teth514-1788-(His)₆ est également nommée Teth-1788 dans la présente.

Les souches transformées ont été mises en culture en erlenmeyer dans un milieu M9 supplémenté en mannose (Figure 5). Les conditions de réalisation de ces cultures étaient identiques à celles de l'exemple 1 ci-dessus.

Des profils d'expressions des protéines ont été déterminés par gel d'électrophorèse SDS-PAGE Any KD de chez Bio-Rad. Les culots cellulaires sont lysés dans du tampon Tris HCl 5 mM contenant du lysosyme (Euromedex ref 5934-C) à 0,5 mg.mL⁻¹ et de la DNAse (NEB, ref M0303L) à 50 µL (100 U) dans 100 mL de tampon Tris HCl 5 mM. Tous les culots ont été repris dans un volume de tampon pour être à une DO₆₀₀ de 45 et incubés 30 min à 37 °C puis congelés à - 20 °C. Les échantillons ont été décongelés, centrifugés 25 min à 5000g. 15 µL de chaque surnageant a été repris avec 5 µL de loading dye blue (NEB, ref 50994905) ont été incubés 10 min à 95 °C avant d'être déposé sur gel pour une migration de 35 min à 100V dans une cuve d'électrophorèse afin de vérifier si la protéine Teth-1788 était effectivement produite dans la souche PFKA1 transformée avec les vecteurs pBAD-His-Teth514-1788 ou pTRC-His-Teth514-1788.

Tel que démontré sur la figure 5A, les résultats obtenus démontrent une production des protéines Teth-1788 suite à l'induction quel que soit le vecteur d'expression utilisé. En effet, comme démontré sur la figure 5 A, après ajout dans le milieu de l'inducteur, à savoir l'arabinose ou IPTG, l'intensité des bandes correspondantes au poids moléculaire des protéines Teth-1788 augmente, avec une différence concernant la cinétique de production intracellulaire de l'enzyme.

### Cet exemple démontre clairement que la souche E. coli PFKA1 peut être i) transformée avec des vecteurs d'expression ; et/ou ii) utilisée pour l'expression de gènes codant pour des protéines recombinantes

### 1.2 Clonage du gène codant pour l'enzyme Uhgb_MP dans les plasmides pBAD-HisA et pTRCa (amorces et séquence plasmidique)

Le gène codant pour la protéine « Unknown human gut bacteria_Mannoside-Phosphorylase », une β-1,4-mannopyranosyl-chitobiose phosphorylase appartenant à la famille GH130 (Uhgb_MP, numéro d'accès Genbank ADD61463.1) a été cloné dans le vecteur pBAD HisA avec les amorces : Uhgb fw : 5' - CACCATGAGTATGAGTAGCAAAGTTATT -3' (SEQ ID NO 33) et Uhgb rev : 5' - GATGATGCTTGTACGTTTGGTAAATTC - 3' (SEQ ID NO 34).

Le procédé de clonage était celui décrit dans le document http://tools.thermofisher.com/content/sfs/manuals/pentr dtopo_man.pdf [51].

La séquence plasmidique obtenue correspond à la séquence SED ID NO 30 représentée sur la figure 16. La souche transformée a été mise en culture dans un milieu M9 supplémenté en mannose (20 g.L⁻¹) à 37°C sous agitation à 180rpm durant 6 jours. L'ajout de L-arabinose à 0,1% dans le milieu de culture lorsque la DO₆₀₀ est ≈ 0,4 permet l'expression du gène présent dans le plasmide et la production d'une protéine recombinante Uhgb_MP-(His)₆, de 56,3 kDa (en fusion avec une thioredoxine).

Le gène codant pour la protéine de fusion Uhgb_MP-(His)₆, a également été cloné dans le vecteur pTRC-His-A entre les sites de restriction *BamH1* et *EcoR1.* La souche transformée a été mise en culture dans un milieu M9 supplémenté en mannose (20 g.L⁻¹) à 37°C sous agitation (180 tours par minutes (rpm), Infors HT Multitron) durant 6 jours. L'ajout d'isopropyl-β-D-thiogalactopyranoside (IPTG) à 1 mM dans le milieu de culture lorsque la DO₆₀₀ est ≈ 0,4 pour expression du gène par ajout d'isopropyl-β-D-thiogalactopyranoside (IPTG) qui a conduit à la synthèse d'une protéine de fusion (His)e-Uhgb_MP, de 43,6 kDa.

La souche *E. coli* PFKA1 a été transformée soit avec le plasmide pBAD-His-Uhgb_MP soit avec le plasmide pTRC-His- Uhgb_MP.

Le schéma de la figure 6 représente le métabolisme central de la souche *E*. *coli* PFKA1 transformée indiquant les modifications des voies métaboliques génétiquement modifiées, la connexion avec les réactions de synthèse de β-1,2-mannobiose et β-1,4-mannobiose par les glycoside-phosphorylases (GPs) respectives et les effets sur l'accumulation intracellulaire des substrats de ces enzymes.

### 1.3 Clonage du gène codant pour une enzyme β-1,3-mannooligosaccharide phosphorylase dans le plasmide pBAD-HisA (amorces et séquence plasmidique)

Le gène synthétique codant pour la protéine d'une β-1,3-mannooligosaccharide phosphorylase a été fourni par la société Biomatik Limited (Cambridge, Ontario, Canada) avec une optimisation de l'usage des codons pour une expression du gène dans *E. coli.* Cette enzyme appartient à la famille GH130 (numéro d'accession GigaBase 340101.Vvad_PD3074).

Ce gène originellement dans le plasmide pET23a(+) a été cloné dans le plasmide pBAD HisA par la méthode "In-Fusion cloning" avec les amorces : fw : 5' - GAGGAATTAACCATGCTGAGCGTGGAAAAACGCTG -3' (SEQ ID NO 35) et rev : 5' - AGCTGCAGATCTCGATCAGTGGTGGTGGTGGTGGTGCTCGA - 3' (SEQ ID NO 36).

Le procédé de clonage était celui décrit sur le site du fournisseur du kit de clonage : https://www.takarabio.com/learning-centers/cloning/in-fusion-cloning-overview [58].

La séquence plasmidique obtenue correspond à la séquence SED ID NO 37 représentée sur la figure 20. La souche transformée a été mise en culture dans un milieu M9 supplémenté en mannose (20 g.L⁻¹) à 37°C sous agitation à 180 rpm durant 6 jours. L'ajout de L-arabinose à 0,1% dans le milieu de culture lorsque la DO₆₀₀ était de = 0,4 a permis l'expression du gène présent dans le plasmide et la production de la protéine recombinante (His)₆-β-1,3-mannooligosaccharide phosphorylase, de masse molaire 44,4 kDa.

La souche transformée a été mise en culture dans un milieu M9 supplémenté en mannose (20 g.L⁻¹) à 37°C sous agitation à 180 rpm durant 6 jours. La souche *E. coli* PFKA1 a été transformée avec le plasmide pBAD-β-1,3-mannooligosaccharide phosphorylase.

Le schéma de la figure 6 représente le métabolisme central de la souche *E*. *coli* PFKA1 transformée indiquant les modifications des voies métaboliques génétiquement modifiées, la connexion avec les réactions de synthèse de β-1,2-mannobiose, β-1,3-mannobiose et β-1,4-mannobiose par les glycoside-phosphorylases (GPs) respectives et les effets sur l'accumulation intracellulaire des substrats de ces enzymes.

### 1.4 Clonage du gène codant pour une enzyme laminaribiose phosphorylase dans le plasmide pBAD-HisA (amorces et séquence plasmidique)

Le gène codant pour la protéine ACL0729 de la souche *Acholeplasma laidlawii PG-8A* (ACL0729, numéro d'accès Genbank ABX81345.1) appartenant à la famille GH94 de la classification CAZy (http://www.cazy.org/) (Lombard, V., Golaconda Ramulu, H., Drula, E., Coutinho, P.M., Henrissat, B., 2014. The carbohydrate-active enzymes database (CAZy) in 2013. Nucleic Acids Res. 42, D490-D495 [30]) a été synthétisé et cloné dans le vecteur pBAD HisA par la société GeneCust (GeneCust, Boynes, France) entre les sites de restriction *NcoI* et *XhoI.*

La séquence plasmidique obtenue correspond à la séquence SED ID NO 38 représentée sur la figure 21. La souche transformée a été mise en culture en milieu M9 supplémenté en glucose (15 g.L⁻¹) à 37°C sous agitation 180 tours par minutes (180 rpm) durant 3 jours. L'ajout de L-arabinose à 0,1% dans le milieu de culture lorsque la DO₆₀₀ est ≈ 0,4 a permisl'expression du gène présent dans le plasmide et la production d'une protéine de fusion ACL0729- (His)₆, de 96,9 kDa.

### 2. Production de β-1,2- mannobiose, du β-1,3- mannobiose, du β-1,4-mannobiose et du laminaribiose (β-1,3-glucobiose)

### 2.1. Production de β-1,2- mannobiose

Des cultures sur la nuit (de 10 à 15 heures) en milieu LB des souches *E. coli* PFKA1-pBAD-Teth-1788 et PFKA1 non transformée (condition contrôle) ont été utilisées pour inoculer à une DO₆₀₀ ≈ 0,1 une culture de 50 mL de milieu M9 additionné de D-Mannose à 3 g/L comme unique source de carbone dans un erlenmeyer bafflé de 250 mL. Les erlenmeyers ont été incubés pendant 24h à 37°C avec agitation orbitale de 220 rpm. Après 24 heures, les cellules ont été récoltées par centrifugation pendant 10 min à 2 000g à température ambiante, à savoir de 24°C, lavées par suspension des cellules dans un milieu M9 et utilisées pour inoculer à DO₆₀₀ ≈ 0,1 un bioréacteur de 500 mL contenant 350mL de milieu M9 « modifié » (M9 modifié sels: KH₂PO₄ 3.02 g/L, NaCl 0,51 g/L, NH₄Cl 2,04 g/L, (NH₄)₂SO₄ 5 g/L. Trace métaux et sels: Na₂EDTA 2 H₂O 15 mg/L, ZnSO₄ 7 H₂O 4,5 mg/L, CoCl₂ 6H₂O 0,3 mg/L, MnCl₂ 4H₂O 1 mg/L, H₃BO₃ 1 mg/L, Na₂MoO₄ 2 H₂O 0,4 mg/L, FeSO₄ 7 H₂O 3 mg/L, CuSO₄ 5 H₂O 0,3 mg/L. MgSO₄ 0,5 g/L CaCl₂ 4,38 mg/L, Thiamine hypochloride 0,1 g/L) supplémenté avec 10 g/L de D-mannose. Les paramètres de fermentation, à savoir un pH de 7,0, une température de 37°C, une pression partielle en O₂ dissout (pO2) supérieure ou égale à 30%, une agitation supérieure ou égale à 500 rpm ont été suivis et contrôlés à l'aide d'un système de bioréacteurs Multifors (Infors, Suisse). La croissance a été estimée par mesure de la turbidimétrie du milieu de culture à une densité optique de 600 nm en utilisant un spectrophotomètre Genesys 6 (Thermo, USA). Des échantillons de culture de la souche PFKA1 - pBAD-Teth-1788 et de la souche contrôle PFKA1 ont été collectés à différents temps de la culture et analysés par résonance magnétique nucléaire (RMN).

### 2.2. Identification des produits par RMN

Des prélèvements réguliers du milieu de culture (1 mL) ont été centrifugés 2 min à 18 000g et 500 µL des surnageants ont été mélangés à 100 µL de D₂O contenant 2,35 g/L d'acide 3-(triméthylsilyl)-1-propanesulfonique tétra-deutéré (TSPd4), composé utilisé comme standard interne pour l'analyse par RMN. Les spectres ¹H-RMN ont été acquis avec un spectroscope RMN Avance 500 MHz équipé d'une sonde BBI 5 mm (Bruker, Rheinstatten, Allemagne). Le traitement des spectres et la quantification des métabolites ont été réalisés avec le logiciel Topspin 3.1 (Bruker, Rheinstatten, Allemagne). Les surnageants de cultures obtenus avec les souches PFKA1-pBAD-Teth-1788 ou PFKA1 ont ainsi été analysés tout au long de la culture. La figure 7 représente les spectres RMN de surnageants de milieu de cultures obtenus avec les souches PFKA1-pBAD-Teth-1788 (courbe supérieure) ou PFKA1 non transformée. Tel que représenté sur la figure 7, les pics caractéristiques du β-1,2-mannobiose dans les cultures PFKA1-pBAD-Teth-1788 ont été détectés alors qu'aucun de ces pics n'est présent dans le surnageant des cultures contrôles PFKA1. En effet, les déplacements chimiques observés sur le spectre des surnageants de culture PFKA1-pBAD-Teth-1788 correspondaient aux pics des atomes d'hydrogène liés au carbone 1 de la partie réductrice du β-1,2-mannobiose (H1 - ManA) et au carbone 1 du mannose de l'extrémité non réductrice du β-1,2-mannobiose (H1 - ManB), donnés à 5,31 et 4,79 ppm respectivement (Faille, C., Michalski, J.C., Strecker, G., Mackenzie, D.W., Camus, D., Poulain, D., 1990. Immunoreactivity of neoglycolipids constructed from oligomannosidic residues of the Candida albicans cell wall. Infect. Immun. 58, 3537-3544. [1], Shibata, N., Hisamichi, K., Kikuchi, T., Kobayashi, H., Okawa, Y., Suzuki, S., 1992. Sequential nuclear magnetic résonance assignment of.beta-1,2-linked mannooligosaccharides isolated from the phosphomannan of the pathogenic yeast Candida albicans NIH B-792 strain. Biochemistry 31, 5680-5686 [11]).

Les résultats obtenus démontrent clairement que la souche selon l'invention permet avantageusement la production d'oligosaccharides. En outre, les résultats obtenus démontrent clairement que la souche selon l'invention permet avantageusement et de manière surprenante et inattendue une excrétion des oligosaccharides produits dans le milieu de culture.

En d'autres termes, les résultats démontrent clairement que la souche selon l'invention permet la synthèse d'oligosaccharides et avantageusement leurs excrétions dans le milieu de culture.

### 2.3. Analyse de la présence de β-1,2-mannobiose dans le surnageant de culture par Chromatographie Haute-Performance Echangeuse d'Anion avec Détection par Ampérométrie Pulsée (HPAEC-PAD)

Une confirmation de la présence de β-1,2-mannobiose dans le milieu extracellulaire a été réalisée via une méthode d'analyse orthogonale. Les échantillons analysés correspondaient à des surnageants de culture de la souche PFKA1 transformée avec pBAD-Teth-1788 ou de la souche PFKA1 telles que décrites ci-dessus.

Des échantillons de 1 mL de surnageants de cultures ont été soumis à un choc thermique de 10 min à 95°C, puis centrifugés 10 minutes à 15 000g et ont été filtrés sur une membrane de 0,22 µm. Une dilution par 25 dans de l'eau ultrapure des échantillons filtrés a été réalisée avant analyse. La séparation des molécules a été réalisée sur une colonne PA100 Dionex Carbopac 2 × 250 mm (Thermo Fisher) avec un gradient d'élution à 0,25 mL/min avec les éluants A (H₂O), B (150mM NaOH) et C (150mM NaOH+ 500mM acétate de sodium) suivant: 0-3 min, 50%A-50%B; 3 min, 100%B; 3-6 min, 100%B; 6-12 min, 50%B-50%C; 12 min, 5%B-95%C; 12-15 min, 5%B-95%C; 15 min, 50%A-50%B; 15-23 min, 50%A-50%B. La détection a été réalisée par un module Dionex ED40 possédant une électrode en or et une référence de pH Ag/AgCl.

Par ailleurs, une production *in vitro* (sans la souche) à partir d'un mélange réactionnel enzymatique avec la Teth-1788 β-1,2-mannobiose phosphorylase a été réalisée en utilisant du mannose à 300 mM, de l'α-D-Mannose 1-Phosphate à 15 mM, de l'enzyme Teth-1788 à 0,16 mg.mL⁻¹ dans un volume total de 10 mL de tampon tris-HCl 20 mM, pH7. La réaction a été réalisée pendant 24 heures dans un bain marie à 37 °C sous agitation. Le β-1,2-mannobiose obtenue a été purifié comme décrit dans Chiku K, Nihira T, Suzuki E, Nishimoto M, Kitaoka M, et al. (2014) Discovery of Two b-1,2Mannoside Phosphorylases Showing Différent Chain-Length Specificities from Thermoanaerobacter sp. X-514. PLoS ONE 9(12): e114882. doi:10.1371/journal.pone.0114882 [61]

Les résultats obtenus, à savoir les chromatogrammes sont représentés sur la figure 8. En particulier, sur cette figure, la courbe en trait continu gras correspond au chromatogramme du surnageant de culture de la souche PFKA1 transformée avec pBAD-Teth-1788, la courbe en pointillé correspond au chromatogramme du surnageant de culture de la souche PFKA1 non transformée, la courbe en trait continu fin correspond à un standard de D-mannose (Carbosynth). Tel que démontré sur la figure 8, la souche selon l'invention permet avantageusement la production d'oligosaccharides. En outre, les résultats obtenus démontrent que les oligosaccharides produits sont excrétés dans le milieu permettant avantageusement une récupération facilitée de l'oligosaccharide produit.

### 2.4. Profil de production du β-1,2-mannobiose en bioréacteur

Une étude de la production de β-1,2-mannobiose a été suivie par RMN au cours de cultures en bioréacteur avec les souches MDO, MGX, MGX1 et PFKA1.

Les souches ont été préalablement transformées avec le plasmide pBAD-Teth-1788. Les conditions de culture de chacune des souches étaient identiques à celles mentionnées ci-dessus. La figure 9 présente le profil de production du β-1,2-mannobiose par la souche PFKA1 transformée avec le plasmide pBAD-Teth-1788. Sur cette figure 9 sont également reportées l'évolution de la densité optique à 600 nm et celle de la concentration en mannose.

La détermination des concentrations ont été réalisée par RMN à partir d'échantillons du surnageant du milieu de culture selon le procédé décrit dans Nord LI, Vaag P, Duus JØ Quantification of organic and amino acids in beer by 1H NMR spectroscopy Anal Chem. 2004 Aug 15;76(16):4790-8 [59] ou Gloriadel Campo, IñakiBerregi, RaúlCaracena, J. IgnacioSantos "Quantitative analysis of malic and citric acids in fruit juices using proton nuclear magnetic résonance spectroscopy" Analytica Chimica Acta, Volume 556, Issue 2, 25 January 2006, Pages 462-468 [60] en utilisant 1 mM de TSP (Acide triméthylsilylpropanoïque) comme standard interne pour calculer la concentration en β-1,2-mannobiose.

La concentration finale en β-1,2-mannobiose dans le milieu de culture était de 1,80 mM et le rendement en β-1,2-mannobiose par g de mannose consommé est de 9,02% (g/g).

De la même façon et en suivant le même protocole, la production de β-1,2-mannobiose a été évaluée en bioréacteur pour les souches MDO, MGX et MGX1, transformées avec le plasmide pBAD-HisA-Teth514-1788 selon le procédé décrit ci-dessus afin de déterminer l'effet de chaque mutation sur la production de β-1,2-mannobiose. Le tableau 9 regroupe les caractéristiques de production calculées pour les différentes souches à partir des concentrations en β-1,2-mannobiose et en mannose résiduelle déterminées par RMN.

**Tableau 9 : Titres et rendements en β-1,2-mannobiose dans les surnageants de cultures en bioréacteur des souches MDO, MGX, MGX1 et PFKA1 exprimant l'enzyme Teth-1788. Les concentrations sont exprimées en mM et les rendements en pourcentage de mannose converti en β-1,2-mannobiose.**

| Souche | Caractéristique | Titre Man₂ (mM) | % Rendement (g Man₂/ g Man) |
|---|---|---|---|
| MDO-Teth-1788 | Souche contrôle | ND | ND |
| MGX-Teth-1788 | PTS-, pas de GalP; manB non induit | 0,21 | 1,02 |
| MGX1-Teth-1788 | PTS- avec GalP induit, manB induit | 0,59 | 2,91 |
| PFKA1-Teth-1788 | PTS- avec GalP; délétion de pfka; manB induit | 1,8 | 9,02 |

| | | | |
|---|---|---|---|
| *ND. Non détecté* | | | |

Tel que démontré ci-dessus, dans le milieu de culture de la souche MDO, le β-1,2-mannobiose n'est pas détecté alors que dans la souche MGX, il est présent en très faible concentration mais quantifiable (0,21 mM). Aussi, il semble que la délétion du gène codant pour le PTS permet l'import du mannose dans la cellule sous une forme non phosphorylée, qui est l'un des substrats de la glycoside-phosphorylase. La souche MGX1 présente une augmentation de la production de β-1,2-mannobiose (0,59 mM). Cette augmentation peut être liée à deux facteurs : 1) l'amélioration du transport du mannose par la sur-expression de GalP et 2) l'augmentation de la concentration intracellulaire en mannose-1-Phosphate (M1P) (en comparaison avec la souche MGX) due à la sur-expression du gène *manB* dont le produit de ce gène, la protéine ManB, catalyse la formation de M1P à partir de Mannose-6-Phosphate (M6P). M1P est le second substrat de la glycoside-phosphorylase et le niveau intracellulaire de M1P est important pour la biosynthèse de β-1,2-mannobiose. De manière surprenante et inattendue, la production de β-1,2-mannobiose par la souche PFKA1 est triplée par rapport à la souche MGX1 (1,8 mM), ce qui représente une augmentation approximativement d'un facteur 9 par rapport à la souche MGX. Avantageusement, l'augmentation intracellulaire de M1P a possiblement un effet dans l'obtention de ce résultat.

Tel que démontré ci-dessus, la souche selon l'invention permet avantageusement et de manière surprenante d'augmenter significativement la production d'oligosaccharides. En outre les résultats obtenus démontrent clairement que la souche selon l'invention permet d'augmenter significativement les rendements de production des oligosaccharides et ainsi d'optimiser/réduire les coûts de production.

### Purification du β-1,2-mannobiose synthétisé in cellulo

Une analyse du β-1,2-mannobiose produit par la souche PFKA1-pBAD-Teth1788 a été réalisée. Pour ce faire 12 mL de surnageant de culture de la souche PFKA1-pBAD-Teth1788 ont été soumis à un choc thermique de 8 min à 95°C, centrifugés 10 min à 15 000g à 15°C. Le surnageant a été filtré sur membrane (sartorius, Minisart) en acétate de cellulose avec des pores de 0,22 µm avant d'être lyophylisé et repris dans 500 µL d'H₂O au total, dans 2 vials avec insert (250 µL chacun). La purification a été réalisée avec une HPLC 1260 infinity (Agilent) couplée à un collecteur de fractions automatique UltiMate 3000 (Thermo scientific). Une colonne asahipak NH2P-50 4E (Shodex) permet la séparation des composés *via* une élution isocratique d'un mélange acétonitrile/H₂O (70/30 respectivement), à un débit de 1 mL/min. Les composés ont été détectés par indice de réfraction (RI). La pureté du β-1,2-mannobiose a été estimée avant (figure 10A) et après la purification (figure 10B). Deux cycles de purification consécutifs ont permis d'obtenir une pureté HPLC ≥ 90 % (tableau 10). La pureté a été calculée en faisant le rapport de l'aire sous le pic du β-1,2-mannobiose et de l'aire sous l'ensemble des pics du chromatogramme.

**Tableau 10 : pourcentage d'aire sous la courbe**

| n° Pic | **1** Contamin ant | **2** Contamin ant | **3** β-1,2-manz | **4** Contami nant | **5** Contamina nt | **6** Contaminant |
|---|---|---|---|---|---|---|
| Aire relativ e (%) | 1,04 | 2,10 | **91,47** | 4,07 | 0,67 | 0,65 |

A partir des chromatogrammes, les titres et les rendements de purification obtenus ont été calculés à partir de courbes étalons représentées sur la figure 17. Le procédé utilisé correspond à celui décrit dans Quantification of Sugar Compounds and Uronic Acids in Enzymatic Hydrolysates of Lignocellulose Using High-Performance Anion Exchange Chromatography with Pulsed Amperometric Détection Energy Fuels 2012, 26, 5, 2942-2947 [53]

Le tableau 11 ci-dessous résume les résultats de rendements et de purification obtenus.

**Tableau 11 : Rendements de purification du β-1,2-mannobiose à partir de 12 mL de surnageant de culture PFKA1-pBAD-Teth-1788.**

| Volume de surnageant culture | Masse de β-1,2-mannobiose (purifié) |
|---|---|
| 12 mL | 6,8 mg |
| Titre dans le surnageant de culture après purification : 567 mg.L⁻¹ | |
| Titres dans le surnageant de culture avant purification, calculé à partir des données RMN, 950 mg.L⁻¹ et des données HPAEC-PAD, 1260 mg.L⁻¹. | |
| Rendement de purification : 45% (HPAEC-PAD), 60 % (RMN) | |

Tel que démontré ci-dessus, la souche selon l'invention permet avantageusement de produire des oligosaccharides avec des rendements importants. Par ailleurs, tel que démontré ci-dessus, la souche selon l'invention permet avantageusement l'accumulation des oligosaccharides produits dans le milieu de culture. En outre, la récupération de l'oligosaccharide produit est facilitée à partir du milieu de culture.

### 3. Production de β-1,4- mannobiose

### 3.1. Production du β-1,4- mannobiose et identification par RMN

Des cultures de la souche *E. coli* PFKA1 non transformée (condition contrôle), transformée avec le plasmide pBAD-UhgbMP ou transformée avec le plasmide pTRC-UhgbMP ont été réalisées en erlenmeyer selon le protocole décrit dans l'exemple 1. Une étude du profil de croissance de ces 3 cultures a été réalisée. L'analyse du profil de croissance a été réalisée tel que décrit ci-dessus. La figure 11 représente les profils de croissance des différentes cultures. Tel que représentés sur cette figure les profils de croissance sont similaires pour les souches *E. coli* PFKA1 et PFKA1-pBAD-UhgbMP et montrent après 72 heures un plateau. Ce plateau est atteint après 120 heures de culture pour ce qui concerne la souche *E. coli* PFKA1-pTRC-UhgbMP.

Un surnageant de la culture avec la souche *E. coli* PFKA1-pBAD-UhgbMP a été analysé par RMN en appliquant les mêmes paramètres que ceux décrits précédemment. La figure 12 présente la zone spectrale caractéristique du β-1,4-mannobiose d'un surnageant récolté d'une culture de la souche *E. coli* PFKA1 sans le plasmide (Condition contrôle), d'un surnageant de la culture de la souche *E. coli* PFKA1_pBAD-UhgbMP et d'une solution du standard commercial β-1,4-mannobiose (Carbosynth, Royaume-Uni). Tel que représenté sur la figure 12, le déplacement chimique à 4,76 ppm est caractéristique du β-1,4-mannobiose. Il s'agit de l'atome d'hydrogène lié au carbone 1 de la partie non réductrice du β-1,4-mannobiose (H1 - ManB). Ce pic est également présent dans le spectre du surnageant de culture de la souche *E. coli* PFKA1-pBAD-UhgbMP alors qu'il est absent dans celui de la souche *E. coli* PFKA1. Les résultats obtenus démontrent donc clairement que la souche transformée permet avantageusement de produire des oligosaccharides, notamment le β-1,4-mannobiose. En outre, cet exemple démontre clairement que la souche permet la production d'oligosaccharides qui sont excrétés dans le milieu de culture.

Les résultats obtenus démontrent donc clairement que la souche selon l'invention permet avantageusement la production d'oligosaccharides. En outre, les résultats obtenus démontrent clairement que la souche selon l'invention permet avantageusement et de manière surprenante et inattendue une excrétion des oligosaccharides produits dans le milieu de culture.

En outre, les résultats démontrent clairement que la souche selon l'invention permet la synthèse d'oligosaccharides et avantageusement leur excrétion dans le milieu de culture. Ainsi la production, la récupération et l'isolement des oligosaccharides ne nécessitent aucune altération ou destruction de la souche, permettant avantageusement une production en continu.

### 3.2. Confirmation de la production du β-1,4-mannobiose par HPAEC-PAD

Les échantillons ont été analysés par HPAEC-PAD en appliquant le même protocole que celui décrit précédemment (voir section 3). La figure 13 présente les chromatogrammes des différents échantillons analysés par cette technique. En particulier, sur cette figure, la courbe en trait continu fin correspond au chromatogramme du surnageant de culture de la souche PFKA1 transformée avec pBAD-Teth-1788, la courbe en trait continu gras correspond au chromatogramme du surnageant de culture de la souche PFKA1 non transformée, la courbe en pointillé correspond à un standard commercial de β-1,4-mannobiose (Carbosynth).

Tel que démontré sur la figure 13, la souche selon l'invention permet avantageusement la production d'oligosaccharides. En outre, les résultats obtenus démontrent que les oligosaccharides produits sont excrétés dans le milieu permettant avantageusement une récupération facilitée de l'oligosaccharide produit.

Tel que représenté sur la figure 13, le β-1,4-mannobiose est présent uniquement dans le surnageant de la culture de la souche PFKA1 transformée avec pBAD-UhgbMP et confirment donc la production du β-1,4-mannobiose par la souche PFKA1-pBAD-UhgbMP.

En outre, les résultats obtenus démontrent que les oligosaccharides produits sont excrétés dans le milieu permettant avantageusement une conservation de la souche/de la culture et également avantageusement un isolement/récupération facilité de l'oligosaccharide produit.

### 4. Production de β-1,3- mannobiose

### 4.1. Production du β-1,3- mannobiose et identification par RMN

Une culture de la souche *E. coli* PFKA1 transformée avec le plasmide pBAD-β-1,3-mannooligosaccharide phosphorylase a été réalisée en erlenmeyer selon le protocole décrit dans l'exemple 1. Le surnageant de la culture avec la souche PFKA1-pBAD-β-1,3-mannooligosaccharide phosphorylase a été analysé par RMN en début (3 heures) et en fin de culture (4 jours) en appliquant les mêmes paramètres que ceux décrits précédemment. La figure 18 présente la zone spectrale caractéristique du β-1,3- mannobiose d'un surnageant de la culture de la souche *E. coli* PFKA1-pBAD-β-1,3-mannooligosaccharide phosphorylase après 3 ou 108 heures de culture et d'une solution du standard commercial β-1,3- mannobiose (Carbosynth, Royaume-Uni). Tel que représenté sur la figure 18, le déplacement chimique à 5,22 ppm est caractéristique du β-1,3-mannobiose. Il s'agit de l'atome d'hydrogène lié au carbone 1 de la partie réductrice du β-1,3-mannobiose (H1 - ManA). Ce pic est également présent dans le spectre du surnageant de culture de la souche *E. coli* PFKA1_pBAD-β-1,3-mannooligosaccharide phosphorylase en fin de culture alors qu'il est absent dans celui de la même souche en début de culture. Les résultats obtenus démontrent donc clairement que la souche transformée permet avantageusement de produire des oligosaccharides, notamment le β-1,3-mannobiose. En outre, cet exemple démontre clairement que la souche permet la production d'oligosaccharides qui sont excrétés dans le milieu de culture.

Les résultats obtenus démontrent donc clairement que la souche selon l'invention permet avantageusement la production d'oligosaccharides. En outre, les résultats obtenus démontrent clairement que la souche selon l'invention permet avantageusement et de manière surprenante et inattendue une excrétion des oligosaccharides produits dans le milieu de culture.

En outre, les résultats démontrent clairement que la souche selon l'invention permet la synthèse d'oligosaccharides et avantageusement leur excrétion dans le milieu de culture.

### 4.2. Confirmation de la production du β-1,3- mannobiose par HPAEC-PAD

Les échantillons ont été analysés par HPAEC-PAD en appliquant le même protocole que celui décrit précédemment (voir section 3). La figure 19 présente les chromatogrammes des différents échantillons analysés par cette technique. En particulier, sur cette figure, la courbe en trait continu gras correspond au chromatogramme du surnageant de culture de la souche PFKA1 transformée avec pBAD-β-1,3-mannooligosaccharide phosphorylase après 108 heures de culture, la courbe en pointillé correspond à un standard de β-1,3-mannobiose (Carbosynth), la courbe en trait continu fin correspond à un standard de D-mannose (Carbosynth).

Tel que démontré sur la figure 19, la souche selon l'invention permet avantageusement la production d'oligosaccharides. En outre, les résultats obtenus démontrent que les oligosaccharides produits sont excrétés dans le milieu permettant une récupération facilitée de l'oligosaccharide produit. Tel que représenté sur la figure 19, le β-1,3-mannobiose est présent dans le surnageant de la culture de la souche PFKA1 transformée avec pBAD-β-1,3-mannooligosaccharide phosphorylase et confirment donc la production du β-1,3-mannobiose par la souche PFKA1-pBAD-β-1,3-mannooligosaccharide phosphorylase.

Tel que démontré sur la figure 19, la souche selon l'invention permet avantageusement la production d'oligosaccharides. En outre, le procédé permet avantageusement une synthèse et/ou production d'oligosaccharides à un coût très inférieur par rapport aux procédés enzymatiques *in vitro, n'utilisant pas de support biologique vivant.* De plus, les résultats obtenus démontrent que les oligosaccharides produits sont excrétés dans le milieu permettant avantageusement une récupération facilitée de l'oligosaccharide produit.

### 5. Production de laminaribiose (β-1,3-glucobiose)

### 5.1. Production du laminaribiose et identification par RMN

La souche *E. coli* PFKA1 transformée avec le plasmide pBAD-His-laminaribiose phosphorylase (pBAD-ACL0729) suivant le protocole classique de transformation de cellules chimio-compétentes (https://www.addgene.org/protocols/bacterial-transformation/ [50]) et la souche PFKA1 non transformée ont été mise en culture en erlenmeyer selon le protocole décrit dans l'exemple 2 paragraphe 1.4.

La figure 22 représente les profils de croissance des deux cultures. Tel que représentés sur cette figure les profils de croissance sont similaires pour les souches *E. coli* PFKA1 et PFKA1-pBAD-ACL0729 et atteignent un plateau après 40 heures de cultures.

Les surnageants des deux cultures, avec la souche PFKA1-pBAD-ACL0729 et pFKA1 non transformée ont été analysés par RMN après 49 heures de culture et comparés en appliquant les mêmes paramètres que ceux décrits précédemment. La figure 23 présente une région spectrale entre 4,76 et 4,67 ppm où des signaux sont présents uniquement dans le surnageant de la culture de la souche *E. coli* PFKA1-pBAD- ACL0729 et du laminaribiose commercial (Carbosynth, Royaume-Uni) mais pas dans le surnageant de la culture avec PFKA1 non transformée. Un ajout de laminaribiose commercial dans le surnageant de la culture de la souche *E. coli* PFKA1-pBAD-ACL0729 permet de constater une augmentation de ces mêmes signaux, confirmant ainsi la présence de laminaribiose dans le surnageant de la culture de la souche *E. coli* PFKA1-pBAD- ACL0729. Les résultats obtenus démontrent donc clairement que la souche transformée permet avantageusement de produire des oligosaccharides, notamment le laminaribiose. En outre, cet exemple démontre clairement que la souche permet la production d'oligosaccharides qui sont excrétés dans le milieu de culture.

Les résultats obtenus démontrent donc clairement que la souche selon l'invention permet avantageusement la production d'oligosaccharides. En outre, les résultats obtenus démontrent clairement que la souche selon l'invention permet avantageusement et de manière surprenante et inattendue une excrétion des oligosaccharides produits dans le milieu de culture.

En outre, les résultats démontrent clairement que la souche selon l'invention permet la synthèse d'oligosaccharides et avantageusement leur excrétion dans le milieu de culture.

### 5.2. Confirmation de la production du laminaribiose par HPAEC-PAD

Les échantillons ont été analysés par HPAEC-PAD en appliquant le même protocole que celui décrit ci-dessus (exemple 2 paragraphe 3). La figure 24 représente les chromatogrammes des différents échantillons analysés par cette technique. En particulier, sur cette figure, la courbe en trait continu correspond au chromatogramme du surnageant de culture de la souche *E*. *coli* PFKA1 transformée avec pBAD-ACL0729 après 49 heures de culture, la courbe en pointillé correspond au chromatogramme du surnageant de culture de la souche *E. coli* PFKA1 non transformée et courbe en pointillé discontinue à un standard commercial de laminaribiose (Carbosynth, Royaume-Uni),

Tel que démontré sur la figure 24, la souche selon l'invention permet avantageusement la production d'oligosaccharides. En outre, les résultats obtenus démontrent que les oligosaccharides produits sont excrétés dans le milieu permettant une récupération facilitée de l'oligosaccharide produit. Tel que représenté sur la figure 24, le laminaribiose est présent uniquement dans le surnageant de la culture de la souche *E. coli* PFKA1 transformée avec pBAD-ACL-0729 et confirment donc la production du laminaribiose par la souche *E. coli* PFKA1-pBAD- ACL-0729.

Le tableau 12 correspond à l'intégration des pics obtenus par HPAEC-PAD sur une gamme standard du laminaribiose commercial (std Laminaribiose) à différentes concentration : 1 mg/L, 5mg/L, 10 mg/L, 50 mg/L ou 100mg/L (gamme étalon de standard commercial de laminaribiose de 1 à 100 mg), sur des échantillons de surnageants des cultures de souche *E. coli* PFKA1 non transformée (PFKA1) à différent temps de culture : 40 heures, 49 heures ou 65 heures et sur des échantillons de surnageants des cultures de souche *E. coli* PFKA1 transformée avec le plasmide pBAD-ACL0729 (PFKA1 pBAD-ACL0729) à différent temps de culture : 40 heures, 49 heures ou 65 heures. Les échantillons de surnageant de culture ont été dilués 20 fois avant la mesure.

**Tableau 12 : Intégration des pics obtenus par HPAEC-PAD et quantité de Laminarobiose correspondant.**

| Nom d'injection | Ret.Time min | Aire nC*min | Quantité (mg.L⁻¹) | Quantité (mM) |
|---|---|---|---|---|
| | ED_1 Laminaribiose | ED_1 Laminaribiose | ED_1 Laminaribiose | ED_1 Laminaribiose |
| PFKA1 pBAD-ACL0729 40h (dil20) | 14,19 | 7,01 | 6,63 | 0,019 |
| PFKA1 pBAD-ACL0729 49h (dil20) | 14,19 | 7,28 | 6,93 | 0,020 |
| PFKA1 pBAD-ACL0729 65h (dil20) | 14,20 | 6,92 | 6,52 | 0,019 |
| std Laminaribiose 100 mg/l | 14,19 | 88,09 | 98,66 | 0,288 |
| std Laminaribiose 50 mg/l | 14,20 | 47,61 | 52,70 | 0,154 |
| std Laminaribiose 10 mg/l | 14,20 | 10,27 | 10,32 | 0,030 |
| std Laminaribiose 5 mg/l | 14,18 | 5,08 | 4,44 | 0,013 |
| std Laminaribiose 1 mg/l | 14,18 | 1,06 | n.d. | n.d. |
| PFKA1_40h (dil20) | n.d. | n.d. | n.d. | n.d. |
| PFKA1_49h (dil20) | 14,19 | 0,10 | n.d. | n.d. |
| PFKA1_65h (dil20) | 14,21 | 0,04 | n.d. | n.d. |

Dans le tableau 12 ci-dessus « Ret. Time » signifie temps de rétention et « n.d. » non déterminé.

Tel que démontré dans le tableau 12, après 49 heures de culture, le surnageant de culture de la souche *E. coli* PFKA1-pBAD-ACL-0729 présente un titre de 139 mg.L⁻¹ de laminaribiose.

Tel que démontré sur la figure 24 et dans le tableau 12, la souche selon l'invention permet avantageusement la production d'oligosaccharides. En outre, le procédé permet avantageusement une synthèse et/ou production d'oligosaccharides à un coût très inférieur par rapport aux procédés enzymatiques *in vitro,* n'utilisant pas de support biologique vivant. De plus, les résultats obtenus démontrent que les oligosaccharides produits sont excrétés dans le milieu permettant avantageusement une récupération facilitée de l'oligosaccharide produit.

### Exemple 3 : fabrication et caractérisation de la souche déposée à la CNCM sous le numéro CNCM I-5681

A partir de la souche décrite dans l'exemple 1, les modifications suivantes ont été apportées pour obtenir des rendements encore améliorés pour la conversion du mannose en mannobiose.

Dans l'exemple ci-dessous, la souche déposée à la CNCM (Collection Nationale de Culture de Microorganismes, Institut Pasteur, 25 rue du Docteur Roux, 75724 Paris Cedex 15, France), sous le numéro CNCM I-5681 est également désignée souche CS1 ou CS0Δ*maa*Δ*manA* ou OLI-CS1.

### 1. Préparation et production de la souche CS1 déposée à la CNCM sous le numéro CNCM 1-5681 et validation phénotypique

### 1.1 Suppression du gène maa :

La production de mannobiose avec la souche *E. coli* PFKA1 également désignée CS0, cultivée en milieu minimal s'accompagne de la production d'un composé, détecté par RMN. Ce composé correspondrait à un sucre acétylé, formé dans le cytosol cellulaire par la maltose acétyltransférase, codée par le gène *maa* (Leila Lo Leggio, Florence Dal Degan, Peter Poulsen, Søren Moller Andersen, and Sine Larsen. The Structure and Specificity of Escherichia coli Maltose Acetyltransferase Give New Insight into the LacA Family of Acyltransferases. Biochemistry 2003, 42, 18, 5225-5235 ; DOI : 10.1021/bi0271446 [63]). Cette enzyme est connue pour être capable d'ajouter un groupement acétyle à une large gamme de sucres. Cette réaction est rendue possible par la physiologie particulière de la souche PFKA1 (CS0) puisque l'inactivation de gènes codants pour des éléments des systèmes PTS, conduit à l'internalisation des monosaccharides sans modification chimique. Afin d'éliminer ce contaminant, le gène *maa* a été inactivé selon le protocole décrit dans Datsenko et Wanner (Kirill A. Datsenko, Barry L. Wanner. One-step inactivation of chromosomal genes in Escherichia coli K-12 using PCR products. Proceedings of the National Academy of Sciences Jun 2000, 97 (12) 6640-6645; DOI: 10.1073/pnas.120163297 [62]). Un fragment d'ADN contenant le gène de résistance à la kanamycine et flanquée par des sites Flippase Recognition Target (FRT) a été amplifiée en ajoutant aux extrémités des deux amorces, 50 pb correspondant aux extrémités 5 'et 3' du gène *maa.* Les amorces utilisées pour l'amplification par PCR étaient les suivantes :
FW: 
5'ATGAGCACAGAAAAAGAAAAGATGATTGCTGGTGAGTTGTATCGCTC
GGCGTGTAGGCTGGAGCTGCTTC 3' (SEQ ID NO : 39)
et RV:

5'TTACAATTTTTTAATTATTCTGGCTGGATTACCGCCCACGACAACGTT
GTCATATGAATATCCTCCTTAG 3' (SEQ ID NO 40)

L'ADN polymérase NEB phusion a été utilisée pour amplifier la cassette en utilisant 1 ng de plasmide comme matrice, selon les instructions du fabricant. L'amplification par PCR a été réalisée de la manière suivante: 1 cycle de 1 min à 98 °C, 5 cycles de 30 sec à 98 °C, 30 sec à 55 °C et 1 min à 72 °C, 30 cycles de 30 sec à 98 °C, 1 min à 72 °C et 1 cycle de 5 min à 72 °C. Le fragment ainsi amplifié par PCR a été purifié à partir d'un gel puis quantifié avec un spectrophotomètre Nanodrop.

La souche *E. Coli* PFKA1 (CS0) a été transformée chimiquement avec le plasmide pKD46 à 30°C sur des géloses LB supplémentées avec de l'ampicilline à 50 µg/ml. Le plasmide pKD46 possède des gènes de phage lambda, β et exo sous le contrôle du promoteur d'arabinose. Les transformants portant le plasmide pKD46 ont été cultivés dans 5 mL de milieu LB contenant 50 µg/mL d'ampicilline et de L-arabinose (0,2% p/v) à 30 °C jusqu'à une DO₆₀₀ de 0,6, puis concentrés 100 fois et en lavés trois fois avec du glycérol froid à 10%. L'électroporation des cellules ainsi obtenues, a été réalisée en utilisant un Cell-Porator avec un amplificateur de tension et des chambres de 0,1 cm selon les instructions du fabricant, en utilisant 50 µL de cellules et 200 ng des fragments PCR obtenus ci-avant. Un millilitre de milieu SOC (2% de tryptone ; 0,5% d'extrait de levure ; 10 mM de NaCl, 2,5 mM de KCl, 10 mM de MgCl₂, 10 mM de MgSO₄, et 20 mM glucose) a été ajouté aux cellules ayant subies ce traitement et, incubées 2 heures à 37 °C, puis étalées sur une gélose LB supplémentée avec 50 µg/mL de kanamycine pour sélectionner les transformants Km. L'élimination du gène *maa* a été confirmée par PCR sur colonie tel que décrit ci-dessous en utilisant les amorces suivantes:
FW: 5'GATGATTGCTGGTGAGTTGTATCG 3' (SEQ ID NO : 41)
et RV: 5' TTAATTATTCTGGCTGGATTACCG 3' (SEQ ID NO : 42)

L'ADN polymérase NEB taq a été utilisée pour amplifier un fragment du gène *maa* en utilisant 1 µL d'une colonie de cellules dissoute dans 50 µL d'eau stérile selon les instructions du fabricant. L'amplification par PCR a été réalisée de la manière suivante: 1 cycle de 5 min à 95 °C, 35 cycles de 30 sec à 95 °C, 30 sec à 55 °C et 1,5 min à 68 °C, et 1 cycle de 5 min à 68 °C. Une fois les colonies ayant perdu le gène identifiées, l'élimination de la cassette de résistance à la kanamycine a été réalisée en transformant, l'une des colonies positives avec le plasmide pCP20. Le plasmide pCP20 plasmide est un plasmide de résistance à l'ampicilline avec une réplication sensible à la température et une induction de la synthèse FLP par choc thermique. Les mutants kanamycine-résistants (KmR) ont été transformés avec le plasmide pCP20, et les transformants résistants à l'ampicilline ont été sélectionnés à 30 °C. Des transformants ont été isolés sur gélose LB de manière non sélective à 43 °C et ensuite testés pour la perte de toutes les résistances aux antibiotiques.

La souche PFKA1, également désignée CSO dans laquelle le gène maa a été inactivé est désignée par CS0Δ*maa*.

### 1.2 Validation phénotypique de la souche

Les souches de *E. coli* PFKA1, également désignées PFKA1, *E. coli* CS0 ou CSO, et CS0Δ*maa* portant le plasmide pBAD-Teth1788 ont été cultivées en milieu minéral M9 complémenté avec du mannose à la concentration de 6 g/L. Les cultures ont été réalisées en double ou en triple dans des erlenmeyers bafflés de 250 mL contenant 50mL de milieu de culture, à 37 °C et sous agitation orbitale de 200 tr/min. La croissance a été suivi par mesure de la turbidimétrie à 600 nm. De la kanamycine et de l'ampicilline ont été ajoutées à une concentration finale de 50 µg/ml, pour assurer le maintien des plasmides dans les cellules. De l'IPTG (concentration finale de 60 µM) et de l'arabinose (concentration finale de 10 mM) ont été ajoutés dans le milieu pour induire l'expression des protéines. Les métabolites extra-cellulaires ont été identifiés et quantifiés par RMN. Des échantillons de la culture ont été collectés à différents temps des cultures et centrifugés 2 min à 18 000g. 500 µL de ces surnageants ont été mélangés à 100 µL de D₂O contenant 2,35 g/L d'acide 3-(triméthylsilyl)-1-propanesulfonique tétra-deutéré (TSPd4) molécule utilisée comme standard interne pour l'analyse par RMN. Les spectres ¹H-RMN ont été acquis avec un spectroscope RMN Avance 500 MHz équipé d'une sonde BBI 5 mm (Bruker, Rheinstatten, Allemagne). Le traitement des spectres et la quantification des métabolites ont été réalisés avec le logiciel Topspin 3.1 (Bruker, Rheinstatten, Allemagne). Les résultats obtenus sont représentés sur la figure 25

Tel que représenté sur la figure 25, les spectres RMN obtenus à partir d'échantillons de milieu de culture prélevé démontrent clairement la présence du produit contaminant, à savoir un sucre acétylé, uniquement dans l'échantillon issu du milieu de culture de la souche CS0 (PFKA1). En outre, le spectre RMN obtenu pour la souche CS0Δ*maa* ne présente pas ce produit contaminant, sucre acétylé, et valide la construction génétique de la souche CS0Δ*maa*.

Cet exemple démontre clairement que la souche CS0Δ*maa* permet avantageusement la production oligosaccharides, par exemple de mannobiose, sans production de sucre acétylé.

### 1.3. Suppression du gène manA

Les inventeurs ont démontré de manière surprenante que la suppression du gène *manA* permet avantageusement de maximiser la conversion du mannose en mannobiose. La figure 26 présente la stratégie générale de production de mannobiose dans cet exemple. Il s'agit d'un schéma métabolique de la souche pour la production de mannobiose à partir de glycérol et de mannose. Lorsque le mannose est la seule source de carbone, il est également la source d'énergie et le substrat pour la synthèse du mannobiose. Par conséquent, le rendement de conversion maximal du mannose en mannobiose est fortement limité. Pour lever cette limitation, l'utilisation du mannose à des fins de croissance peut être fortement réduite en supprimant le gène *manA,* qui catalyse la conversion du mannose-6-phosphate en fructose-6-phosphate, intermédiaire du métabolisme central et en ajoutant une autre source de carbone et d'énergie, moins onéreuse que le mannose et n'interagissant pas avec la production de mannobiose. Le glycérol a été donc choisi et ajouté comme source de carbone et d'énergie.

La suppression du gène *manA* a été réalisée selon le protocole Datsenko et Wanner (Kirill A. Datsenko, Barry L. Wanner. One-step inactivation of chromosomal genes in Escherichia coli K-12 using PCR products. Proceedings of the National Academy of Sciences Jun 2000, 97 (12) 6640-6645; DOI: 10.1073/pnas.120163297 [62]). Un fragment d'ADN contenant le gène de résistance à la kanamycine et flanquée par des sites « Flippase Recognition Target » (FRT) a été amplifiée en ajoutant aux extrémités des deux amorces, 50 pb correspondant aux extrémités 5 'et 3' du gène *manA.* Les amorces utilisées pour l'amplification par PCR étaient les suivantes :
FW: 
5’ATTATGCGCAGCACAGCCACTCTCCATTCAGGTTCATCCAAACAAAC
ACAGTGTAGGCTGGAGCTGCTTC 3’ (SEQ ID NO : 43)
et RV:

5’ACACGCGCTAAACGGCCGTGGCCTTTGACAGTCACCGGTGATTCGT
TGGCCATATGAATATCCTCCTTAG 3’ (SEQ ID NO : 44)

L'ADN polymérase NEB phusion a été utilisée pour amplifier la cassette en utilisant 1 ng de plasmide pKD4 comme matrice, selon les instructions du fabricant. L'amplification par PCR a été réalisée de la manière suivante: 1 cycle de 1 min à 98 °C, 5 cycles de 30 sec à 98 °C, 30 sec à 55 °C et 1 min à 72 °C, 30 cycles de 30 sec à 98 °C, 1 min à 72 °C et 1 cycle de 5 min à 72 °C. Le fragment ainsi amplifié par PCR a été purifié à partir d'un gel puis quantifié avec un spectrophotomètre Nanodrop.

La souche CS0Δ*maa* a été transformée avec le plasmide pKD46 à 30 °C sur des géloses LB supplémentées avec de l'ampicilline à 50 µg/ml. Les transformants portant le plasmide pKD46 ont été cultivés dans 5 mL de milieu LB contenant 50 µg/mL d'ampicilline et de L-arabinose (0,2% p/v), à 30 °C jusqu'à une DO₆₀₀ de 0,6, puis concentrés 100 fois et lavés trois fois avec du glycérol froid à 10%. L'électroporation des cellules ainsi obtenues, a été réalisée en utilisant un Cell-Porator avec un amplificateur de tension et des chambres de 0,1 cm selon les instructions du fabricant, en utilisant 50 µL de cellules et 200 ng des fragments PCR obtenus ci-avant. 1 ml de milieu SOC (voir composition ci-dessus) a été ajouté aux cellules ayant subies ce traitement et, incubées 2 h à 37 °C, puis étalées sur une gélose LB supplémentée avec 50 µg/mL de kanamycine pour sélectionner les transformants Km. L'élimination du gène *manA* a été confirmée par PCR sur colonie (selon le procédé ci-dessous) en utilisant les amorces suivantes: FW: 5' GTGCGGGTCTGACGCCTAAATAC 3' (SEQ ID NO : 45) et RV: 5' GATGGGTTTCAATCTTCTTGCCT 3'(SEQ ID NO : 46).

L'ADN polymérase NEB taq a été utilisée pour amplifier un fragment du gène *manA* en utilisant 1 µL d'une colonie de cellules dissoute dans 50 µL d'eau stérile, selon les instructions du fabricant. L'amplification par PCR a été réalisée de la manière suivante: 1 cycle de 5 min à 95 °C, 35 cycles de 30 sec à 95 °C, 30 sec à 55 °C et 1,5 min à 68 °C, et 1 cycle de 5 min à 68 °C. Une fois les colonies ayant perdu le gène identifiées, l'élimination de la cassette de résistance à la kanamycine a été réalisée en transformant l'une des colonies positives avec le plasmide pCP20. Les mutants kanamycine-résistants (KmR) ont été transformés avec le plasmide pCP20, et les transformants résistants à l'ampicilline ont été sélectionnés à 30 °C. Des transformants ont été isolés sur gélose LB de manière non sélective à 43 °C et ensuite testés pour la perte de toutes les résistances aux antibiotiques. La souche ainsi obtenue correspond à la souche PFKA1, également désignée CSO, dans laquelle le gène maa et le gène manA ont été en outre inactivés.

La souche PFKA1, également désignée CSO dans laquelle le gène maa et le gène *manA* ont été inactivés est désignée CS0Δ*maa*Δ*manA* ou CS1.

### 1.4. Construction du plasmide pTC-galP

L'induction de *galP* par l'IPTG dans un plasmide à copie élevée (pWKS) entraîne un coût énergétique supplémentaire significatif pour la bactérie, pouvant affecter le fonctionnement cellulaire. Par conséquent, le gène *galP* a été introduit dans un plasmide à copie moyenne sous le contrôle d'un promoteur constitutif fort. Dans ce but, le plasmide pTC-galP a été construit, en utilisant le promoteur constitutif d'*E*. *coli* pIHF et l'origine de réplication pA15. Le clonage du plasmide a été réalisé par recombinaison homologue en amplifiant 3 fragments d'ADN différents avec des extrémités homologues pour assembler les différents éléments du plasmide.

Les fragments d'ADN ont été amplifiés comme suit :
a) Origine de réplication et *kanR* en utilisant le plasmide pZA23 comme matrice avec les amorces suivantes:
   FW:5' AAATAGGCGCTCACGATTAAAAGGAAGCTGAGTTGGCTGC 3' (SEQ ID NO : 47)
   RV: 5' TAGCTTTGCACTGTTTCAGAGTGAAGACGAAAGGGCCTCG 3' (SEQ ID NO 48)
   L'ADN polymérase NEB phusion a été utilisée pour amplifier la cassette en utilisant 1 ng de plasmide pKD4 comme matrice, selon les instructions du fabricant. L'amplification par PCR a été réalisée de la manière suivante: 1 cycle de 1 min à 98 °C, 5 cycles de 30 sec à 98 °C, 30 sec à 60 °C et 1 min à 72 °C, 30 cycles de 30 sec à 98 °C, 1 min à 72 °C et 1 cycle de 5 min à 72 °C. Le fragment ainsi amplifié par PCR a été purifié à partir d'un gel puis quantifié avec un spectrophotomètre Nanodrop.
b) Promoteur IHF du plasmide pBS1C3-IHF avec les amorces suivantes:
   FW:5' CGAGGCCCTTTCGTCTTCACTCTGAAACAGTGCAAAGCTA 3' (SEQ ID NO : 49)
   RV: 5' TGTTTTTTAGCGTCAGGCATCTCTAGGATTCCTCCGGTTC 3' (SEQ ID NO : 50)
   L'ADN polymérase NEB phusion a été utilisée pour amplifier la cassette en utilisant 1 ng de plasmide pBS1C3-IHF comme matrice, selon les instructions du fabricant. L'amplification par PCR a été réalisée de la manière suivante: 1 cycle de 1 min à 98 °C, 5 cycles de 30 sec à 98 °C, 30 sec à 60 °C et 1 min à 72 °C, 30 cycles de 30 sec à 98 °C, 1 min à 72 °C et 1 cycle de 5 min à 72 °C. Le fragment ainsi amplifié par PCR a été purifié à partir d'un gel puis quantifié avec un spectrophotomètre Nanodrop.
c)Transporteur GalP en utilisant le plasmide pWKS-galP comme matrice avec les amorces suivantes :
   FW:5' GAACCGGAGGAATCCTAGAGATGCCTGACGCTAAAAAACA 3' (SEQ ID NO 51)
   RV: 5' GCAGCCAACTCAGCTTCCTTTTAATCGTGAGCGCCTATTT 3' (SEQ ID NO 52)

L'ADN polymérase NEB phusion a été utilisée pour amplifier la cassette en utilisant 1 ng de plasmide pWKS-galP comme matrice, selon les instructions du fabricant. L'amplification par PCR a été réalisée de la manière suivante: 1 cycle de 1 min à 98 °C, 5 cycles de 30 sec à 98 °C, 30 sec à 62 °C et 1 min à 72 °C, 30 cycles de 30 sec à 98 °C, 1 min à 72 °C et 1 cycle de 5 min à 72 °C. Le fragment ainsi amplifié par PCR a été purifié à partir d'un gel puis quantifié avec un spectrophotomètre Nanodrop.

Les trois fragments purifiés ont été incubés ensemble et le clonage a été réalisé en utilisant le kit de clonage inFusion^{®} Takara selon les instructions du fabricant, qui a permis la réalisation de la réaction de ligation et la transformation de cellules *E. coli* Top10 chimiquement compétentes. La sélection a été effectuée sur des géloses de LB supplémentées avec de 50 µg/mL de kanamycine.

L'assemblage correct du plasmide a été confirmé par PCR sur colonie en utilisant les amorces suivantes :
FW: 5' TCTGAAACAGTGCAAAGCTA 3' (SEQ ID NO : 53) et
RV: 5' TTAATCGTGAGCGCCTATTT 3' (SEQ ID NO : 54

L'ADN polymérase NEB taq a été utilisée pour amplifier un fragment d'ADN entre la séquence du promoteur IHF et celle du gène *galP* en utilisant 1 µL d'une colonie de cellules dissoute dans 50 µL d'eau stérile, selon les instructions du fabricant. L'amplification par PCR a été réalisée de la manière suivante:
1 cycle de 5 min à 95 °C, 35 cycles de 30 sec à 95 °C, 30 sec à 55 °C et 1,5 min à 68 °C, et 1 cycle de 5 min à 68 °C.

Les colonies positives ont été cultivées dans 5 ml de milieu liquide LB supplémenté avec 50 µg/mL de kanamycine pendant une nuit à 37 °C. Ensuite, des « miniprep » du plasmide pTC-galP ont été réalisés avec le kit de préparation Qiagen pour récupérer et concentrer le plasmide pTC-galP.

### 1.5. Production de mannobiose sur un mélange de glycérol et de mannose.

a) La souche obtenue CS1 (CS0Δ*maa*Δ*manA*) a ensuite été transformée avec les couples de plasmides pWKS-galP/pBAD-Teth88 (A1) ou pTC-galP/pBAD-Teth88 (C1). La croissance a été réalisée dans des fioles erlenmeyers bafflés de 250 mL contenant 50mL de milieu M9, à 37 °C et sous agitation orbitale de 200 tr/min. Le milieu M9 a été supplémenté avec 6 g/L de glycérol et 1 g/L de mannose, 0,06 mM d'IPTG et 10 mM de L-arabinose. L'analyse du surnageant du milieu de culture a été réalisée par RMN comme décrit ci-dessus.

Les souches ont été cultivés après culture un échantillon de surnageant a été analysé avec de déterminer la concentration de β-1,2-mannobiose, selon le procédé décrit dans l'exemple 2 ci-dessus, présente en fonction des plasmides pWKS-galP/pBAD-Teth88 (A1) ou pTC-galP/pBAD-Teth88 (C1), présent dans la souche CS1. La concentration de β-1,2-mannobiose mesurée pour la souche CS1 transformée avec les plasmides pWKS-galP/pBAD-Teth88 (A1) était de 0,78 mM dans le surnageant du milieu de culture et de 0,91 mM dans le surnageant du milieu de culture pour la souche CS1 transformée avec les plasmides pTC-galP/pBAD-Teth88 (C1). Une détermination du taux de conversion de mannose en β-1,2-mannobiose a été également estimée et correspondait à 48% et à 60% respectivement. Cet exemple démontre donc clairement que la souche CS1, également dénommé CS0Δ*maa*Δ*manA,* correspondant à la souche PFKA1 dans laquelle le gène maa et le gène *manA* ont été inactivés permet avantageusement une production d'oligosaccharides. En outre, les résultats obtenus démontrent clairement qu'un exemple de souche selon l'invention permet avantageusement et de manière surprenante et inattendue une excrétion des oligosaccharides produits dans le milieu de culture.

Cet exemple démontre également qu'un exemple de souche selon l'invention permet avantageusement une production d'oligosaccharides avec des rendements de production élevé.

En d'autres termes, les résultats démontrent clairement qu'un exemple de souche selon l'invention permet avantageusement la synthèse d'oligosaccharides et avantageusement leurs excrétions dans le milieu de culture.

### b) Production de β-1,2-mannobiose dans un bioreacteur

Une production en bioréacteur de β-1,2-mannobiose par la souche CS1 avec le couple de plasmides C1 a été réalisé. Pour cette expérience en bioréacteur, la composition des sels M9 a été modifiée comme suit: KH2PO4 3,02 g/L, NaCl 0,51 g/L, NH4Cl 2,04 g/L, (NH₄)2SO₄ 5 g/L. Tous les autres composants pour la culture sont identiques à ceux décrit pour la culture ci-dessus. Les cultures en bioréacteur ont été réalisées dans 500 ml de ce milieu M9 complémenté avec 3 g/L de D-mannose et 20 g/L de glycérol. L'analyse du surnageant a été réalisée par RMN comme décrit dans l'exemple 2. En fin de culture un échantillon de surnageant a été analysé avec de déterminer la concentration de β-1,2-mannobiose, selon le procédé décrit dans l'exemple 2 ci-dessus. La concentration en β-1,2-mannobiose déterminé était de 1,21 mM et le taux de conversion de 58%, similaire aux valeurs obtenues lors de la culture précitée en fioles Erlenmeyer.

Une comparaison de la production de β-1,2-mannobiose en bioréacteur a été également réalisée avec les souches MDO, MGX, MGX1, PFKA1, CS0 Δmaa, exprimant l'enzyme Teth-1788

Le tableau 13 regroupe les caractéristiques de production calculées pour les différentes souches à partir des concentrations en β-1,2-mannobiose et en mannose résiduelle déterminées par RMN.

**Tableau 13 : Titres et rendements en β-1,2-mannobiose dans les surnageants de cultures en bioréacteur des souches MDO, MGX, MGX1, PFKA1, CS0 Δmaa, et CS1 (Δmaa ΔmanA) exprimant l'enzyme Teth-1788. Les concentrations (titre) sont exprimées en mM et les rendements en pourcentage de mannose converti en β-1,2-mannobiose.**

| **Souche** | **Caractéristique** | **Titre Man2 (mM)** | **% Rendement (g Man2 / g Man)** |
|---|---|---|---|
| **MDO-Teth-1788** | Souche contrôle | ND¹ | ND |
| **MGX-Teth-1788** | PTS-, pas de GalP ; man B non induit | 0,21¹ | 1,02 |
| **MGX1-Teth-1788** | PTS-, avec GalP induit, manB induit | 0,59¹ | 2,91 |
| **PFKA1-Teth-1788 (CS0)** | PTS- avec GalP ; délétion de pfka ; manB induit | 1,8¹ | 9,02 |
| **CS0 Δmaa-Teth-1788** | PTS- avec GalP ; délétion de pfka ; manB induit. Délétion de Maa | 1,75¹ | 8,98 |
| **CS0 maa-Teth-1788** | PTS- avec GalP ; délétion de pfka ; manB induit. Délétion de maa; croissance glycérol | 0,58² | 18,22 |
| **CS1 Δmaa ΔmanA -Teth-1788** | PTS- avec GalP ; délétion de pfka ; manB induit. Délétion de maa, délétion de manA ; croissance glycérol | 1,21¹ | 58-72 |

Dans le tableau ¹ signifie croissance avec 10 g/L de mannose comme source de carbone et d'énergie, et ² signifie croissance avec 3 g/L de mannose (conversion en β-1,2-mannobiose) et 20 g/L de glycérol (source de carbone et d'énergie).

Tel que démontré ci-dessus, la souche selon l'invention permet avantageusement et de manière surprenante d'augmenter significativement la production d'oligosaccharides. En outre les résultats obtenus démontrent clairement que la souche selon l'invention permet d'augmenter significativement les rendements de production des oligosaccharides et ainsi d'optimiser/réduire les coûts de production.

Cet exemple démontre donc clairement que la souche qu'un exemple de souche selon l'invention permet avantageusement une production d'oligosaccharides. En outre, les résultats obtenus démontrent clairement qu'un exemple de souche selon l'invention permet avantageusement et de manière surprenante et inattendue une excrétion des oligosaccharides produits dans le milieu de culture.

Cet exemple démontre également qu'un exemple de souche selon l'invention permet avantageusement une production d'oligosaccharides avec des rendements de production élevé.

En d'autres termes, les résultats démontrent clairement que des exemples de souches selon l'invention permettent avantageusement la synthèse d'oligosaccharides et avantageusement leurs excrétions dans le milieu de culture.

### Exemple 4 : fabrication et caractérisation de la souche déposée à la CNCM sous le numéro CNCM 1-5682

A partir de la souche décrite dans l'exemple 3, les modifications suivantes ont été apportées pour obtenir des rendements encore améliorés pour la conversion du mannose en mannobiose.

Dans l'exemple ci-dessous, la souche déposée à la CNCM (Collection Nationale de Culture de Microorganismes, Institut Pasteur, 25 rue du Docteur Roux, 75724 Paris Cedex 15, France), sous le numéro CNCM I-5682 est également désignée souche CS1 IG ou OLI-CS1 IG ou CS1 IG-galP.

Le gène *GalP* est faiblement exprimé du fait de deux répresseurs galR et galS. Les inventeurs ont mis en avant de façon surprenante qu'il est possible d'éviter l'utilisation de plasmides pour l'expression du gène *GalP* et de stabiliser le châssis bactérien en augmentant l'expression du gène *GalP* via la modification de son promoteur. La région en amont du gène *GalP* (de 400 paires de bases), qui est reconnu par les répresseurs, est substituée avec un fragment ADN portant le promoteur IHF d'E. coli (Zhou, K., Zhou, L., Lim, Q. 'En, Zou, R., Stephanopoulos, G., and Too, H.-P. (2011) Novel reference gènes for quantifying transcriptional responses of Escherichia coli to protein overexpression by quantitative PCR. BMC Mol Biol 12: 18 [65])..

Dans la présente, la substitution d'une séquence d'ADN peut être effectuée par tout procédé adapté connu de l'homme du métier. Il peut s'agir par exemple du protocole Datsenko et Wanner (Kirill A. Datsenko, Barry L. Wanner. One-step inactivation of chromosomal genes in Escherichia coli K-12 using PCR products. Proceedings of the National Academy of Sciences Jun 2000, 97 (12) 6640-6645; DOI: 10.1073/pnas.120163297 [62]).

### 1. Construction du fragment de remplacement portant le promoteur IHF

La première étape est l'assemblage du fragment destiné à être intégré au génome. L'échantillon comprend le gène de résistance à la kanamycine (1500bp) et le promoteur IHF. Ces deux fragments ont été amplifiés individuellement puis combinés ensemble pour former le fragment de remplacement de 2300bp.

Un fragment d'ADN contenant le gène de résistance à la kanamycine et flanquée par des sites Flippase Recognition Target (FRT) a été amplifiée avec les amorces suivantes :
fw: 5'-GCACAATAACATCATTCTTCCTGATCACGTTTCACCGCAGATTATCATC AAGATTGCAGCATTACACGTCTT 3' (SEQ ID NO : 56)
Et rev: 5'-TGTGGGTTTCCGCGAACTGCTGCCACGGGTTAGCTTTGCACTGTTTC AGATCCATATGAATATCCTCCTTAGTTCCT 3' (SEQ ID NO : 57)

L'amorce fw a une homologie de 50bp avec la région immédiatement en amont de la séquence à déléter. L'amorce rev a une homologie de 20bp avec l'extrémité 5' du promoteur IHF. La polymérase ADN Phusion (NEB) a été utilisée pour amplifier la cassette en utilisant 1ng de plasmide pKD4 comme matrice, suivant le protocole du fournisseur. Les cycles de PCR sont réalisés de la façon suivante :
1 cycle d'1 min à 98 °C, 5 cycles de 30 secondes à 98°C, 30 secondes à 63°C et 1 min à 72°C, 30 cycles de 30 secondes à 98°C, 1 min à 72°C, et 1 cycle de 5 min à 72°C.

Le fragment du promoteur a été amplifié avec les amorces suivantes :
fw: 5'-AGGAACTAAGGAGGATATTCATATGGATCTGAAACAGTGCAAAGCTAA CCCGTGGCAGCAGTTCGCGGAAACCCACA 3' (SEQ ID NO 58)

Et rev: 5'-AAAAACGTCATTGCCTTGTTTGACCGCCCCTGTTTTTTAGCGTCAGGC ATCTCTAGGATTCCTCCGGTTCCT 3'(SEQ ID NO 59)

L'amorce FW (SEQ ID NO 58) à une homologie de 20bp avec l'extrémité 3' de la séquence de la cassette kan. L'amorce rev (SEQ ID NO 59) a une homologie de 50 bp avec la région du génome immédiatement en amont de la séquence à déléter.

La polymérase ADN Phusion (NEB) est utilisée pour amplifier la cassette en utilisant 1ng de plasmide pKD4 comme matrice, suivant le protocole du fournisseur. Les cycles de PCR sont réalisés de la façon suivante :
1 cycle de 1 min à 98 °C, 5 cycles de 30 secondes à 98°C, 30 secondes à 64°C et 30 sec à 72°C, 30 cycles de 30 secondes à 98°C, 30 secondes à 72°C, et 1 cycle de 5 min à 72°C.

Pour générer le fragment final une troisième PCR est réalisé avec les avec les amorces suivantes :
fw: 5'GCACAATAACATCATTCTTCCTGATCACG 3' (SEQ ID NO 60)
et rev: 5' AAAAACGTCATTGCCTTGTTTGACCG 3' (SEQ ID NO 61)

Comme matrice 1 µL de PCR1 et 1 µL de PCR2 sont utilisés. Les cycles de PCR sont réalisés de la façon suivante: 1 cycle de 1 min à 98 °C, 35 cycles de 30 secondes à 98°C, 30 secondes à 65°C et 1 min 30 secondes à 72°C, et 1 cycle de 5 min à 72°C.

L'amplicon de la PCR est purifié sur gel and quantifié. Ce fragment est utilisé pour la substitution du promoteur du gène *GalP.*

### 2. Recombinaison homologue :

La souche CS1 a été transformé avec le plasmide pKD46 à 30°C sur gélose d'agar LB supplémenté avec de l'ampicilline à 50 µg/mL. La souche transformée portant le plasmide pKD46 a été mise en culture dans 50 ml de milieu LB comprenant de l'ampicilline et du L-arabinose (0,2% w/v) à une température de 30°C pour une OD600 de 0,6 puis concentré 100 fois pour la rendre électrocompétentes, lavé trois fois avec de l'eau froide contenant 10% de glycérol. Une électroporation a été réalisée en utilisant un Cell-Porator avec un amplificateur de tension et des chambres de 0,1 cm selon les instructions du fabricant, en utilisant 50 µL de cellules et 200 ng des fragments PCR obtenus ci-avant.

Un millilitre de milieu SOC (2% de tryptone ; 0,5% d'extrait de levure ; 10 mM de NaCl, 2,5 mM de KCl, 10 mM de MgCl₂, 10 mM de MgSO₄, et 20 mM glucose) a été ajouté aux cellules ayant subies ce traitement, incubées 2 heures à 37 °C, puis étalées sur une gélose LB comprenant de la kanamycine pour sélectionner les souches. L'introduction du promoteur IHF en amont du gène *GalP* a été confirmée par PCR sur colonie en utilisant les amorces suivantes:
fw: 5' CAGCCTGTCTGTTCGTGCGAAAGA 3' (SEQ ID NO : 62) et
rev: 5' TCAGAGAGGTACAGCGGTG 3' (SEQ ID NO : 63)

L'ADN polymérase NEB taq a été utilisée pour amplifier un fragment du promoteur IHF et du gène *GalP* en utilisant 1 µL d'une colonie de cellules dissoute dans 50 µL d'eau stérile selon les instructions du fabricant. L'amplification par PCR a été réalisée de la manière suivante: 1 cycle de 5 min à 95 °C, 35 cycles de 30 sec à 95 °C, 30 sec à 55 °C et 1,5 min à 68 °C, et 1 cycle de 5 min à 68 °C. Une fois les colonies ayant perdu le gène identifiées, l'élimination de la cassette de résistance à la kanamycine a été réalisée en transformant, l'une des colonies positives avec le plasmide pCP20. Le plasmide pCP20 est un plasmide de résistance à l'ampicilline avec une réplication sensible à la température et une induction de la synthèse FLP par choc thermique. Les mutants kanamycine-résistants (KmR) ont été transformés avec le plasmide pCP20, et les transformants résistants à l'ampicilline ont été sélectionnés à 30 °C. Des souches transformées ont été isolés sur gélose LB de manière non sélective à 43 °C et ensuite testés pour la perte de toutes les résistances aux antibiotiques.

### 3. Clonage du gène codant pour une enzyme β-1,2-mannobiose phosphorylase dans le plasmide pBAD-HisA (amorces et séquence plasmidique)

Le gène codant pour la protéine Lin0857 de la souche *Listeria innocua* (Lin0857, numéro d'accès Genbank CAC96089) appartenant à la famille GH130 de la classification CAZy (http://www.cazy.org/) (Lombard, V., Golaconda Ramulu, H., Drula, E., Coutinho, P.M., Henrissat, B., 2014. The carbohydrate-active enzymes database (CAZy) in 2013. Nucleic Acids Res. 42, D490-D495 [30]) a été synthétisé et cloné dans le vecteur pET28a par la société TWIST Bioscience (San Francisco, CA 94080, États-Unis) avec une optimisation de l'usage des codons pour une expression du gène dans *E. coli.* Le gène codant pour la Lin0857 et la plasmide pBAD-Hisa ont été purifiés sur gel après une digestion par les enzymes de restriction *Ncol-HF* et *Xhol* suivant le protocole du kit QIAEX II Gel Extraction Kit (Hilden, Allemagne). La ligation du vecteur pBAD et du gène codant pour la Lin0857 (insert) a été réalisée en présence de 10 µL de vecteur à 8 ng/µL, 7 µL d'insert à 7 ng/µL, 2 µL de tampon de la DNA ligase 10 X et 1,5 µL de T4 DNA ligase à 400 000 U/mL (New England Biolabs, Ipswich, Massachusetts, États-Unis). La réaction est incubée 1 heure à température ambiante puis 10 min à 65 °C et 10 min dans la glace et 6 µL de cette réaction sont déposés dans 50 µL de cellules compétentes commerciales Stellar (Clontech Laboratories, Mountain View, USA) pour une transformation suivant le protocole classique de transformation de cellules chimio-compétentes (https://www.addgene.org/protocols/bacterial-transformation/ [50]). La construction a été contrôlée par séquençage auprès de la société Eurofins genomics (Luxembourg) en utilisant les amorces suivantes : pBAD-fw : 5'-ATGCCATAGCATTTTTATCC-3' (SEQ ID NO : 64) et pBAD-rev : 5'-GATTTAATCTGTATCAGG-3' (SEQ ID NO : 65).

La séquence plasmidique obtenue correspond à la séquence SED ID NO : 55 représentée sur la figure 28.

Les souches CS0, CS1 et CS1 IG ont été transformées suivant le protocole classique de transformation de cellules chimio-compétentes (https://www.addgene.org/protocols/bacterial-transformation/ [50]) avec le plasmide pWKS-galP, pBAD-Teth1788 et/ou pBAD-Lin0857 comme indiqué dans le tableau (14) ci-dessous.

**Tableau 14 : Souches et caractéristiques**

| Identifiant | Souche | Caractéristiques |
|---|---|---|
| A | CS0 | MDO ptsG manXYZ pfkA |
| B | CS0-gaIP-Teth 1788 | CS0 pWKS-gaIP; pBAD-Teth1788 |
| C | CS1-gaIP-Teth1788 | CS0 ΔmaaΔmanA; pWKS-galP ; pBAD-Teth 1788 |
| D | CS1 IG-Teth1788 | CS1 galP: Pihf; pBAD-Teth1788 |
| E | CS1-gaIP-Lin0857 | CS0 ΔmaaΔmanA; pWKS-galP ; pBAD-Lin0857 |
| F | CS1 IG-galP-Lin0857 | CS1 galP: Pihf pWKS-galP ; pBAD-Lin0857 |

| | | |
|---|---|---|
| Dans le tableau ci-dessus, « Pihf » signifie promoteur IHF | | |

Ces 6 souches ont été mises en précultures sur la nuit (de 10 à 15 heures) en milieu sélectif LB pour inoculer une seconde préculture (de 15 à 20 heures) en milieu sélectif M9 additionnée de glycérol (10 g/L) et D-Mannose (5 g/L) comme source de carbone. Les 6 cultures de 50 mL ont été inoculées avec les précultures correspondantes pour obtenir une DO₆₀₀ = 0,15 à t=0h en milieu sélectif M9 additionné de glycérol (10 g/L) et D-Mannose à 5 g/L comme source de carbone dans des erlenmeyer bafflés de 500 mL. Les erlenmeyers ont été incubés pendant 47h à 37°C avec agitation orbitale de 220 tours par minutes (rpm). L'ajout de L-arabinose à 0,1% dans le milieu de culture lorsque la DO₆₀₀ est ≈ 0,4 permet l'expression du gène de la glycoside phosphorylase présent dans le plasmide pBAD (Teth1788 ou Lin0857) et l'ajout d'IPTG à 60 µM final permet l'expression du gène GalP dans les cultures contenant le plasmide pWKS-galP.

La figure 27 représente les courbes de croissances des différentes cultures des souches mentionnées dans le tableau 14 ci-dessus. La détermination de la croissance a été effectuée par mesure de la densité optique à 600 nm (ordonnée) (OD₆₀₀) en fonction du temps en heures (abscisse).

Des échantillons de surnageants ont été prélevés après 31 heures de culture dans les 6 cultures et dilué 10 fois dans l'eau ultra pure pour être analysés par HPAEC-PAD suivant le protocole décrit précédemment (voir section 2.3 de l'exemple 2 ) Les titres ou concentration en β-1,2-mannobiose présent dans les surnageants et les rendements de conversion du mannose consommé converti en mannobiose ont été calculés et présentés dans le tableau 15 ci-dessous. Les concentrations (titre) sont exprimées en mM et les rendements en pourcentage de mannose converti en β-1,2-mannobiose. Tableau 15: pourcentage de mannose consommé, rendements et concentration en β-1,2-mannobiose (mannobiose) dans les surnageants après 31 heures de cultures des 6 souches.

| | Souche | Mannose consommé (%) | Mannose converti en mannobiose (%) (g Man2 / g Man) | Concentration en mannobiose (mM) |
|---|---|---|---|---|
| A | CS0 | 68,5 | **0** | **0** |
| B | CS0-gaIP-Teth1788 | 91,6 | **9** | **1,22** |
| C | CS1-galP-Teth1788 | 21,7 | **71** | **2,24** |
| D | CS1 IG-Teth1788 | 14,1 | **49** | **1,01** |
| E | CS1-gaIP-Lin0857 | 19,6 | **51** | **1,47** |
| F | CS1 IG-gaIP-Lin0857 | 21,0 | **54** | **1,67** |

Tel que démontré dans le tableau 15 ci-dessus, les résultats démontrent que le rendement de conversion est nettement amélioré avec les souches CS1 et CS1 IG (> à 49 %). Cela démontre donc clairement que le mannose est utilisé principalement comme substrat pour la synthèse du mannobiose et le glycérol comme source de carbone pour la croissance. Ces résultats démontrent également que le remplacement du promoteur natif de galP par un promoteur fort constitutif sur le génome permet d'obtenir un rendement de conversion élevé (49 %), ce qui présente un avantage important pour le procédé de production car cela peut permettre avantageusement de s'affranchir du plasmide contenant le gène GalP et donc de l'antibiotique nécessaire à son maintien dans la souche. Tel que démontré, la souche comprenant un promoteur constitutif en amont du gène GalP à savoir la souche CS1 IG (condition F) permet également d'obtenir un meilleur rendement de conversion et un titre en mannobiose plus élevé en présence du vecteur pWKS-GalP comparé à la souche CS1 (condition E) qui ne contient pas cette modification du promoteur.

Tel que démontré ci-dessus, des souches selon l'invention permettent avantageusement et de manière surprenante d'augmenter significativement la production d'oligosaccharides. En outre les résultats obtenus démontrent clairement que la souche selon l'invention permet d'augmenter significativement les rendements de production des oligosaccharides et ainsi d'optimiser/réduire les coûts de production.

Cet exemple démontre donc clairement que la souche qu'un exemple de souche selon l'invention permet avantageusement une production d'oligosaccharides. En outre, les résultats obtenus démontrent clairement qu'un exemple de souche selon l'invention permet avantageusement et de manière surprenante et inattendue une excrétion des oligosaccharides produits dans le milieu de culture.

Cet exemple démontre également qu'un exemple de souche selon l'invention permet avantageusement une production d'oligosaccharides avec des rendements de production élevé.

### Listes des références

**[1]** Faille, C., Michalski, J.C., Strecker, G., Mackenzie, D.W., Camus, D., Poulain, D., 1990. Immunoreactivity of neoglycolipids constructed from oligomannosidic residues of the Candida albicans cell wall. Infect. Immun. 58, 3537-3544.
**[2]** Fierfort, N., Samain, E., 2008. Genetic engineering of Escherichia coli for the economicFaille, C., Michalski, J.C., Strecker, G., Mackenzie, D.W., Camus, D., Poulain, D., 1990. Immunoreactivity of neoglycolipids constructed from oligomannosidic residues of the Candida albicans cell wall. Infect. Immun. 58, 3537-3544.
**[3]** Fierfort, N., Samain, E., 2008. Genetic engineering of Escherichia coli for the economical production of sialylated oligosaccharides. J. Biotechnol. 134, 261-265.
**[4]** Ishii, N., Nakahigashi, K., Baba, T., Robert, M., Soga, T., Kanai, A., Hirasawa, T., Naba, M., Hirai, K., Hoque, A., Ho, P.Y., Kakazu, Y., Sugawara, K., Igarashi, S., Harada, S., Masuda, T., Sugiyama, N., Togashi, T., Hasegawa, M., Takai, Y., Yugi, K., Arakawa, K., Iwata, N., Toya, Y., Nakayama, Y., Nishioka, T., Shimizu, K., Mori, H., Tomita, M., 2007. Multiple High-Throughput Analyses Monitor the Response of E. coli to Perturbations. Science 316, 593-597.
**[5]** Kiefer, P., Nicolas, C., Letisse, F., Portais, J.-C., 2007. Détermination of carbon labeling distribution of intracellular metabolites from single fragment ions by ion chromatography tandem mass spectrometry. Anal. Biochem. 360, 182-188.
**[6]** Liang, Q., Zhang, F., Li, Y., Zhang, X., Li, J., Yang, P., Qi, Q., 2015. Comparison of individual component deletions in a glucose-specific phosphotransferase system revealed their différent applications. Sci. Rep. 5, 13200.
**[7]** Link, A.J., Phillips, D., Church, G.M., 1997. Methods for generating precise deletions and insertions in the genome of wild-type Escherichia coli: application to open reading frame characterization. J. Bacteriol. 179, 6228-6237. https://doi.org/10.1128/jb.179.20.6228-6237.1997.
**[8]** Lombard, V., Golaconda Ramulu, H., Drula, E., Coutinho, P.M., Henrissat, B., 2014. The carbohydrate-active enzymes database (CAZy) in 2013. Nucleic Acids Res. 42, D490-D495.
**[9]** Mashego, M.R., Wu, L., Van Dam, J.C., Ras, C., Vinke, J.L., Van Winden, W.A., Van Gulik, W.M., Heijnen, J.J., 2004. MIRACLE: mass isotopomer ratio analysis of U-13C-labeled extracts. A new method for accurate quantification of changes in concentrations of intracellular metabolites. Biotechnol. Bioeng. 85, 620-628.
**[10]** Rong Fu Wang, Kushner, S.R., 1991. Construction of versatile low-copy-number vectors for cloning, sequencing and gene expression in Escherichia coli. Gene 100, 195-199.
**[11]** Shibata, N., Hisamichi, K., Kikuchi, T., Kobayashi, H., Okawa, Y., Suzuki, S., 1992. Sequential nuclear magnetic résonance assignment of .beta.-1,2-linked mannooligosaccharides isolated from the phosphomannan of the pathogenic yeast Candida albicans NIH B-792 strain. Biochemistry 31.
**[12]** Wu, L., Mashego, M.R., van Dam, J.C., Proell, A.M., Vinke, J.L., Ras, C., van Winden, W.A., van Gulik, W.M., Heijnen, J.J., 2005. Quantitative analysis of the microbial metabolome by isotope dilution mass spectrometry using uniformly 13C-labeled cell extracts as internal standards. Anal. Biochem. 336, 164-171.
**[13]** Combes, Monsan, 2009. Biocatalyse ou catalyse enzymatique. Procédés chimie - bio- agro I Chimie verte, Techniques de l'ingénieur 1-18.
**[14]** Rudroff, F., Mihovilovic, M.D., Gröger, H., Snajdrova, R., Iding, H., Bornscheuer, U.T., 2018. Opportunities and challenges for combining chemo- and biocatalysis. Nature Catalysis 1, 12-22. https://doi.org/10.1038/s41929-017-0010-4.
**[15]** Anastas et Warner, 2000 Anastas, P.T., Warner, J.C., 2000. Green chemistry: theory and practice, 1. paperback. ed. Oxford Univ. Press, Oxford. Imeson, 2009 Hydrocolloids as thickening and gelling agents in food: a critical review, J Food Sci Technol. 2010 Dec; 47(6): 587-597.
**[16]** Tathiana Souza Martins Meyer, Ângelo Samir Melim Miguel, Daniel Ernesto Rodríguez Fernández and Gisela Maria Dellamora Ortiz « Biotechnological Production of Oligosaccharides - Applications in the Food Industry » October 22nd 2015.
**[17]** Rudroff et al., Rudroff, F., Mihovilovic, M.D., Gröger, H., Snajdrova, R., Iding, H., Bornscheuer, U.T., 2018. Opportunities and challenges for combining chemo- and biocatalysis. Nature Catalysis 1, 12-22. 08 January 2018.
**[18]** Ager, D., 2012. Final Analysis: Biological or Chemical Catalysis? Platinum Metals Review. Volume 56, Number 2, April 2012, pp. 140-142(3).
**[19]** Reetz, M.T., 2013. Biocatalysis in Organic Chemistry and Biotechnology: Past, Présent, and Future. Journal of the American Chemical Society 135, 12480-12496.
**[20]** https://doi.org/10.1021/ja405051f.
**[21]** Puchart, V., 2015. Glycoside phosphorylases: Structure, catalytic properties and biotechnological potential. Biotechnology Advances 33, 261-276.
**[22]** Cobucci-Ponzano, B., Moracci, M., 2012. Glycosynthases as tools for the production of glycan analogs of natural products. Natural Product Reports 29, 697. March 2012.
**[23]** WO0104341, Samain & Priem (2001) Procédé de production d'oligosaccharides .
**[24]** WO2008040717 , Samain (2008) High yield production of sialic acid (Neu5ac) by fermentation.
**[25]** Murray E. J. (éd.), Methods in Molecular Biology, Vol. 7, Gene Transfer and Expression Protocols, Humana Press (1991).
**[26]** Johnston, Biolistic transformation: microbes to mice. Nature, 346 : 776-777 (1990).
**[27]** Brash et al., Strontium phosphate transfection of human cells in primary culture: stable expression of the simian virus 40 large-T-antigen gene in primary human bronchial épithélial cells. Mol. Cell Biol. 7 : 2031-2034 (1987).
**[28]** Quanfeng Liang,1 Fengyu Zhang,1 Yikui Li,1 Xu Zhang,1 Jiaojiao Li,1 Peng Yang,1 and Qingsheng Qia,1 Comparison of individual component deletions in a glucose-specific phosphotransferase system revealed their different applications Sci Rep. 2015; 5: 13200. Published online 2015 Aug 19.
**[29]** Sonja Steinsiek and Katja Bettenbrock Glucose Transport in Escherichia coli Mutant Strains with Defects in Sugar Transport Systems, J Bacteriol. 2012 Nov; 194(21): 5897-5908.
**[30]** Lombard, V., Golaconda Ramulu, H., Drula, E., Coutinho, P.M., Henrissat, B., 2014. The carbohydrate-active enzymes database (CAZy) in 2013. Nucleic Acids Res. 42, D490-D495.
**[31]** https://www.addgene.org/mol-bio-reference/cloning.
**[32]** Erju Tang, Xialin Shen, Jia Wang, Xinxiao Sun, Qipeng Yuan, "Synergetic utilization of glucose and glycerol for efficient myo-inositol biosynthesis" Biotechnology and Bioengineering 2020,.
**[33]** Constantin Ruprecht, Friedericke Bönisch, Nele Ilmbergera, Tanja V. Heyera, Erhard T.K. Haupt, Wolfgang R. Streit, Ulrich Rabausch "High level production of flavonoid rhamnosides by metagenome-derived Glycosyltransferase C in Escherichia coli utilizing dextrins of starch as a single carbon source", Metabolic Engineering Volume 55, September 2019, Pages 212-219.
**[34]** Tatiana Antoine, Alain Heyraud Dr., Claude Bosso Dr., Eric Samain Dr "Highly Efficient Biosynthesis of the Oligosaccharide Moiety of the GD3 Ganglioside by Using Metabolically Engineered Escherichia coli", Angewandte Chemie, Volume117, Issue9, February 18, 2005 Pages 1374-1376.
**[35]** https://france.promega.com/products/vectors/protein-expression-vectors.
**[36]** https://www.thermofisher.com/search/browse/category/fr/fr/85801/Bact erial%20Expression%20Vectors
**[37]** WO 83/004261.
**[38]** https://www.embl.de/pepcore/pepcore_services/strains_vectors/vector s/bacterial_expression_vectors/popup_bacterial_expression_vectors/index.html.
**[39]** http://babel.ucmp.umu.se/cpep/web_content/Pages/CPEP_09_vectors .html.
**[40]** https://www.embl.de/pepcore/pepcore_services/strains_vectors/vector s/gateway_vectors/index.htm.
**[41]** http://parts.igem.org/Promoters/Catalog/Constitutive.
**[42]** https://biocyc.org/group?id=:ALL-PROMOTERS&orgid=ECOLI.
**[43]** http://parts.igem.org/Promoters/Catalog.
**[44]** Israel Pedruzzi, Eduardo Borges da Silva, Alirio E Rodrigues "Sélection of resins, equilibrium and sorption kinetics of lactobionic acid, fructose, lactose and sorbitol" Séparation and Purification Technology 2008 Volume 63, Issue 3, 3 November 2008, Pages 600-611.
**[45]** Clarisse Nobre, José A. Teixeira & Lígia R. Rodrigues (2015) "New Trends and Technological Challenges in the Industrial Production and Purification of Fructo-oligosaccharides", Critical Reviews in Food Science and Nutrition, 55:10, 1444-1455, October 2013.
**[46]** https://www.novasep.com/technologies/industrial-technologies-for-evaporation-concentration-and-crystallization.html.
**[47]** https://international.neb.com/protocols/2014/05/07/double-digest-protocol-with-standard-restriction-enzymes.
**[48]** https://international.neb.com/protocols/0001/01/01/pcr-protocol-m0530.
**[49]** https://international.neb.com/protocols/0001/01/01/dna-ligation-with-t4-dna-ligase-m0202.
**[50]** https://www.addgene.org/protocols/bacterial-transformation/.
**[51]** http://tools.thermofisher.com/content/sfs/manuals/pentr_dtopo_man.p df.
**[52]** Karen L. Elbing Roger Brent, Recipes and Tools for Culture of Escherichia coli, Current Tools, 09 November 2018.
**[53]** Quantification of Sugar Compounds and Uronic Acids in Enzymatic Hydrolysates of Lignocellulose Using High-Performance Anion Exchange Chromatography with Pulsed Amperometric Détection Energy Fuels 2012, 26, 5, 2942-2947.
**[54]** WO2007101862, Samain (2007) Method of producing sialylated oligosaccharides.
**[55]** WO2007023348, SAMAIN et al. (2007) Production of globoside oligosaccharides using metabolically engineered microorganisms.
**[56]** Fort, S., Birikaki, L., Dubois, M. P., Antoine, T., Samain, E., & Driguez, H. (2005). Biosynthesis of conjugatable saccharidic moieties of GM 2 and GM 3 gangliosides by engineered E. coli. Chemical communications, (20), 2558-2560.
**[57]** Fierfort, N., & Samain, E. (2008). Genetic engineering of Escherichia coli for the economical production of sialylated oligosaccharides. Journal of biotechnology, 134(3-4), 261-265.
**[58]** https://www.takarabio.com/learning-centers/cloning/in-fusion-cloning-overview.
**[59]** Nord Li, Vaag P, Duus JØ Quantification of organic and amino acids in beer by 1H NMR spectroscopy Anal Chem. 2004 Aug 15;76(16):4790-8.
**[60]**Gloriadel CampoIñakiBerregiRaúlCaracenaJ. IgnacioSantos "Quantitative analysis of malic and citric acids in fruit juices using proton nuclear magnetic résonance spectroscopy.
**[61]**Chiku K, Nihira T, Suzuki E, Nishimoto M, Kitaoka M, et al. (2014) Discovery of Two b-1,2Mannoside Phosphorylases Showing Différent Chain-Length Specificities from Thermoanaerobacter sp. X-514. PLoS ONE 9(12): e114882. doi:10.1371/journal.pone.0114882.
**[62]**Kirill A. Datsenko, Barry L. Wanner. One-step inactivation of chromosomal genes in Escherichia coli K-12 using PCR products. Proceedings of the National Academy of Sciences Jun 2000, 97 (12) 6640-6645; DOI: 10.1073/pnas.120163297
**[63]**Leila Lo Leggio, Florence Dal Degan, Peter Poulsen, Soren Moller Andersen, and Sine Larsen. The Structure and Specificity of Escherichia coli Maltose Acetyltransferase Give New Insight into the LacA Family of Acyltransferases. Biochemistry 2003, 42, 18, 5225-5235 ; DOI : 10. 1021 /bi0271446
**[64]**M Freundlich , N Ramani, E Mathew, A Sirko, P Tsui, « The role of intégration host factor in gene expression in Escherichia coli » Mol Microbiol. 1992 Sep;6(18):2557-63
**[65]**Zhou, K., Zhou, L., Lim, Q. 'En, Zou, R., Stephanopoulos, G., and Too, H.-P. (2011) Novel référencé genes for quantifying transcriptional responses of Escherichia coli to protein overexpression by quantitative PCR. BMC Mol Biol 12: 18

## Revendications

1. Souche *Escherichia coli* dont les gènes recA1, gyrA96, thi-1, glnV44, relA1, hsdR17, endA1, lacZ, nanKETA, lacA, melA, wcaJ, mdoH, ptsG manX, manY, et pfkA sont inactivés.

2. Souche selon la revendication 1, déposée auprès de la CNCM (Collection Nationale de Culture de Microorganismes, Institut Pasteur, 25 rue du Docteur Roux, 75724 Paris Cedex 15, France), sous le numéro CNCM I-5499.

3. Souche selon la revendication 1 ou 2 comprenant en outre les mutations Δmaa et ΔmanA.

4. Souche selon la revendication 3, déposée auprès de la CNCM (Collection Nationale de Culture de Microorganismes, Institut Pasteur, 25 rue du Docteur Roux, 75724 Paris Cedex 15, France), sous le numéro CNCM I-5681.

5. Souche selon la revendication 3 dans laquelle le promoteur du gène GalP est le promoteur HIF.

6. Souche selon l'une quelconque des revendication 1 à 5 comprenant en outre un vecteur d'expression d'une glycoside-phosphorylase choisie parmi les β-glycoside- ou α-glycoside- phosphorylases.

7. Souche selon la revendication 6, dans laquelle les β-glycoside- ou α-glycoside- phosphorylases sont choisies dans le groupe comprenant les α-1,3-glucopyranosyl-L-rhamnose-phosphorylases, les α-1,2-glucosyl-glycérol-phosphorylases, les tréhaloses phosphorylases, les laminaribiose-phosphorylases, les D-galactosyl-β-1,4-L-rhamnose phosphorylases, les β-1,4-mannosyl-glucose phosphorylases, les β-1,2-oligomannane phosphorylases, les β-1,2-mannobiose phosphorylases, les β-1,4-mannopyranosyl-[N-glycan]-phosphorylases / les β-1,4-mannopyranosyl-chitobiose-phosphorylases, les β-1,4-mannooligosaccharide phosphorylases, les β-1,3-mannooligosaccharide phosphorylases, les β-1,3-mannosyl-glucose phosphorylases, les β-1,4-mannosyl-glucuronate phosphorylases .

8. Utilisation *in vitro* d'une souche selon l'une quelconque des revendications 1 à 7 pour la production d'oligosaccharides et/ou dans un procédé de production d'oligosaccharides.

9. Procédé *in vitro,* en particulier *in cellulo*, de production d'oligosaccharides comprenant les étapes de :
a) transformation de la souche selon l'une quelconque des revendications 1 à 5 avec un vecteur d'expression d'une enzyme, ladite enzyme étant une glycoside-phosphorylase,
b) culture de la souche transformée obtenue à l'étape a) et/ou d'une souche selon l'une quelconque des revendications 1 à 7 dans un milieu de culture, et
c) récupération des oligosaccharides produits.

10. Procédé selon la revendication 9, dans lequel la récupération des oligosaccharides produits est effectuée dans le milieu de culture.

11. Procédé selon la revendication 9 ou 10, dans lequel le procédé comprend une étape a') de transformation de ladite souche avec un vecteur d'expression d'au moins une protéine de transport ou perméase.

12. Procédé selon la revendication 11, dans lequel le milieu de culture comprend au moins un hydrate de carbone non phosphorylé choisi dans le groupe comprenant le N-acétyl-α-D-glucosamine, du galactose, du glucose, du lactose, du glycérol, du mannose, du N-acétyl-glucosamine-β-1,4-N-acétyl-glucosamine, du fucose.

13. Procédé selon l'une quelconque des revendications 9 à 12, dans lequel l'enzyme est choisie parmi les β-glycoside- ou α-glycoside-phosphorylases.

14. Procédé selon la revendication 13, dans lequel l'enzyme est choisie parmi les α-1,3-glucopyranosyl-L-rhamnose-phosphorylases, les α-1,2-glucosyl-glycérol-phosphorylases, les tréhalose-phosphorylases, les laminaribiose-phosphorylases, les D-galactosyl-β-1,4-L-rhamnose phosphorylases, les β-1,4-mannosyl-glucose-phosphorylases, les β-1,2-oligomannane phosphorylases, les β-1,2-mannobiose-phosphorylases, les β-1,4-mannopyranosyl-[N-glycane]-phosphorylases, les β-1,4-mannopyranosyl-chitobiose-phosphorylases, les β-1,4-mannooligosaccharide-phosphorylases, les β-1,3-mannooligosaccharide-phosphorylases, les β-1,3-mannosyl-glucose phosphorylases, les β-1,4-mannosyl-glucuronate phosphorylases.

15. Procédé selon l'une quelconque des revendications 9 à 14, dans lequel le milieu est choisi parmi un milieu riche, de préférence le milieu LB (lysogeny broth), Superbroth, TB (Terrific Broth), YPB (Yeast Extract-Peptone Dextrose), un milieu minimum de préférence le milieu M9 ou M63 complété avec une source de carbone, un milieu sélectif, de préférence le milieu YNB (Yeast Nitrogen Base)

## Patentansprüche

1. *Escherichia* coli-Stamm mit den Genen recA1, gyrA96, thi-1, gInV44, relA1, hsdR17, endA1, lacZ, nanKETA, lacA, melA, wcaJ, mdoH, ptsG, manX, manY und pfkA die inaktiviert sind.

2. Stamm nach Anspruch 1, hinterlegt bei der CNCM (Collection Nationale de Culture de Microorganismes, Institut Pasteur, 25 rue du Docteur Roux, 75724 Paris Cedex 15, Frankreich), unter der Nummer CNCM I-5499.

3. Stamm nach Anspruch 1 oder 2, der außerdem die Mutationen Δmaa und ΔmanA aufweist.

4. Stamm nach Anspruch 3, hinterlegt bei CNCM (Collection Nationale de Culture de Microorganismes, Institut Pasteur, 25 rue du Docteur Roux, 75724 Paris Cedex 15, Frankreich), unter der Nummer CNCM I-5681.

5. Stamm nach Anspruch 3, wobei der GalP-Genpromotor der HIF-Promotor ist.

6. Stamm nach einem der Ansprüche 1 bis 5, der ferner einen Expressionsvektor für eine Glycosidphosphorylase, ausgewählt aus β-Glycosid- oder α-Glycosidphosphorylasen, umfasst.

7. Stamm nach Anspruch 6, wobei die β-Glykosid- oder α-Glykosid-Phosphorylasen ausgewählt sind aus der Gruppe umfassend α-1,3-Glucopyranosyl-L-Rhamnose-Phosphorylasen, α-1,2-Glucosyl-Glycerin-Phosphorylasen, Trehalose-Phosphorylasen, Laminaribiose-Phosphorylasen, D-Galactosyl-β-1,4-L-Rhamnose-Phosphorylasen, β-1,4-Mannosyl-Glucose-Phosphorylasen, β-1,2-Oligomannan-Phosphorylasen, β-1,2-Mannobiose-Phosphorylasen, β-1,4-Mannopyranosyl-[N-Glykan]-Phosphorylasen / β-1,4-Mannopyranosyl-Chitobiose-Phosphorylasen, β-1,4-Mannooligosaccharid-Phosphorylasen, β-1,3-Mannooligosaccharid-Phosphorylasen, β-1,3-Mannosyl-Glucose-Phosphorylasen, β-1,4-Mannosyl-Glucuronat-Phosphorylasen.

8. *In* vitro-Verwendung eines Stammes nach einem der Ansprüche 1 bis 7 zur Herstellung von Oligosacchariden und/oder in einem Verfahren zur Herstellung von Oligosacchariden.

9. In-vitro-Verfahren, insbesondere *in Zellulo*, zur Herstellung von Oligosacchariden, das die folgenden Schritte umfasst: :
a) Transformation des Stammes nach einem der Ansprüche 1 bis 5 mit einem Enzym-Expressionsvektor, wobei das Enzym eine Glykosidphosphorylase ist,
b) Kultivierung des in Schritt a) erhaltenen transformierten Stammes und/oder eines Stammes nach einem der Ansprüche 1 bis 7 in einem Kulturmedium, und
c) die Verwertung der hergestellten Oligosaccharide.

10. Verfahren nach Anspruch 9, wobei die Gewinnung der hergestellten Oligosaccharide im Kulturmedium erfolgt.

11. Verfahren nach Anspruch 9 oder 10, wobei das Verfahren einen Schritt a') des Transformierens des Stammes mit einem Expressionsvektor für mindestens ein Transportprotein oder eine Permease umfasst.

12. Verfahren nach Anspruch 11, wobei das Kulturmedium mindestens ein nicht-phosphoryliertes Kohlenhydrat enthält, das aus der Gruppe ausgewählt ist, die N-Acetyl-α-D-glucosamin, Galactose, Glucose, Lactose, Glycerin, Mannose, N-Acetyl-glucosamin-β-1,4-N-Acetyl-glucosamin und Fucose umfasst.

13. Verfahren nach einem der Ansprüche 9 bis 12, wobei das Enzym ausgewählt ist aus β-Glykosid- oder α-Glykosid-Phosphorylasen.

14. Verfahren nach Anspruch 13, wobei das Enzym ausgewählt ist aus α-1,3-Glucopyranosyl-L-Rhamnose-Phosphorylasen, α-1,2-Glucosyl-Glycerin-Phosphorylasen, Trehalose-Phosphorylasen, Laminaribiose-Phosphorylasen, D-Galactosyl-β-1,4-L-Rhamnose-Phosphorylasen, β-1,4-Mannosyl-Glucose-Phosphorylasen, β-1,2-Oligomannan-Phosphorylasen, β-1,2-Mannobiose-Phosphorylasen, β-1,4-Mannopyranosyl-[N-Glykan]-Phosphorylasen, β-1,4-Mannopyranosyl-Chitobiose-Phosphorylasen, β-1,4-Mannooligosaccharid-Phosphorylasen, β-1,3-Mannooligosaccharid-Phosphorylasen, β-1,3-Mannosyl-Glucose-Phosphorylasen, β-1,4-Mannosyl-Glucuronat-Phosphorylasen.

15. Verfahren nach einem der Ansprüche 9 bis 14, **dadurch gekennzeichnet, dass** das Medium ausgewählt ist aus einem reichhaltigen Medium, vorzugsweise LB (Lysogeny Broth), Superbroth, TB (Terrific Broth), YPB (Yeast Extract-Peptone Dextrose), einem Minimalmedium, vorzugsweise M9 oder M63, ergänzt mit einer Kohlenstoffquelle, einem selektiven Medium, vorzugsweise YNB (Yeast Nitrogen Base).

## Claims

1. *Escherichia coli* strain with recA1, gyrA96, thi-1, glnV44, relA1, hsdR17, endA1, lacZ, nanKETA, lacA, melA, wcaJ, mdoH, ptsG, manX, manY, and pfkA genes are inactivated.

2. Strain according to claim 1, deposited with the CNCM (Collection Nationale de Culture de Microorganismes, Institut Pasteur, 25 rue du Docteur Roux, 75724 Paris Cedex 15, France), under number CNCM I-5499.

3. Strain according to claim 1 or 2 further comprising the Δmaa and ΔmanA mutations.

4. Strain according to claim 3, deposited with CNCM (Collection Nationale de Culture de Microorganismes, Institut Pasteur, 25 rue du Docteur Roux, 75724 Paris Cedex 15, France), under number CNCM I-5681.

5. Strain according to claim 3 wherein the GalP gene promoter is the HIF promoter.

6. Strain according to any one of claims 1 to 5 further comprising an expression vector for a glycoside phosphorylase selected from β-glycoside or α-glycoside phosphorylases.

7. Strain according to claim 6, wherein the β-glycoside- or α-glycoside-phosphorylases are selected from the group comprising α-1,3-glucopyranosyl-L-rhamnose-phosphorylases, α-1,2-glucosyl-glycerol phosphorylases, trehalose phosphorylases, laminaribiose phosphorylases, D-galactosyl-β-1,4-L-rhamnose phosphorylases, β-1,4-mannosyl-glucose phosphorylases, β-1,2-oligomannan phosphorylases, β-1,2-mannobiose phosphorylases, β-1,4-mannopyranosyl-[N-glycan]-phosphorylases / β-1,4-mannopyranosyl-chitobiose-phosphorylases, β-1,4-mannooligosaccharide phosphorylases, β-1,3-mannooligosaccharide phosphorylases, β-1,3-mannosyl-glucose phosphorylases, β-1,4-mannosyl-glucuronate phosphorylases.

8. *In vitro* use of a strain according to any one of claims 1 to 7 for the production of oligosaccharides and/or in a process for the production of oligosaccharides.

9. An *in vitro,* in particular *in cellulo*, process for the production of oligosaccharides comprising the steps of :
a) transformation of the strain according to any one of claims 1 to 5 with an enzyme expression vector, said enzyme being a glycoside phosphorylase,
b) culture of the transformed strain obtained in step a) and/or of a strain according to any one of claims 1 to 7 in a culture medium, and
c) recovery of the oligosaccharides produced.

10. Process according to claim 9, wherein recovery of the oligosaccharides produced is carried out in the culture medium.

11. A process according to claim 9 or 10, wherein the process comprises a step a') of transforming said strain with an expression vector for at least one transport protein or permease.

12. Process according to claim 11, wherein the culture medium comprises at least one non-phosphorylated carbohydrate selected from the group comprising N-acetyl-α-D-glucosamine, galactose, glucose, lactose, glycerol, mannose, N-acetyl-glucosamine-β-1,4-N-acetyl-glucosamine, fucose.

13. Process according to any one of claims 9 to 12, wherein the enzyme is selected from β-glycoside- or α-glycoside- phosphorylases.

14. Process according to claim 13, wherein the enzyme is selected from α-1,3-glucopyranosyl-L-rhamnose-phosphorylases, α-1,2-glucosyl-glycerol-phosphorylases, trehalose-phosphorylases, laminaribiose-phosphorylases, D-galactosyl-β-1,4-L-rhamnose phosphorylases, β-1,4-mannosyl-glucose-phosphorylases, β-1,2-oligomannan phosphorylases, β-1,2-mannobiose-phosphorylases, β-1,4-mannopyranosyl-[N-glycan]-phosphorylases, β-1,4-mannopyranosyl-chitobiose-phosphorylases, β-1,4-mannooligosaccharide phosphorylases, β-1,3-mannooligosaccharide phosphorylases, β-1,3-mannosyl-glucose phosphorylases, β-1,4-mannosyl-glucuronate phosphorylases.

15. Process according to any one of claims 9 to 14, wherein the medium is chosen from a rich medium, preferably LB (lysogeny broth), Superbroth, TB (Terrific Broth), YPB (Yeast Extract-Peptone Dextrose), a minimum medium, preferably M9 or M63 medium supplemented with a carbon source, a selective medium, preferably YNB (Yeast Nitrogen Base) medium.
